# EUROPEAN PATENT APPLICATION

(11) **EP 2 423 176 A1**
(43) Date of publication of application: **29.02.2012**
(21) Application number: 10767073.9
(22) Date of filing: 21.04.2010
(51) Int. Cl.: C07C 43/225, A61K 31/40, A61K 31/4015, A61P 3/10, C07C 49/417, C07C 69/76, C07C 235/18, C07C 303/36, C07D 207/06, C07D 207/27, C07D 239/34, C07D 305/06, C07D 307/94, C07D 309/06, C07D 309/10, C07D 317/22, C07D 405/12, C07K 14/705, C12N 15/09

(54) **CARBOXYLIC ACID COMPOUND**

(30) Priority: 22.04.2009 JP 2009103765
(71) Applicant: Astellas Pharma Inc., Tokyo 103-8411 (JP)
(72) Inventor: NEGORO, Kenji, Tokyo 103-8411 (JP); OHNUKI, Kei, Tokyo 103-8411 (JP); YONETOKU, Yasuhiro, Tokyo 103-8411 (JP); KURAMOTO, Kazuyuki, Tokyo 103-8411 (JP); URANO, Yasuharu, Tokyo 103-8411 (JP); WATANABE, Hideyuki, Tokyo 103-8411 (JP)
(74) Representative: Gille Hrabal
(86) International application number: PCT/JP2010/057034
(87) International publication number: WO 2010/123016

(57) **Abstract**

[Problem]

The present invention has an object to provide a compound having a GPR40 agonistic activity, which is useful as a pharmaceutical composition, an insulin secretion promoter, or an agent for preventing/treating diabetes.

[Means for Solution]

The present inventors have extensively studied a compound having a GPR40 agonistic activity, and as a result, they have found that the compound (I) of the present invention or a pharmaceutically acceptable salt thereof, in which a carboxylic acid is bonded to a bicyclic or tricyclic moiety through methylene, and further, a benzene ring substituted with a monocyclic 6-membered aromatic ring is bonded to a bicyclic or tricyclic moiety through -O-methylene or -NH-methylene, has an excellent GPR40 agonistic activity. They have also found that the compound has an excellent insulin secretion promoting action and strongly inhibits increase in the blood glucose after glucose loading, thereby completing the present invention.

SEQUENCE LISTING

<110>  Astellas Pharma Inc.

<120>  carboxyric acid compounds

<130>  A10019

<160>  4

<170>  PatentIn version 3.1

<210>  1
<211>  25
<212>  DNA
<213>  Artificial

<220>
<223>  Description of Artifical Sequence:an artificailly synthesized pri
       mer seqence
<220>
<223>  Inventor: Negoro, Ohnuki, Kei; Yonetoku, Yasuhiro;
         Inventor: Kuramoto, Kazuyuki; Urano, Yasuharu, Watanabe, Hideyuki

<400>  1
ggtctagaat ggacctgccc ccgca                                          25


<210>  2
<211>  27
<212>  DNA
<213>  Artificial

<220>
<223>  Description of Artifical Sequence:an artificailly synthesized pri
       mer seqence

<400>  2
ggtctagatt acttctggga cttgccc                                        27


<210>  3
<211>  903
<212>  DNA
<213>  Homo sapiens

<400>  3
atggacctgc ccccgcagct ctccttcggc ctctatgtgg ccgcctttgc gctgggcttc      60

ccgctcaacg tcctggccat ccgaggcgcg acggcccacg cccggctccg tctcacccct     120

agcctggtct acgccctgaa cctgggctgc tccgacctgc tgctgacagt ctctctgccc     180

ctgaaggcgg tggaggcgct agcctccggg gcctggcctc tgccggcctc gctgtgcccc     240

gtcttcgcgg tggcccactt cttcccactc tatgccggcg ggggcttcct ggccgccctg      300

agtgcaggcc gctacctggg agcagccttc cccttgggct accaagcctt ccggaggccg     360

tgctattcct ggggggtgtg cgcggccatc tgggccctcg tcctgtgtca cctgggtctg     420

gtctttgggt tggaggctcc aggaggctgg ctggaccaca gcaacacctc cctgggcatc     480

aacacaccgg tcaacggctc tccggtctgc ctggaggcct gggacccggc ctctgccggc     540

ccggcccgct tcagcctctc tctcctgctc ttttttctgc ccttggccat cacagccttc     600

tgctacgtgg gctgcctccg ggcactggcc cactccggcc tgacgcacag gcggaagctg     660

cgggccgcct gggtggccgg cggggccctc ctcacgctgc tgctctgcgt aggaccctac     720

aacgcctcca acgtggccag cttcctgtac cccaatctag gaggctcctg gcggaagctg     780

gggctcatca cgggtgcctg gagtgtggtg cttaatccgc tggtgaccgg ttacttggga     840

aggggtcctg gcctgaagac agtgtgtgcg gcaagaacgc aagggggcaa gtcccagaag     900

taa                                                                   903

<210>  4
<211>  300
<212>  PRT
<213>  Homo sapiens

<400>  4

Met Asp Leu Pro Pro Gln Leu Ser Phe Gly Leu Tyr Val Ala Ala Phe
1               5                   10                  15

Ala Leu Gly Phe Pro Leu Asn Val Leu Ala Ile Arg Gly Ala Thr Ala
            20                  25                  30

His Ala Arg Leu Arg Leu Thr Pro Ser Leu Val Tyr Ala Leu Asn Leu
        35                  40                  45

Gly Cys Ser Asp Leu Leu Leu Thr Val Ser Leu Pro Leu Lys Ala Val
    50                  55                  60

Glu Ala Leu Ala Ser Gly Ala Trp Pro Leu Pro Ala Ser Leu Cys Pro
65                  70                  75                  80

Val Phe Ala Val Ala His Phe Phe Pro Leu Tyr Ala Gly Gly Gly Phe
                85                  90                  95

Leu Ala Ala Leu Ser Ala Gly Arg Tyr Leu Gly Ala Ala Phe Pro Leu
            100                 105                 110

Gly Tyr Gln Ala Phe Arg Arg Pro Cys Tyr Ser Trp Gly Val Cys Ala
        115                 120                 125

Ala Ile Trp Ala Leu Val Leu Cys His Leu Gly Leu Val Phe Gly Leu
    130                 135                 140

Glu Ala Pro Gly Gly Trp Leu Asp His Ser Asn Thr Ser Leu Gly Ile
145                 150                 155                 160

Asn Thr Pro Val Asn Gly Ser Pro Val Cys Leu Glu Ala Trp Asp Pro
                165                 170                 175

Ala Ser Ala Gly Pro Ala Arg Phe Ser Leu Ser Leu Leu Leu Phe Phe
            180                 185                 190

Leu Pro Leu Ala Ile Thr Ala Phe Cys Tyr Val Gly Cys Leu Arg Ala
        195                 200                 205

Leu Ala His Ser Gly Leu Thr His Arg Arg Lys Leu Arg Ala Ala Trp
    210                 215                 220

Val Ala Gly Gly Ala Leu Leu Thr Leu Leu Leu Cys Val Gly Pro Tyr
225                 230                 235                 240

Asn Ala Ser Asn Val Ala Ser Phe Leu Tyr Pro Asn Leu Gly Gly Ser
                245                 250                 255

Trp Arg Lys Leu Gly Leu Ile Thr Gly Ala Trp Ser Val Val Leu Asn
            260                 265                 270

Pro Leu Val Thr Gly Tyr Leu Gly Arg Gly Pro Gly Leu Lys Thr Val
        275                 280                 285

Cys Ala Ala Arg Thr Gln Gly Gly Lys Ser Gln Lys
    290                 295                 300

## Description

### Technical Field

The present invention relates to a novel carboxylic acid compound or a pharmaceutically acceptable salt thereof, which is useful as a pharmaceutical, in particular, an insulin secretion promoter, or an agent for preventing/treating diabetes.

### Background Art

Diabetes is a disease having a chronically high blood glucose levels as the main symptom, which is generated by absolute or relative insufficiency of insulin action. Clinically, it is roughly divided into insulin-dependent diabetes mellitus (IDDM) and non-insulin-dependent diabetes mellitus (NIDDM). In non-insulin-dependent diabetes mellitus (NIDDM), lowering of insulin secretion from pancreatic β cells is one of the main causes of the onset of the disease, and particularly a high blood glucose level after meals is recognized due to an initial stage insulin secretion disorder.

Recently, it has been confirmed by large scale clinical tests that correction of high blood glucose levels after meals is important for the onset and suppression of diabetic complications. In addition, it has been reported that arteriosclerosis is generated only at a stage of high blood glucose levels after meals, and that continuation of slightly high blood glucose levels after meals increases mortality rates caused by vascular disease and the like. It has been shown that a high blood glucose level after meals is an independent risk factor for cardiovascular death even when it is slight. Based on the above information, the necessity for a drug therapy for high blood glucose levels after meals has been recognized.

Currently, sulfonylurea (SU) preparations are mainstream as the insulin secretion promoters, but it is known that they are apt to cause hypoglycemia and induce secondary invalidity due to exhaustion of the pancreas in the case of its long-time administration. In addition, the SU preparations are effective in controlling blood glucose levels during meals, but have difficulty in suppressing blood glucose level after meals.

GPR40 is a G protein-coupled receptor which has been identified as a fatty acid receptor and is highly expressed in β cells of the pancreas, and it has been reported that it is concerned in the insulin secretory action of fatty acids (Non-patent Document 1).
Accordingly, since correction of high blood glucose levels after meals is expected based on its insulin secretion promoting action, the GPR40 receptor agonist is useful as an agent for preventing/treating insulin dependent diabetes mellitus (IDDM), non-insulin-dependent diabetes mellitus (NIDDM), or borderline type (abnormal glucose tolerance and fasting blood glucose level) mild diabetes.

In Patent Document 1, it is reported that a compound of the formula (A) including a broad range of compounds has the GPR40 receptor-controlling action, and is therefore useful as an insulin secretion promoter or a drug for preventing and/or treating diabetes. However, there is no specific disclosure of a compound having the structure of the invention of the present Application. 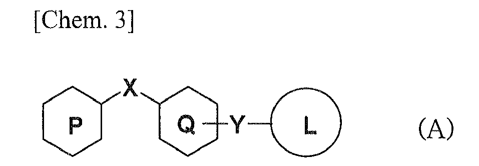 (wherein a ring P represents an aromatic ring which may have a substituent, a ring Q represents an aromatic ring which may have a substituent other than: 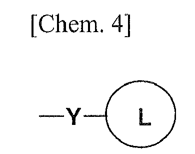 and X and Y represent spacers, and 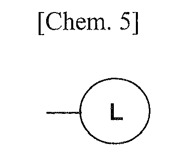 represents a group capable of discharging positive ions).

In Patent Document 2, it is reported that a compound of the formula (B) has the GPR40 receptor-controlling action, and is therefore useful as an insulin secretion promoter or a drug for preventing and/or treating diabetes. 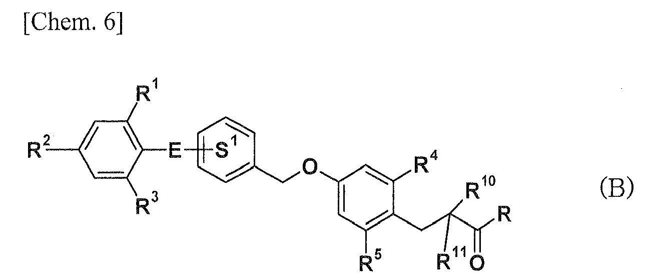 (for the symbols in the formula, refer to the patent publication).

In Patent Document 3, it is reported that a compound of the formula (C) has the GPR40 receptor-controlling action, and is therefore useful as an insulin secretion promoter or a drug for preventing and/or treating diabetes. 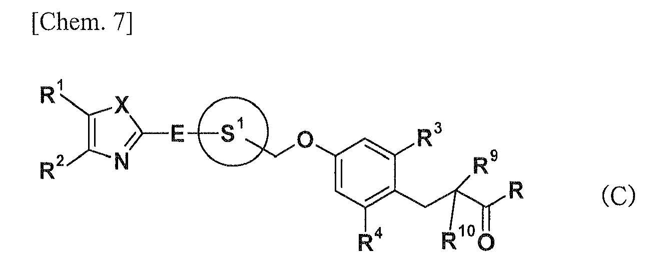 (for the symbols in the formula, refer to the patent publication).

In Patent Document 4, it is reported that an oxadiazolidinedione compound of the formula (D) has a blood glucose level-lowering action and a blood lipid-lowering action, and is therefore useful in treating diabetes. 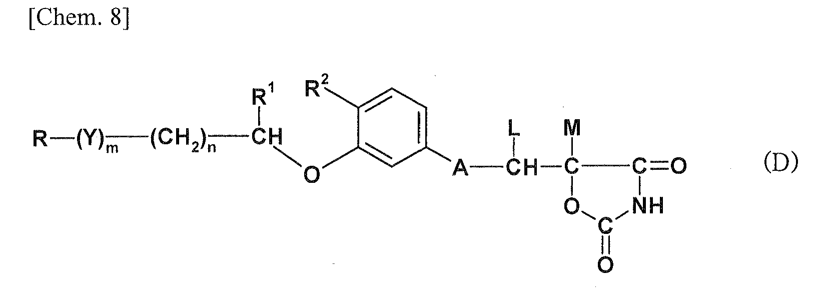 (for the symbols in the formula, refer to the patent publication).

In Patent Document 5, it is reported that a compound of the formula (E) is useful for hyperlipemia, hyperglycemia, obesity, or the like. 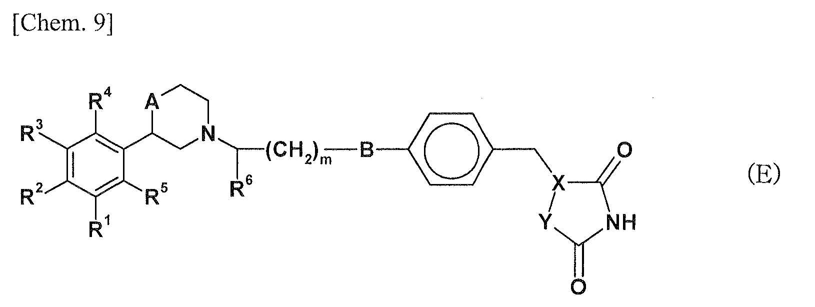 (A in the formula means an oxygen atom or a sulfur atom; for the other symbols, refer to the patent publication).

In Non-Patent Document 2, it is reported that an oxadiazolidinedione compound of the formula (F) has a blood glucose level-lowering action, and is therefore useful in treating diabetes. 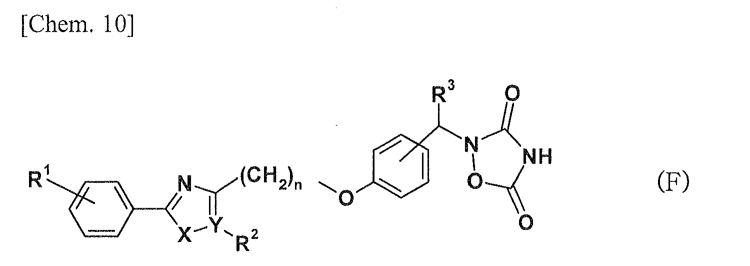 (wherein X means O, S or N, Y means C or N, and n means 1 or 2; for the symbols in the formula, refer to the patent publication).

In Patent Document 6, it is reported that a compound of the formula (G) has the GPR40 receptor-controlling action, and is therefore useful as an insulin secretion promoter or a drug for preventing and/or treating diabetes. 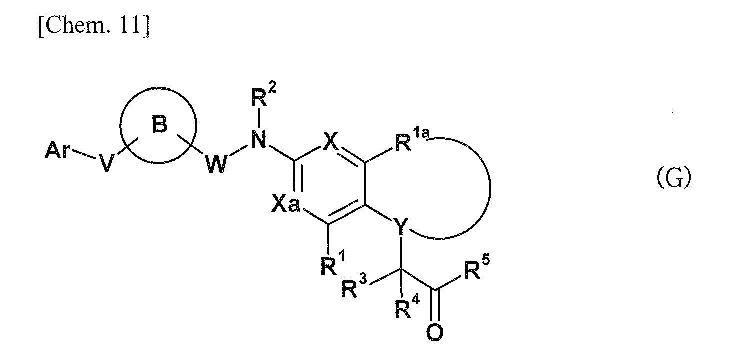 (for the symbols in the formula, refer to the patent publication).

In Patent Document 7, it is reported that a compound of the formula (H) has the GPR40 receptor-controlling action, and is therefore useful as an insulin secretion promoter or a drug for preventing and/or treating diabetes. 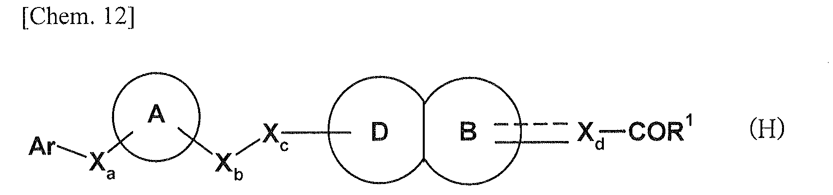 (wherein 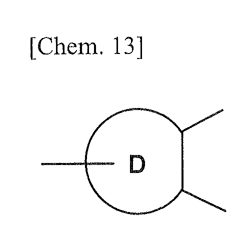 is 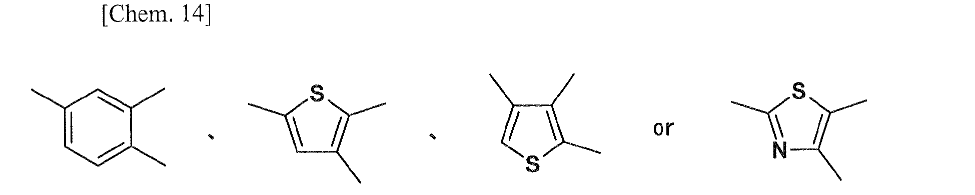 and for the symbols in the formula, refer to the patent publication).

In Patent Document 8, it is reported that a compound of the formula (J) has the GPR40 receptor-controlling action, and is therefore useful as an insulin secretion promoter or a drug for preventing and/or treating diabetes. 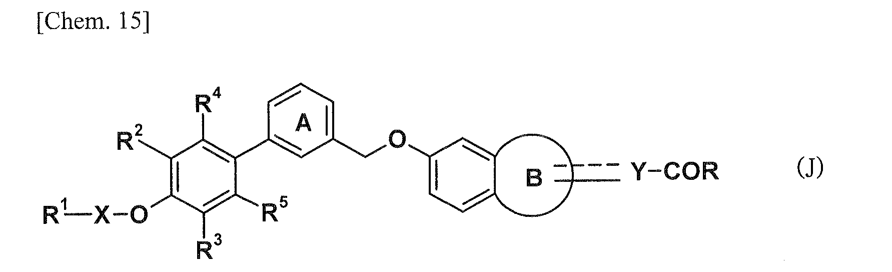 (for the symbols in the formula, refer to the patent publication).

In Patent Document 9, it is reported that a compound of the formula (K) has the GPR40 receptor-controlling action, and is therefore useful as an insulin secretion promoter or a drug for preventing and/or treating GPR40-related diseases such as diabetes (IDDM, NIDDM, etc.), and the like. 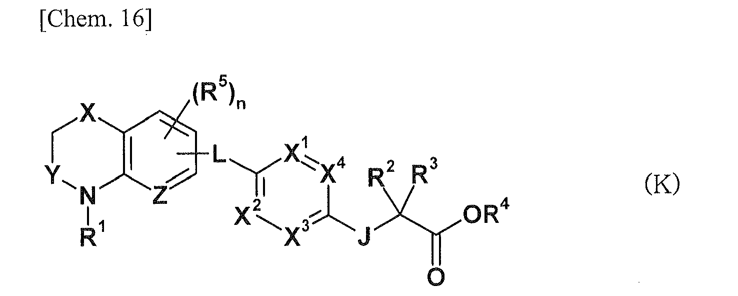 (for the symbols in the formula, refer to the patent publication).

### Prior Art

### Patent Document

Patent Document 1 Pamphlet of International Publication WO 2004/041266
Patent Document 2 Pamphlet of International Publication WO 2005/063729
Patent Document 3 Pamphlet of International Publication WO 2005/063725
Patent Document 4 JP-A-2000-212174
Patent Document 5 JP-A-7-2848
Patent Document 6 Pamphlet of International Publication WO 2005/087710
Patent Document 7 Pamphlet of International Publication WO 2004/106276
Patent Document 8 Pamphlet of International Publication WO 2008/001931
Patent Document 9 Pamphlet of International Publication WO 2008/066097

### Non-Patent Document

Non-Patent Document 1 "Nature", (UK), 2003, Vol. 422, p. 173-176
Non-Patent Document 2 "European Journal of Medicinal Chemistry", (France), 2001, Vol. 36, p. 31-42

### Summary of Invention

### Problems to Be Solved by the Invention

It is an object of the present invention to provide a compound having a GPR40 agonistic activity, which is useful as a pharmaceutical composition, an insulin secretion promoter, or an agent for preventing/treating diabetes.

### Means for Solving the Problems

The present inventors have extensively studied a compound having a GPR40 agonistic activity, and as a result, they have found that the compound (I) of the present invention or a pharmaceutically acceptable salt thereof, in which a carboxylic acid is bonded to a bicyclic or tricyclic moiety through methylene, and further, a benzene ring substituted with a monocyclic 6-membered aromatic ring is bonded to a bicyclic or tricyclic moiety through -O-methylene or -NH-methylene, has an excellent GPR40 agonistic activity. They have also found that the compound has an excellent insulin secretion promoting action and strongly inhibits increase in the blood glucose after glucose loading, thereby completing the present invention.

Thus, the present invention relates to a compound of the following formula (I) or a pharmaceutically acceptable salt thereof, and a composition comprising the compound of the following formula (I) or a pharmaceutically acceptable salt thereof: 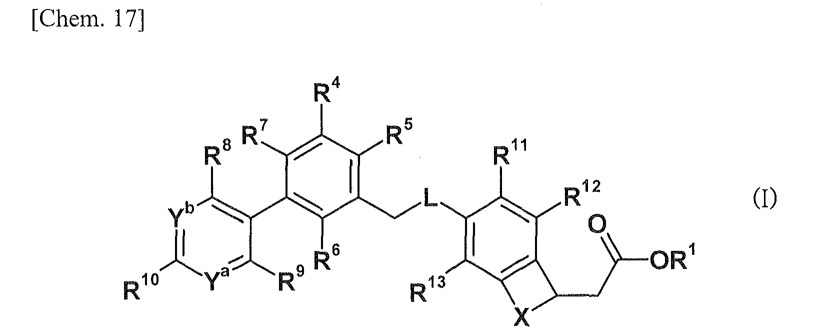 (wherein
L represents O or NH,
R¹ represents H or lower alkyl,
X represents 1,2-phenylene or -Z-C(R²)(R³)-,
Z represents O or CH₂,
R² and R³ are combined with each other to form C₂₋₇ alkylene which may be substituted,
R⁴, R⁵, R⁶, R⁷, R⁸, and R⁹ are the same as or different from each other and represent H, halogen, lower alkyl which may be substituted, or -O-(lower alkyl which may be substituted),
R¹⁰ represents H, OH, -O-(hetero ring group which may be substituted), or -O-(CR¹⁰¹R¹⁰²)ₙ-R¹⁰³,
R¹⁰¹ and R¹⁰² are the same as or different from each other and represent H, OH, halogen, or lower alkyl which may be substituted, or
R¹⁰¹ and R¹⁰² are combined with each other to form oxo (=O),
n represents 1, 2, 3, or 4,
R¹⁰³ represents H, OH, halogen, NR^{N1}R^{N2}, -SO₂-(lower alkyl which may be substituted), aryl which may be substituted, -O-(lower alkyl which may be substituted), or a hetero ring group which may be substituted,
R^{N1} and R^{N2} are the same as or different from each other and represent H, -SO₂-(lower alkyl which may be substituted), or lower alkyl which may be substituted,
R¹¹, R¹², and R¹³ are the same as or different from each other and represent H, halogen, lower alkyl which may be substituted, or -O-(lower alkyl which may be substituted),
Y^{a} and Y^{b} are the same as or different from each other, N, or C-R^{Y}, and
R^{Y} represents H, halogen, lower alkyl which may be substituted, or -O-(lower alkyl which may be substituted)).

Further, unless specifically described otherwise, in the case where the symbols in any of the formulae in the present specification are also used in other formulae, the same symbols denote the same meanings. In addition, when of -O-(CR¹⁰¹R¹⁰²)ₙ-R¹⁰³ in R¹⁰ is 2, 3, or 4, and CR¹⁰¹R^{102,}s may be the same as or different from each other, and for example, in the case of n=2, they may be -O-C(=O)-CH₂-R¹⁰³.

Moreover, the present invention relates to a pharmaceutical composition for preventing or treating GPR40-related diseases, comprising the compound of the formula (I) or a salt thereof, that is, an agent for preventing or treating GPR40-related diseases, including the compound of the formula (I) or a salt thereof.
Furthermore, the present invention relates to use of the compound of the formula (I) or a salt thereof for the manufacture of a pharmaceutical composition for preventing or treating GPR40-related diseases, the compound of the formula (I) or a salt thereof for preventing or treating GPR40-related diseases, and a method for preventing or treating GPR40-related diseases, including administering to a patient an effective amount of the compound of the formula (I) or a salt thereof.

### Effects of the Invention

The compound of the present invention has an excellent GPR40 agonistic activity, and is therefore useful as an insulin secretion promoter, or an agent for preventing/treating GPR40-related diseases, such as diabetes (insulin-dependent diabetes (IDDM), non-insulin-dependent diabetes (NIDDM), or borderline type (abnormal glucose tolerance and fasting blood glucose level) mild diabetes), and the like.

### Best Mode for Carrying Out the Invention

Hereinafter, the present invention will be explained in more detail. Further, "the compound of the formula (I) or a salt thereof" may be denoted as "the compound of the present invention (I)" or "the compound (I)" below in some cases.

In the present specification, the "lower alkyl" is straight or branched alkyl having 1 to 6 carbon atoms (hereinafter simply referred to as C₁₋₆), for example, methyl, ethyl, n-propyl, isopropyl, n-butyl, isobutyl, sec-butyl, tert-butyl, n-pentyl, n-hexyl, and the like. In another embodiment, it is C₁₋₄ alkyl, and in a further embodiment, C₁₋₃ alkyl.

The "alkylene" is straight or branched C₁₋₆ alkylene, for example methylene, ethylene, trimethylene, tetramethylene, pentamethylene, hexamethylene, propylene, methylmethylene, ethylethylene, 1,2-dimethylethylene, 1,1,2,2-tetrarnethylethylene, and the like. In another embodiment, it is C₁₋₆ alkylene, in a further embodiment, C₁₋₄ alkylene, in a still further embodiment, C₁₋₃ alkylene, and in a still further embodiment, C₂₋₇ alkylene.

The "aryl" is to a C₆₋₁₄ monocyclic to tricyclic aromatic hydrocarbon ring group, and includes a ring group fused with C₅₋₈ cycloalkene at its double bond site. It is, for example, phenyl, naphthyl, 5-tetrahydronaphthyl, 4-indenyl, 1-fluorenyl, or the like.

The "hetero ring" means a ring group containing i) a monocyclic 3- to 8-membered, and in another embodiment, a 5- to 7-membered hetero ring, containing 1 to 4 hetero atoms selected from oxygen, sulfur, and nitrogen, and ii) a bicyclic to tricyclic hetero ring (in which the bicyclic to tricyclic heterocyclic ring includes a spiro ring) containing 1 to 5 hetero atoms selected from oxygen, sulfur, and nitrogen, formed by condensation of the monocyclic hetero ring with one or two rings selected from the group consisting of a monocyclic hetero ring, a benzene ring, C₅₋₈ cycloalkane, and C₅₋₈ cycloalkene. The ring atom, sulfur or nitrogen, may be oxidized to form an oxide or a dioxide.

Examples of the "hetero ring" include the following embodiments:
(1) Monocyclic Saturated Hetero Ring Groups
   (a) those containing 1 to 4 nitrogen atoms, for example, azepanyl, diazepanyl, aziridinyl, azetidinyl, pyrrolidinyl, imidazolidinyl, piperidyl, piperazolidinyl, piperazinyl, azocanyl, hexamethyleneimino, homopiperazinyl, and the like;
   (b) those containing 1 to 3 nitrogen atoms and 1 to 2 sulfur atoms and/or 1 to 2 oxygen atoms, for example, thiomorpholinyl, thiazolidinyl, isothiazolidinyl, oxazolidinyl, morpholinyl, and the like;
   (c) those containing 1 to 2 sulfur atoms, for example, tetrahydrothiopyranyl and the like;
   (d) those containing 1 to 2 sulfur atoms and 1 to 2 oxygen atoms, for example, oxathiolanyl and the like; and
   (e) those containing 1 to 2 oxygen atoms, for example, oxiranyl, oxetanyl, dioxolanyl, tetrahydrofuranyl, tetrahydropyranyl, 1,4-dioxanyl, and the like;

(2) Monocyclic Unsaturated Hetero Ring Groups
   (a) those containing 1 to 4 nitrogen atoms, for example, pyrrolyl, 2-pyrrolinyl, imidazolyl, 2-imidazolinyl, pyrazolyl, 2-pyrazolinyl, pyridyl, dihydropyridyl, tetrahydropyridinyl, pyrimidinyl, pyrazinyl, pyridazinyl, triazolyl, tetrazolyl, triazinyl, dihydrotriazinyl, azepinyl, and the like;
   (b) those containing 1 to 3 nitrogen atoms and 1 to 2 sulfur atoms and/or 1 to 2 oxygen atoms, for example, thiazolyl, isothiazolyl, thiadiazolyl, dihydrothiazinyl, oxazolyl, isoxazolyl, oxadiazolyl, oxazinyl, and the like;
   (c) those containing 1 to 2 sulfur atoms, for example, thienyl, thiepinyl, dihydrodithiopyranyl, dihydrodithionyl, 2H-thiopyranyl, and the like;
   (d) those containing 1 to 2 sulfur atoms and 1 to 2 oxygen atoms, for example, dihydroxythiopyranyl and the like; and
   (e) those containing 1 to 2 oxygen atoms, for example, furyl, dihydrofuryl, pyranyl, 2H-pyranyl, oxepinyl, dioxolyl, and the like;

(3) Fused Polycyclic Saturated Hetero Ring Group
   (a) those containing 1 to 5 nitrogen atoms, for example, quinuclidinyl, 7-azabicyclo[2.2.1]heptyl, 3-azabicyclo[3.2.2]nonanyl, 2,8-diazaspiro[4.5]deca-8-yl, 2,3,6,8-tetraazaspiro[4.5]decan-8-yl, and the like;
   (b) those containing 1 to 4 nitrogen atoms and 1 to 3 sulfur atoms, and/or 1 to 3 oxygen atoms, for example, trithiadiazaindenyl, dioxoloimidazolidinyl, 6-oxa-2,8-diazaspiro[4.5]decan-8-yl, 6-thia-2,8-diazaspiro[4.5]decan-8-yl, and the like; and
   (c) those containing 1 to 3 sulfur atoms and/or 1 to 3 oxygen atoms, for example, 2,6-dioxabicyclo[3.2.2]octo-7-yl, 2-oxa-6-thiaspiro[4.5]decan-8-yl, and the like;

(4) Fused Polycyclic Unsaturated Hetero Ring Groups
   (a) those containing 1 to 5 nitrogen atoms, for example, indolyl, isoindolyl, indolinyl, indolizinyl, benzoimidazolyl, dihydrobenzoimidazolyl, tetrahydrobenzoimidazolyl, quinolyl, tetrahydroquinolyl, isoquinolyl, tetrahydroisoquinolyl, indazolyl, imidazopyridyl, benzotriazolyl, tetrazolopyridazinyl, carbazolyl, acridinyl, quinoxalinyl, dihydroquinoxalinyl, tetrahydroquinoxalinyl, phthalazinyl, dihydroindazolyl, benzopyrimidinyl, naphthyridinyl, quinazolinyl, cinnolinyl, pyridopyrrolidinyl, triazolopiperidinyl, 9,10-dihydroacridine, 2,8-diazaspiro[4.5]deca-3-en-8-yl, 2,3,6,8-tetraazaspiro[4.5]deca-1-en-8-yl, and the like;
   (b) those containing 1 to 4 nitrogen atoms, and 1 to 3 sulfur atoms and/or 1 to 3 oxygen atoms, for example, benzothiazolyl, dihydrobenzothiazolyl, benzothiadiazolyl, imidazothiazolyl, imidazothiadiazolyl, benzoxazolyl, dihydrobenzoxazolyl, dihydrobenzoxazinyl, benzoxadiazolyl, benzoisothiazolyl, benzoisoxazolyl, thiazolopiperidinyl, 10H-phenothiazine, 6-oxa-2,8-diazaspiro[4.5]deca-3-en-8-yl, 6-thia-2,8-diazaspiro[4.5]deca-3-en-8-yl, and the like;
   (c) those containing 1 to 3 sulfur atoms, for example, benzothienyl, benzodithiopyranyl, chromanyl, dibenzo[b,d]thienyl, and the like;
   (d) those containing 1 to 3 sulfur atoms and 1 to 3 oxygen atoms, for example, benzoxathiopyranyl, phenoxazinyl, 2-oxa-6-thiaspiro[4.5]deca-3-en-8-yl, and the like; and
   (e) those containing 1 to 3 oxygen atoms, for example, benzodioxolyl, benzofuranyl, dihydrobenzofuranyl, isobenzofuranyl, chromanyl, chromenyl, isochromenyl, dibenzo[b,d]furanyl, methylenedioxyphenyl, ethylenedioxyphenyl, xanthenyl, and the like;
etc.

The "nitrogen-containing hetero ring" group refers to one containing 1 to 5 nitrogen atoms, as in (1)(a), (1)(b), (2)(a), (2)(b), (3)(a), (3)(b), (4)(a), (4)(b), and the like, among the "hetero ring" groups above.

The "nitrogen-containing monocyclic saturated hetero ring" group refers to one containing 1 to 5 nitrogen atoms, as in (1)(a), (1)(b), and the like, among the "monocyclic saturated hetero ring" groups above.

The "nitrogen-containing monocyclic unsaturated hetero ring" group refers to one containing 1 to 5 nitrogen atoms, as in (2)(a), (2)(b), and the like, among the "hetero ring" groups above.

The "condensed nitrogen-containing polycyclic saturated hetero ring" group refers to one containing 1 to 5 nitrogen atoms, as in (3)(a), (3)(b), and the like, among the "hetero ring" groups above.

The "condensed nitrogen-containing polycyclic unsaturated hetero ring" group refers to one containing 1 to 5 nitrogen atoms, as in (4)(a), (4)(b), and the like, among the "hetero ring" groups above.

The "monocyclic 6-membered aromatic ring" refers to a monocyclic ring group having an aromatic 6-membered structure, among the "aryl" and "hetero ring" groups above, and examples thereof include phenyl, pyridyl, pyrimidyl, and the like.

Furthermore, the "aryl" and "hetero ring" groups above are described as monovalent groups, but they may be represented by divalent or higher groups in some cases.

The "halogen" means F, Cl, Br, or I, and preferably F, Cl, or Br.

The expression "R² and R³ are combined with each other to form C₂₋₇ alkylene" indicates that R² and R³ are combined with a carbon atom to which they are bonded to form a saturated C₃₋₈ hydrocarbon ring. The saturated hydrocarbon ring is, for example, cyclopropane, cyclobutane, cyclopentane, cyclohexane, cycloheptane, cyclooctane, or the like, in another embodiment, C₂₋₆ alkylene, and in a further embodiment, C₂₋₄ alkylene.

In the present specification, the expression "which may be substituted" represents "which is not substituted" or "which is substituted with 1 to 5 substituents". Further, if it has a plurality of substituents, the substituents may be the same as or different from each other. For example, in the case where with regard to -NR^{N1}R^{N2}, R^{N1} and R^{N2} are both lower alkyl, the present substituent includes an ethylmethylamino group.

Examples of the embodiments of the substituent acceptable in the "aryl which may be substituted" and "hetero ring which may be substituted" groups in R¹⁰³ include the groups shown in (a) to (i) below, and oxo (=O), in another embodiment, the groups shown in (a), (b), (f), and (i) below, and oxo (=O), and in a further embodiment, for example, the groups shown in (i), and oxo (=O).
(a) Halogen.
(b) -OH or -O-lower alkyl (in which the lower alkyl may be substituted with 1 to 3 halogen atoms).
(c) Amino which may be substituted with 1 or 2 lower alkyl groups; or nitro.
(d) -SH or -S-lower alkyl (in which the lower alkyl may be substituted with 1 to 3 halogen atoms).
(e) -SO₂-lower alkyl, -SO₂-cycloalkyl, -SO₂-hetero ring group, -SO₂-aryl, or sulfamoyl which may be substituted with 1 or 2 lower alkyl groups.
(f) -CHO, -CO-lower alkyl, -CO-cycloalkyl, -CO-monocyclic saturated hetero ring group (in which the hetero ring group may be substituted with halogen, lower alkyl, -O-lower alkyl or oxo (=O)), or cyano.
(g) Aryl or cycloalkyl; this group may be substituted with halogen, lower alkyl, or -O-lower alkyl.
(h) Hetero ring group; this hetero ring group may be substituted with halogen, lower alkyl, -O-lower alkyl, or oxo (=O).
(i) Lower alkyl which may be substituted with at least one group selected from the substituents shown in (a) to (h) above.

Examples of the embodiments of the substituent acceptable in the "R² and R³ are combined with each other to form C₂₋₇ alkylene which may be substituted" include the groups shown in (a) to (h) above, in another embodiment, the groups shown in (a), (b), and (f) above, and oxo (=O), and in a further embodiment, the groups shown in (a) and (b) above, and oxo (=O).

Examples of the embodiments of the substituent acceptable in the "hetero ring group which may be substituted" in R¹⁰ include the groups shown in (a) to (i) above, and oxo (=O), in another embodiment, the groups shown in (a), (b), (f), and (i) above, and oxo (=O), and in a further embodiment, the groups shown in (i) above, and oxo (=O).

Examples of the embodiments of the substituent acceptable in the "lower alkyl which may be substituted" in R¹⁰¹ and R¹⁰² include the groups shown in (a) to (h) above, in another embodiment, the groups shown in (a) to (e) above, and oxo (=O), in a further embodiment, the groups shown in (b) above, and oxo (=O).

Examples of the embodiments of the substituent acceptable in the "lower alkyl which may be substituted" in R¹⁰³ include the groups shown in (a) to (h) above, in another embodiment, the groups shown in (g) and (h) above, and oxo (=O), and in a further embodiment, the groups shown in (g) above, and oxo (=O).

Examples of the embodiments of the substituent acceptable in the "lower alkyl which may be substituted" in R⁴, R⁵, R⁶, R⁷, R⁸, and R⁹ include the groups shown in (a) to (h) above, in another embodiment, the groups shown in (a) and (b) above, and oxo (=O), and in a further embodiment, the groups shown in (a) above, and oxo (=O).

Examples of the embodiments of the substituent acceptable in the "lower alkyl which may be substituted" in R¹¹, R¹², and R¹³ include the groups shown in (a) to (h) above, in another embodiment, the groups shown in (a) and (b) above, and oxo (=O), and in a further embodiment, the groups shown in (a) above, and oxo (=O).

Examples of the embodiments of the substituent acceptable in the "lower alkyl which may be substituted" in R^{N1} and R^{N2} include the groups shown in (a) to (h) above, in another embodiment, the groups shown in (a) and (b) above, and oxo (=O), and in a further embodiment, the groups shown in (a) above, and oxo (=O).

Examples of the embodiments of the substituent acceptable in the "lower alkyl which may be substituted" in R^{Y} include the groups shown in (a) to (h) above, in another embodiment, the groups shown in (a) and (b) above, and oxo (=O), and in a further embodiment, the groups shown in (a) above, and oxo (=O).

As an embodiment of the compound (I) of the present invention, a compound of the formula (I') or a salt thereof is shown. 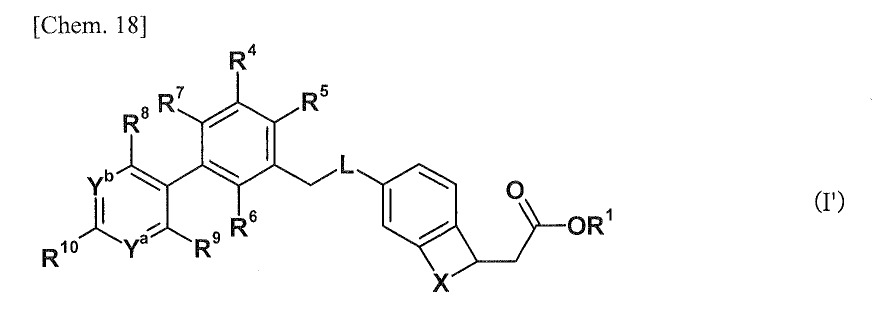 (wherein
L represents O or NH,
R¹ represents H or lower alkyl,
X represents 1,2-phenylene or -Z-C(R²)(R³)-,
Z represents O or CH₂,
R² and R³ are combined with each other to form C₂₋₇ alkylene,
R⁴, R⁵, R⁶, R⁷, R⁸, and R⁹ are the same as or different from each other and represent H, halogen, lower alkyl, or -O-lower alkyl,
R¹⁰ represents H, OH, -O-hetero ring group, or -O-(CR¹⁰¹R¹⁰²)ₙ-R¹⁰³,
R¹⁰¹ and R¹⁰² are the same as or different from each other and represent H, OH, halogen, or lower alkyl which may be substituted with OH, or
R¹⁰¹ and R¹⁰² are combined with each other to form oxo (=O),
n represents 1, 2, 3, or 4,
R¹⁰³ represents H, OH, halogen, NR^{N1}R^{N2}, -SO₂-lower alkyl, or -O-lower alkyl which may be substituted with aryl or oxo (=O), or a hetero ring group which may be substituted with lower alkyl or oxo (=O),
R^{N1} and R^{N2} are the same as or different from each other and represent H, -SO₂-lower alkyl, or lower alkyl which may be substituted with oxo (=O),
Y^{a} and Y^{b} are the same as or different from each other and represent N or C-R^{Y}, and
R^{Y} represents H, halogen, lower alkyl, or -O-lower alkyl).
Embodiments of the compounds (I) and (I') of the present invention are shown below.
(1) The compound, wherein R¹ is H, methyl, or ethyl.
(2) The compound, wherein R¹ is H.
(3) The compound, wherein X is 1,2-phenylene.
(4) The compound, wherein X is -Z-C(R²)(R³)-, and Z is CH₂.
(5) The compound, wherein X is -Z-C(R²)(R³)-, and Z is O.
(6) The compound, wherein R² and R³ are combined with each other to form C₂₋₇ alkylene.
(7) The compound, wherein R² and R³ are combined with each other to form ethylene.
(8) The compound, wherein R⁶ is lower alkyl.
(9) The compound, wherein R⁶ is methyl.
(10) The compound, wherein R⁴, R⁵, and R⁷ are H.
(11) The compound, wherein R⁸ and R⁹ are lower alkyl.
(12) The compound, wherein R⁸ and R⁹ are methyl.
(13) The compound, wherein R¹⁰ is H or -O-(CR¹⁰¹R¹⁰²)ₙ-R¹⁰³, R¹⁰¹ and R¹⁰² are the same as or different from each other and represent H, OH, or lower alkyl, n is 2, 3, or 4, and R¹⁰³ is OH, or -O-lower alkyl which may be substituted with aryl or oxo (=O).
(14) The compound, wherein R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, and R¹⁰³ is OH or methoxy.
(15) The compound, wherein Y^{a} and Y^{b} are C-R^{Y}, and R^{Y} is H.
   Furthermore, other embodiments of the compounds (I) and (I') of the present invention include the compound including combinations of two or more of the groups described in (1) to (15) above, specifically, the following compounds.
(16) The compound, wherein R¹ is H, methyl, or ethyl, X is 1,2-phenylene, R⁶ is lower alkyl, R⁴, R⁵, and R⁷ are H, R⁸ and R⁹ are lower alkyl, R¹⁰ is H or -O-(CR¹⁰¹R¹⁰²)ₙ-R¹⁰³, R¹⁰¹ and R¹⁰² are the same as or different from each other and represent H, OH, or lower alkyl, n is 2, 3, or 4, R¹⁰³ is OH, or -O-lower alkyl which may be substituted with aryl or oxo (=O), Y^{a} and Y^{b} are C-R^{Y}, and R^{Y} is H.
(17) The compound, wherein R¹ is H, methyl, or ethyl, X is 1,2-phenylene, R⁶ is methyl, R⁴, R⁵, and R⁷ are H, R⁸ and R⁹ are methyl, R¹⁰ is H or -O-(CR¹⁰¹R¹⁰²)ₙ₋R¹⁰³, R¹⁰¹ and R¹⁰² are the same as or different from each other and represent H, OH, or methyl, n is 2, 3, or 4, R¹⁰³ is OH or methoxy, Y^{a} and Y^{b} are C-R^{Y}, and R^{Y} is H.
(18) The compound, wherein R¹ is H, X is 1,2-phenylene, R⁶ is lower alkyl, R⁴, R⁵, and R⁷ are H, R⁸ and R⁹ are lower alkyl, R¹⁰ is H or -O-(CR¹⁰¹R¹⁰²)ₙ-R¹⁰³, R¹⁰¹ and R¹⁰² are the same as or different from each other and represent H, OH, or lower alkyl, n is 2, 3, or 4, R¹⁰³ is OH, or -O-lower alkyl which may be substituted with aryl or oxo (=O), Y^{a} and Y^{b} are C-R^{Y}, and R^{Y} is H.
(19) The compound, wherein R¹ is H, X is 1,2-phenylene, R⁶ is methyl, R⁴, R⁵, and R⁷ are H, R⁸ and R⁹ are methyl, and R¹⁰ is H or -O-(CR¹⁰¹R¹⁰²)ₙ-R¹⁰³, and R¹⁰¹ and R¹⁰² are the same as or different from each other and represent H, OH, or methyl, n is 2, 3, or 4, and R¹⁰³ is OH or methoxy, Y^{a} and Y^{b} are C-R^{Y}, and R^{Y} is H.
(20) The compound, wherein R¹ is H, methyl, or ethyl, X is -Z-C(R²)(R³)-, Z is CH₂, R² and R³ are combined with each other to form C₂₋₇ alkylene, R⁶ is lower alkyl, R⁴, R⁵, and R⁷ are H, R⁸ and R⁹ are lower alkyl, R¹⁰ is H or -O-(CR¹⁰¹R¹⁰²)ₙ-R¹⁰³, R¹⁰¹ and R¹⁰² are the same as or different from each other and represent H, OH, or lower alkyl, n is 2, 3, or 4, R¹⁰³ is OH, or -O-lower alkyl which may be substituted with aryl or oxo (=0), Y^{a} and Y^{b} are C-R^{Y}, and R^{Y} is H.
(21) The compound, wherein R¹ is H, methyl, or ethyl, X is -Z-C(R²)(R³)-, Z is CH₂, R² and R³ are combined with each other to form ethylene, R⁶ is methyl, R⁴, R⁵, and R⁷ are H, R⁸ and R⁹ are methyl, R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, R¹⁰³ is OH or methoxy, Y^{a} and Y^{b} are C-R^{Y}, and R^{Y} is H.
(22) The compound, wherein R¹ is H, X is -Z-C(R²)(R³)-, Z is CH₂, R² and R³ are combined with each other to form C₂₋₇ alkylene, R⁶ is lower alkyl, R⁴, R⁵, and R⁷ are H, R⁸ and R⁹ are lower alkyl, R¹⁰ is H or -O-(CR¹⁰¹R¹⁰²)ₙ-R¹⁰³ R¹⁰¹ and R¹⁰² are the same as or different from each other and represent H, OH, or lower alkyl, n is 2, 3, or 4, R¹⁰³ is OH, or -O-lower alkyl which may be substituted with aryl or oxo (=O), Y^{a} and Y^{b} are C-R^{Y}, and R^{Y} is H.
(23) The compound, wherein R¹ is H, X is -Z-C(R²)(R³)-, Z is CH₂, R² and R³ are combined with each other to form ethylene, R⁶ is methyl, R⁴, R⁵, and R⁷ are H, R⁸ and R⁹ are methyl, R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, R¹⁰³ is OH or methoxy, Y^{a} and Y^{b} are C-R^{Y}, and R^{Y} is H.
(24) The compound, wherein R¹ is H, methyl, or ethyl, X is -Z-C(R²)(R³)-, Z is O, R² and R³ are combined with each other to form C₂₋₇ alkylene, R⁶ is lower alkyl, R⁴, R⁵, and R⁷ are H, R⁸ and R⁹ are lower alkyl, and R¹⁰ is H or -O-(CR¹⁰¹R¹⁰²)ₙ-R¹⁰³, and R¹⁰¹ and R¹⁰² are the same as or different from each other and represent H, OH, or lower alkyl, n is 2, 3, or 4, R¹⁰³ is OH, or -O-lower alkyl which may be substituted with aryl or oxo (=O), Y^{a} and Y^{b} are C-R^{Y}, and R^{Y} is H.
(25) The compound, wherein R¹ is H, methyl, or ethyl, X is -Z-C(R²)(R³)-, Z is O, R² and R³ are combined with each other to form ethylene, R⁶ is methyl, R⁴, R⁵, and R⁷ are H, R⁸ and R⁹ are methyl, R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, R¹⁰³ is OH or methoxy, Y^{a} and Y^{b} are C-R^{Y}, and R^{Y} is H.
(26) The compound, wherein R¹ is H, X is -Z-C(R²)(R³)-, Z is O, R² and R³ are combined with each other to form C₂₋₇ alkylene, R⁶ is lower alkyl, R⁴, R⁵, and R⁷ are H, R⁸ and R⁹ are lower alkyl, R¹⁰ is H or -O-(CR¹⁰¹R¹⁰²)ₙ-R¹⁰³, R¹⁰¹ and R¹⁰² are the same as or different from each other and represent H, OH, or lower alkyl, n is 2, 3, or 4, R¹⁰³ is OH, or -O-lower alkyl which may be substituted with aryl or oxo (=O), Y^{a} and Y^{b} are C-R^{Y}, and R^{Y} is H.
(27) The compound, wherein R¹ is H, X is -Z-C(R²)(R³)-, Z is O, R² and R³ are combined with each other to form ethylene, R⁶ is methyl, R⁴, R⁵, and R⁷ are H, R⁸ and R⁹ are methyl, R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, R¹⁰³ is OH or methoxy, Y^{a} and Y^{b} are C-R^{Y}, and R^{Y} is H.
   Furthermore, other embodiments of the compounds (I) and (I') of the present invention include the following compounds.
(28) The compound, wherein R⁴, R⁵, R⁶, R⁷, R⁸, and R⁹ are the same as or different from each other and represent H or lower alkyl.
(29) The compound, wherein R⁴, R⁵, R⁶, R⁷, R⁸, and R⁹ are the same as or different from each other and represent H or methyl.
(30) The compound, wherein Y^{a} and Y^{b} are N.
(31) The compound, wherein Ya and Yb are C-R^{Y}.
(32) The compound, wherein L is O.
(33) The compound, wherein L is NH.
(34) The compound, wherein R¹⁰ is H or -O-(CR¹⁰¹R¹⁰²)ₙ-R¹⁰³.
(35) The compound, wherein R¹⁰¹ and R¹⁰² are the same as or different from each other and represent H, OH, or lower alkyl.
(36) The compound, wherein R¹⁰¹ and R¹⁰² are the same as or different from each other and represent H, OH, or methyl.
(37) The compound, wherein n is 2, 3, or 4.
(38) The compound, wherein R¹⁰³ is OH, or -O-lower alkyl which may be substituted with aryl or oxo (=O).
(39) The compound, wherein R¹⁰³ is OH or methoxy.
(40) The compound, wherein R¹¹, R¹², and R¹³ are H.
   Furthermore, other embodiments of the compounds (I) and (I') of the present invention include the compound including combinations of two or more of the groups described in (1) to (15) and (28) to (40) above, specifically, the following compounds.
(41) The compound as described in (28), wherein R⁶ is lower alkyl.
(42) The compound as described in (28) or (29), wherein R⁶ is methyl.
(43) The compound as described in (28) or (29), wherein R⁴, R⁵, and R⁷ are H.
(44) The compound as described in (28), wherein R⁸ and R⁹ are lower alkyl.
(45) The compound as described in (28) or (29), wherein R⁸ and R⁹ are methyl.
(46) The compound as described in any one of (8) to (12), (28) to (29), or (41) to (45), wherein R¹ is H, methyl, or ethyl.
(47) The compound as described in any one of (8) to (12), (28) to (29), or (41) to (45), wherein R¹ is H.
(48) The compound as described in any one of (8) to (12), (28) to (29), or (41) to (47), wherein X is 1,2-phenylene.
(49) The compound as described in any one of (8) to (12), (28) to (29), or (41) to (47), wherein X is -Z-C(R²)(R³)- and Z is CH₂.
(50) The compound as described in any one of (8) to (12), (28) to (29), or (41) to (47), wherein X is -Z-C(R²)(R³)- and Z is O.
(51) The compound as described in any one of (8) to (12), (28) to (29), (41) to (47), or (50), wherein R² and R³ are combined with each other to form C₂₋₇ alkylene.
(52) The compound as described in any one of (8) to (12), (28) to (29), (41) to (47), or (50), wherein R² and R³ are combined with each other to form ethylene.
(53) The compound as described in any one of (8) to (12), (28) to (29), or (41) to (52), wherein R¹⁰ is H or -O-(CR¹⁰¹R¹⁰²)ₙ-R¹⁰³_{.}
(54) The compound as described in any one of (8) to (12), (28) to (29), or (41) to (53), wherein R¹⁰¹ and R¹⁰² are the same as or different from each other and represent H, OH, or lower alkyl.
(55) The compound as described in any one of (8) to (12), (28) to (29), or (41) to (53), wherein R¹⁰¹ and R¹⁰² are the same as or different from each other and represent H, OH, or methyl.
(56) The compound as described in any one of (8) to (12), (28) to (29), or (41) to (55), wherein n is 2, 3, or 4.
(57) The compound as described in any one of (8) to (12), (28) to (29), or (41) to (56), wherein R¹⁰³ is OH, or -O-lower alkyl which may be substituted with aryl or oxo (=O).
(58) The compound as described in any one of (8) to (12), (28) to (29), or (41) to (56), wherein R¹⁰³ is OH or methoxy.
(59) The compound as described in any one of (8) to (12), (28) to (29), or (41) to (52), wherein R¹⁰ is H or -O-(CR¹⁰¹R¹⁰²)ₙ-R¹⁰³, R¹⁰¹ and R¹⁰² are the same as or different from each other and represent H, OH, or lower alkyl, n is 2, 3, or 4, and R¹⁰³ is OH, or -O-lower alkyl which may be substituted with aryl or oxo (=O).
(60) The compound as described in any one of (8) to (12), (28) to (29), or (41) to (52), wherein R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, and R¹⁰³ is OH or methoxy.
(61) The compound as described in any one of (8) to (12), (28) to (29), or (41) to (60), wherein Y^{a} and Y^{b} are N.
(62) The compound as described in any one of (8) to (12), (28) to (29), or (41) to (60), wherein Y^{a} and Y^{b} are C-R^{Y}.
(63) The compound as described in any one of (8) to (12), (28) to (29), or (41) to (60), wherein Y^{a} and Y^{b} are C-R^{Y}, and R^{Y} is H
(64) The compound as described in any one of (8) to (12), (28) to (29), or (41) to (63), wherein L is O.
(65) The compound as described in any one of (8) to (12), (28) to (29), or (41) to (63), wherein L is NH.
(66) The compound as described in any one of(1) to (39), or (41) to (65), wherein R¹¹, R¹², and R¹³ are H.

Examples of the specific compound encompassed by the present invention include:
(3-{[4'-(2-hydroxyethoxy)-2,2',6'-trimethylbiphenyl-3-yl]methoxy} -9H-fluoren-9-yl)acetic acid,
{5'-[(4'-{[(2R)-2,3-dihydroxypropyl]oxy}-2,2',6'-trimethylbiphenyl-3-yl)methoxy]-1',3'-dihydrospiro[cyclopropan-1,2'-inden]-1'-yl}acetic acid,
{5'-[(4'-{[(2S)-2,3-dihydroxypropyl]oxy}-2,2',6'-trimethylbiphenyl-3-yl)methoxy]-1',3'-dihydrospiro[cyclopropan-1,2'-inden]-1'-yl}acetic acid,
{3-[(2,2',6'-trimethylbiphenyl-3-yl)methoxy]-9H-fluoren-9-yl}acetic acid,
{3-[(4'-{[(2R)-2,3-dihydroxypropyl]oxy}-2,2',6'-trimethylbiphenyl-3-yl)methoxy]-9H-fluoren-9-yl}acetic acid,
{3-[(4'-{[(2S)-2,3-dihydroxypropyl]oxy}-2,2',6'-trimethylbiphenyl-3-yl)methoxy]-9H-fluoren-9-yl}acetic acid,
[5'-({[4'-(2-hydroxyethoxy)-2,2',6'-trimethylbiphenyl-3-yl]methyl}amino)-1',3'-dihydrospiro cyclopropan-1,2'-inden]-1'-yl]acetic acid,
(5'-{[(4'-{[(2R)-2,3-dihydroxypropyl]oxy} -2,2',6'-trimethylbiphenyl-3-yl)methyl]amino}-1',3'-dihydrospiro[cyclopropan-1,2'-inden]-1'-yl)acetic acid,
(5'-{[(4'-{[(2S)-2,3-dihydroxypropyl]oxy}-2,2',6'-trimethylbiphenyl-3-yl)methyl]amino}-1',3'-dihydrospiro[cyclopropan-1,2'-inden]-1'-yl)acetic acid,
[5'-({[4'-(3-hydroxy-3-methylbutoxy)-2,2',6'-trimethylbiphenyl-3-yl]methyl}amino)-1',3'-dihydrospiro[cyclopropan-1,2'-inden]-1'-yl]acetic acid,
(6-{[4'-(2-hydroxyethoxy)-2,2',6'-trimethylbiphenyl-3-yl]methoxy}-3H-spiro[1-benzofuran-2,1'-cyclopropan]-3-yl)acetic acid,
(6-{[4'-(3-hydroxy-3-methylbutoxy)-2,2',6'-trimethylbiphenyl-3-yl]methoxy}-3H-spiro[1-benzofuran-2,1'-cyclopropan]-3-yl)acetic acid,
{6-[(4'-{[(2R)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
{6-[(4'-{[(2S)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
[5'-({[4'-(2-methoxyethoxy)-2,2',6'-trimethylbiphenyl-3-yl]methyl}amino)-1',3'-dihydrospiro [cyclopropan-1,2'-inden]-1'-yl]acetic acid,
[5'-({3-[2-(2-hydroxyethoxy)-4,6-dimethylpyrimidin-5-yl]-2-methylbenzyl}oxy)-1',3'-dihydrospiro cyclopropan-1,2'-inden]-1'-yl]acetic acid,
[5'-({3-[2-(3-hydroxy-3-methylbutoxy)-4,6-dimethylpyrimidin-5-yl]-2-methylbenzyl}oxy)-1',3'-dihydrospiro[cydopropan-1,2'-inden]-1'-yl]acetic acid,
[(1'S)-5'-({[4'-(2-hydroxyethoxy)-2,2',6'-trimethylbiphenyl-3-yl]methyl}amino)-1',3'-dihydrospiro[cyclopropan-1,2'-inden]-1'-yl]acetic acid,
[(1'R)-5'-({[4'-(2-hydroxyethoxy)-2,2',6'-trimethylbiphenyl-3-yl]methyl}amino)-1',3'-dihydrospiro[cyclopropan-1,2'-inden]-1'-yl]acetic acid,
(6-{[4'-(2-methoxyethoxy)-2,2',6'-trimethylbiphenyl-3-yl]methoxy}-3H-spiro[1-benzofuran-2,1'-cyclopropan]-3-yl)acetic acid,
{6-[(4'-{[(3R)-3,4-dihydroxybutyl]oxy}-2,2',6'-trirnethylbiphenyl-3-yl)methoxy]-3H-spiro[1-benzofuran-2,1'-cyclopropan]-3-yl}acetic acid,
{6-[(4'-{[(3S)-3,4-dihydroxybutyl]oxy}-2,2',6'-trimethylbiphenyl-3-yl)methoxy]-3H-spiro[1-benzofuran-2,1'-cyclopropan]-3-yl acetic acid,
(6-{[4'-(2-ethoxyethoxy)-2,2',6'-trimethylbiphenyl-3-yl]methoxy}-3H-spiro[1-benzofuran-2,1'-cyclopropan]-3-yl)acetic acid,
(6-{[4'-(3-methoxypropoxy)-2,2',6'-trimethylbiphenyl-3-yl]methoxy}-3H-spiro[1-benzofuran-2,1'-cyclopropan]-3-yl)acetic acid,
[(9S)-3-{[4'-(2-hydroxyethoxy)-2,2',6'-trimethylbiphenyl-3-yl]methoxy}-9H-fluoren-9-yl]acetic acid,
[(9R)-3-{[4'-(2-hydroxyethoxy)-2,2',6'-trimethylbiphenyl-3-yl]methoxy}-9H-fluoren-9-yl]acetic acid,
[(3R)-6-{[4'-(2-methoxyethoxy)-2,2',6'-trimethylbiphenyl-3-yl]methoxy}-3H-spiro[1-benzofuran-2,1'-cyclopropan]-3-yl]acetic acid,
[(3S)-6-{[4'-(2-methoxyethoxy)-2,2',6'-trimethylbiphenyl-3-yl]methoxy}-3H-spiro[1-benzofuran-2,1'-cyclopropan]-3-yl]acetic acid,
[(3R)-6-{[4'-(3-hydroxy-3-methylbutoxy)-2,2',6'-trimethylbiphenyl-3-yl]methoxy}-3H-spiro[1-benzofuran-2,1'-cyclopropan]-3-yl)acetic acid,
[(3S)-6-{[4'-(3-hydroxy-3-methylbutoxy)-2,2',6'-trimethylbiphenyl-3-yl]methoxy}-3H-spiro[1-benzofuran-2,1'-cyclopropan]-3-yl]acetic acid,
[(3R)-6-{ [4'-(2-hydroxyethoxy)-2,2',6'-trimethylbiphenyl-3-yl]methoxy}-3H-spiro[1-benzofuran-2,1'-cyclopropan]-3-yl]acetic acid,
[(3S)-6-{[4'-(2-hydroxyethoxy)-2,2',6'-trimethylbiphenyl-3-yl]methoxy}-3H-spiro[1-benzofuran-2,1'-cyclopropan]-3-yl]acetic acid,
{(3R)-6-[(4'-{[(2R)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
{(3S)-6-[(4'-{[(2R)-2,3-dihydroxypropyl]oxy} -2,2',6'-trimethylbiphenyl-3-yl)methoxy]-3H-spiro[1-benzofuran-2,1'-cyclopropan]-3-yl acetic acid,
{(3R)-6-[(4'-{[(2S)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
{(3S)-6-[(4'-{[(2S)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
[(1'S)-5'-({[4'-(2-methoxyethoxy)-2,2',6'-trimethylbiphenyl-3-yl]methyl} amino)-1',3'-dihydrospiro[cyc1opropan-1,2'-inden]-1'-yl]aceti acid,
[(1'R)-5'-({[4'-(2-methoxyethoxy)-2,2',6'-trimethylbiphenyl-3-yl]methyl}amino)-1',3'-dihydrospiro[cyclopropan-1,2'-inden]-1'-yl]acetic acid,
{(3R)-6-[(4'-{[(3S)-3,4-dihydroxybutyl]oxy}-2,2',6'-trimethylbiphenyl-3-yl)methoxy]-3H-spiro[1-benzofuran-2,1'-cyclopropan]-3-yl}acetic acid,
{(3S)-6-[(4'-{[(3S)-3,4-dihydroxybutyl]oxy}-2,2',6'-trimethylbiphenyl-3-yl)methoxy]-3H-spiro[1-benzofuran-2,1'-cyclopropan]-3-yl}acetic acid,
{(3R)-6-[(4'-{[(3R)-3,4-dihydroxybutyl]oxy}-2,2',6'-trimethylbiphenyl-3-yl)methoxy]-3-H-spiro[1-benzofuran-2,1'-cyclopropan]-3-yl}acetic acid, or
{(3S)-6-[(4'-{[(3R)-3,4-dihydroxybutyl]oxy}-2,2',6'-trimethylbiphenyl-3-yl)methoxy]-3H-spiro[1-benzofuran-2,1'-cyclopropan]-3-yl}acetic acid.

The compound of the formula (I) may exist in the form of tautomers or geometrical isomers depending on the kind of substituents. In the present specification, the compound of the formula (I) shall be described in only one form of isomer, yet the present invention includes such an isomer, isolated forms of the isomers, or a mixture thereof.
In addition, the compound of the formula (I) may have asymmetric carbon atoms or axial asymmetry in some cases, and correspondingly, it may exist in the form of optical isomers. The present invention includes both an isolated form of the optical isomers of the compound of the formula (I) or a mixture thereof.

Moreover, the present invention also includes a pharmaceutically acceptable prodrug of the compound of the formula (I). The pharmaceutically acceptable prodrug is a compound having a group that can be converted into an amino group, a hydroxyl group, a carboxyl group, or the like through solvolysis or under physiological conditions. Examples of the group forming the prodrug include the groups described in Prog. Med., 5, 2157-2161 (1995) and Pharmaceutical Research and Development, Drug Design, Hirokawa Publishing Company (1990), Vol. 7, 163-199.

Furthermore, the salt of the compound of the formula (I) is a pharmaceutically acceptable salt of the compound of the formula (I) and may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or organic bases such as methylamine, ethylamine, ethanolamine, lysine, ornithine, and the like, salts with various amino acids or amino acid derivatives such as acetylleucine and the like, ammonium salts, etc.

In addition, the present invention also includes various hydrates or solvates, and polymorphic crystalline substances of the compound of the formula (I) and a salt thereof. In addition, the present invention also includes compounds labeled with various radioactive or non-radioactive isotopes.

Furthermore, in the present specification, the following symbols are used.
Pr: Preparation Example No.,
Ex: Example No.,
Data: physicochemical data,
FAB+: represening an m/z value in FAB-MS (positive ion), and representing a [M+H]+ peak unless otherwise specified,
FAB-: represening an m/z value in FAB-MS (negative ion), and representing a [M-H]- peak unless otherwise specified,
ESI+: represening an m/z value in ESI-MS (positive ion), and representing a [M+H]+ peak unless otherwise specified,
ESI-: represening an m/z value in ESI-MS (negative ion), and representing a [M-H]- peak unless otherwise specified,
EI: represening an m/z value in EI-MS (positive ion), and representing a M+ peak unless otherwise specified,
NMR1: δ (ppm) of peak in ¹H NMR in DMSO-d₆,
NMR2: δ (ppm) of peak in ¹H NMR in CDCl₃,
Structure: Structural Formula (*: the compounds have steric isomers due to presence of an asymmetric carbon, in which the absolute configuration is not determined),
TBDMS: tert-Butyldimethylsilyl,
NMP: N-Methyl-2-pyrrolidone,
DMSO: Dimethylsulfoxide,
THF: Tetrahydrofuran,
EtOAc: Ethyl acetate,
DMF: N,N-Dimethylformamide,
CDI: Carbonyldiimidazole,
DBU: Diazabicycloundecene.

### (Preparation Methods)

The compound of the formula (I) and a salt thereof can be prepared by using the characteristics based on the basic structure or the type of substituents thereof and by applying various known synthesis methods. During the preparation, replacing the relevant functional group with a suitable protective group (a group that can be easily converted into the functional group) at the stage from starting material to an intermediate may be effective depending on the type of the functional group in the production technology in some cases. The protective group for such a functional group may include, for example, the protective groups described in "Greene's Protective Groups in Organic Synthesis (4th Ed., 2006)", P. G. M. Wuts and T. W. Greene, and one of these may be selected and used as necessary depending on the reaction conditions. In this kind of method, a desired compound can be obtained by introducing the protective group, by carrying out the reaction and by eliminating the protective group as necessary.
In addition, the prodrug of the compound of the formula (I) can be produced by introducing a specific group at the stage from a starting material to an intermediate or by carrying out the reaction using the obtained compound of the formula (I), just as in the case of the above-mentioned protective group. The reaction can be carried out using methods known to those skilled in the art, such as ordinary esterification, amidation, dehydration, and the like.
Hereinafter, the representative preparation methods for the compound of the formula (I) will be described. Each of the production processes may also be carried out with reference to the References appended in the present description. Further, the preparation methods of the compound of the formula (I) are not limited to the examples as shown below.

### (Production Process 1)

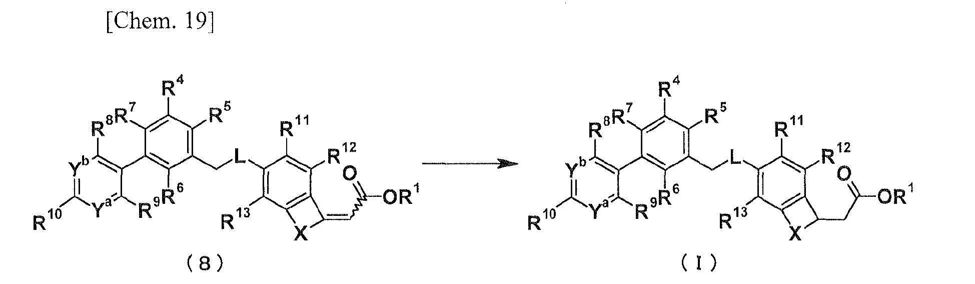

The compound (I) of the present invention can be obtained by subjecting a compound (8) to a hydrogenation reaction.
In this reaction, the compound (8) is stirred usually for 1 hour to 5 days, under a hydrogen atmosphere in a solvent which is inert to the reaction in the presence of a metal catalyst. This reaction is usually carried out under any temperature condition from cooling to heating, and preferably at room temperature. Examples of the solvent as used herein are not particularly limited, but include alcohols such as methanol, ethanol, 2-propanol, and the like, ethers such as diethyl ether, tetrahydrofuran, dioxane, dimethoxyethane, and the like, water, ethyl acetate, N,N-dimethylformamide, dimethylsulfoxide, and a mixture thereof. As the metal catalyst, palladium catalysts such as palladium on carbon, palladium black, palladium hydroxide, and the like, platinum catalysts such as a platinum plate, platinum oxide, and the like, nickel catalysts such as reduced nickel, Raney nickel, and the like, rhodium catalysts such as tetrakistriphenylphosphine chlororhodium, and the like, or iron catalysts such as reduced iron and the like are suitably used. Instead of hydrogen gas, formic acid, ammonium formate, or the like in an equivalent amount or an excess amount, relative to the compound (8), can be used as a hydrogen source.
Furthermore, the present reaction may also be carried out by bringing the compound (8) into contact with magnesium in the presence of methanol. This reaction is usually carried out under any temperature condition from cooling to heating, and preferably at room temperature. Examples of the solvent as used herein are not particularly limited, but include alcohols such as methanol, ethanol, 2-propanol, and the like, ethers such as diethyl ether, tetrahydrofuran, dioxane, dimethoxyethane, and the like, water, ethyl acetate, N,N-dimethylformamide, dimethylsulfoxide, and a mixture thereof.

### (References)

"Reductions in Organic Chemistry, 2nd Ed. (ACS Monograph: 188)", M. Hudlicky, ACS, 1996
"Courses in Experimental Chemistry (5th Ed.)", edited by The Chemical Society of Japan, Vol. 19 (2005) (Maruzen)

### (Production Process 2)

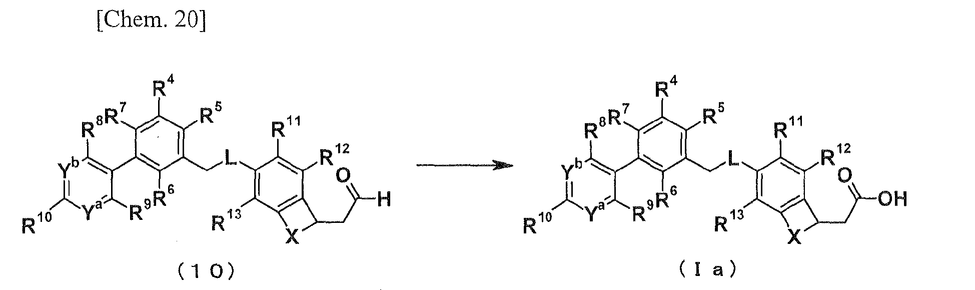

A compound (Ia) in which R¹=H, among the compounds (I) of the present invention, can be obtained by subjecting a compound (10) to an oxidation reaction.
In this reaction, the compound (10) is treated with an equivalent amount or an excess amount of an oxidant under any temperature condition from cooling to heating, and preferably -20°C to 80°C, usually for 0.1 hours to 3 days, in a solvent which is inert to the reaction. Examples of the solvent as used herein are not particularly limited, but include ethers such as diethyl ether, tetrahydrofuran, dioxane, dimethoxyethane, and the like, halogenated hydrocarbons such as dichloromethane, 1,2-dichloroethane, chloroform, and the like, aromatic hydrocarbons such as benzene, toluene, xylene, and the like, N,N-dimethylformamide, dimethylsulfoxide, ethyl acetate, water, and a mixture thereof. As the oxidant, sodium hypochlorite, hydrogen peroxide, cumene hydroperoxide, peracetic acid, perbenzoic acid, m-chloroperbenzoic acid, Oxone (registered trademark), activated manganese dioxide, chromic acid, potassium permanganate, or sodium peroxoate is suitably used. In addition, in the case where sodium hydrochlorite is used as an oxidant, the reaction may be in some cases advantageously carried out in the presence of an acid such as sodium dihydrogen phosphate and the like, using a compound such as 2-methyl-2-butene so as to capture a chlorine compound in the reaction system.

### (References)

"Comprehensive Organic Synthesis", B. M. Trost, Vol. 7, 1991
"Oxidation in Organic Chemistry (ACS Monograph: 186)", M. Hudlicky, ACS, 1990
"Courses in Experimental Chemistry (5th Ed.)", edited by The Chemical Society of Japan, Vol. 17 (2005) (Maruzen)

### (Production Process 3)

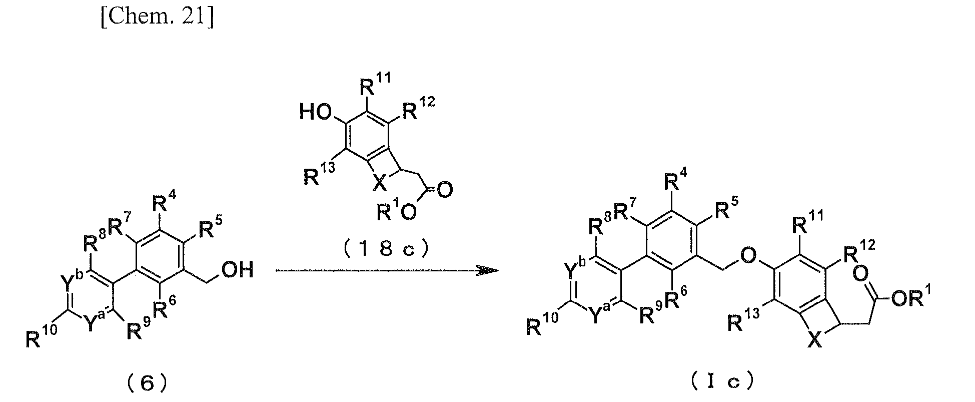

A compound (Ic), wherein L is 0, among the compounds (I) of the present invention, can be obtained by subjecting a compound (6) and a compound (18c) to a Mitsunobu reaction.
In this reaction, a compound (6) is treated with an equivalent amount or an excess amount of (18c) under any temperature condition from cooling to heating, and preferably -20°C to 80°C, usually for 0.1 hours to 3 days, in a solvent which is inert to the reaction, in the presence of an azo compound and a phosphorous compound. Examples of the solvent as used herein are not particularly limited, but include ethers such as diethyl ether, tetrahydrofuran, dioxane, dimethoxyethane, and the like, halogenated hydrocarbons such as dichloromethane, 1,2-dichloroethane, chloroform, and the like, aromatic hydrocarbons such as benzene, toluene, xylene, and the like, N,N-dimethylformamide, dimethylsulfoxide, and a mixture thereof. As the azo compound, 1,1'-(azodicarbonyl)dipiperidine, diethyl azodicarboxylate, or diisopropyl azodicarboxylate can be used, and as the phosphorous compound, for example, tributylphosphine, or triphenylphosphine is suitably used. Further, instead of the azo compound and the phosphorous compound, for example, a phosphorous ylide such as (cyanomethylene)trimethylphosphorane, (cyanomethylene)tributylphosphorane, and the like can also be used.

### (Production Process 4)

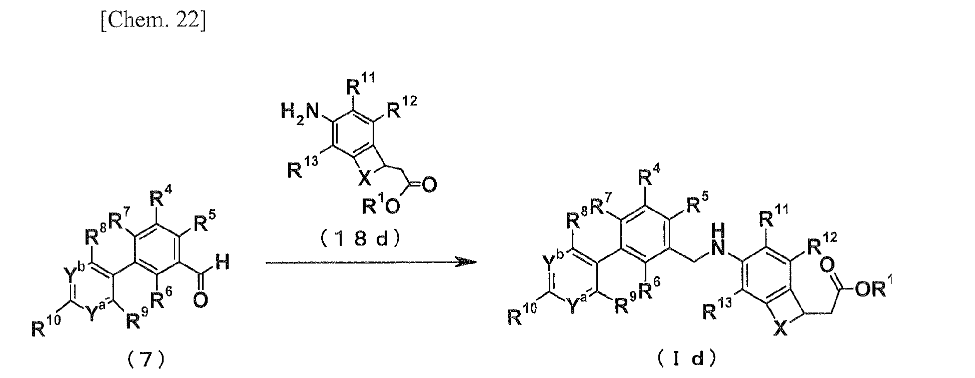

The compound of the present invention (Id) can be obtained by reacting a compound (7) with a compound (18d).
In this reaction, the compound (7) and the compound (18d) in equivalent amounts, or with either thereof in an excess amount are used, and a mixture thereof is stirred under any temperature condition from -45°C to heating and refluxing, and preferably at 0°C to 80°C, usually for 0.1 hours to 5 days, in a solvent which is inert to the reaction, in the presence of a reducing agent. Examples of the solvent as used herein are not particularly limited, but include halogenated hydrocarbons such as dichloromethane, 1,2-dichloroethane, chloroform, and the like, alcohols such as methanol, ethanol, and the like, ethers such as diethyl ether, tetrahydrofuran, dioxane, dimethoxyethane, and the like, N,N-dimethylformamide, dimethylsulfoxide, and a mixture thereof. Examples of the reducing agent include sodium cyanoborohydride, sodium triacetoxyborohydride, sodium borohydride, and the like. It is preferable in some cases to carry out the reaction in the presence of a dehydrating agent such as molecular sieves, and the like or an acid such as acetic acid, hydrochloric acid, a titanium (IV) isopropoxide complex, and the like. According to the reaction, an imine produced by condensation of the compound (7) and the compound (18d) may be isolated as a stable intermediate in some cases. In such a case, the imine intermediate is produced, and isolated, as necessary, and then subjected to a reduction reaction to obtain a compound (Id). Further, instead of treatment with such a reducing agent, the reaction can also be carried out using a reduction catalyst (for example, palladium on carbon, Raney nickel, and the like) in a solvent such as methanol, ethanol, ethyl acetate, and the like, in the presence or absence of an acid such as acetic acid, hydrochloric acid, and the like. In this case, it is preferable to carry out the reaction under a hydrogen atmosphere from normal pressure to 50 atmospheres under any temperature condition from cooling to heating.

### (References)

"Comprehensive Organic Functional Group Transformations II", A. R. Katritzky and R. J. K. Taylor, Vol. 2, Elsevier Pergamon, 2005
"Courses in Experimental Chemistry (5th Ed.)", edited by The Chemical Society of Japan, Vol. 14 (2005) (Maruzen)

### (Other Production Processes)

Furthermore, several substituents in the formula (I) can also be easily converted into other functional groups by using the compound of the present invention (I) as a starting material by means of the reactions apparent to a person skilled in the art, or modified methods thereof. The reaction can be carried out by any combination of the processes that can be usually employed by a person skilled in the art, such as hydrolysis, alkylation, halogenation, hydrogenation, and the like. Several examples thereof are presented below. Further, in R¹⁰, a compound having a dihydroxy group can be obtained by hydrolyzing a compound having a methylenedioxy group or a dimethylmethylenedioxy group.

### (Production Process 5)

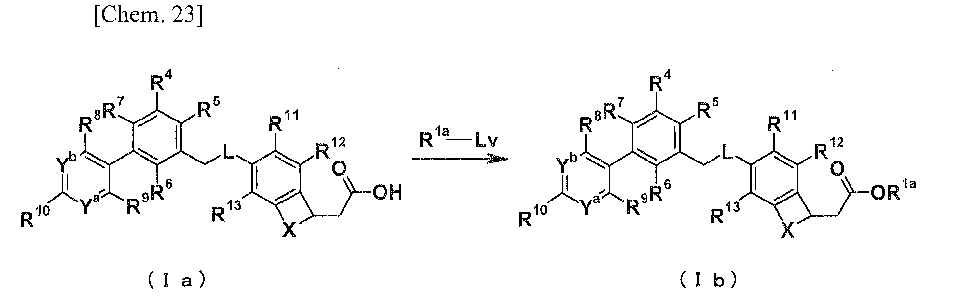

(wherein Lv represents a leaving group).
The compound (Ib) of the present invention can be obtained by reacting a compound (Ia) with R^{1a}-Lv. Here, examples of the leaving group include halogen, a methanesulfonyloxy group, a p-toluenesulfonyloxy group, and the like.
In this reaction, the compound (Ia) and an equivalent amount or an excess amount of R^{1a}-Lv are used, and a mixture thereof is stirred under any temperature condition from cooling to heating and refluxing, and preferably at 0°C to 80°C, usually for 0.1 hours to 5 days in a solvent which is inert to the reaction or without a solvent. The solvent as used herein is not particularly limited, but examples thereof include aromatic hydrocarbons such as benzene, toluene, xylene, and the like, ethers such as diethyl ether, tetrahydrofuran, dioxane, dimethoxyethane, and the like, halogenated hydrocarbons such as dichloromethane, 1,2-dichloroethane, chloroform, and the like, N,N-dimethylformamide, dimethylsulfoxide, ethyl acetate, acetonitrile, and a mixture thereof. It may be advantageous in some cases for the smooth progress of the reaction to carry out the reaction in the presence of an organic base such as triethylamine, N,N-diisopropylethylamine, N-methylmorpholine, and the like, or an inorganic base such as cesium carbonate, potassium phosphate, potassium carbonate, sodium carbonate, potassium hydroxide, and the like.
Furthermore, the reaction may be carried out using a catalyst which is not particularly limited, but includes catalysts used for an Ullmann reaction, a Buchwald-Hartwig reaction, or the like. The catalyst as used herein is not particularly limited, but a suitable combination of tris(dibenzylideneacetone)palladium, tetrakis(triphenylphosphine) palladium, or the like with 4,5-bis(diphenylphosphino)-9,9'-dimethylxanthene (Xantphos), 2-dicyclohexylphosphino-2',6'-dimethoxybiphenyl (SPhos), 2-dicyclohexylphosphino-2',4',6'-triisopropylbiphenyl (XPhos), and the like can be used.
Moreover, the reaction can also be carried out in the presence of a condensing agent. Examples of the condensing agent as used herein are not not particularly limited, but dicyclohexylcarbodiimide, diisopropylcarbodiimide, 1-ethyl-3-(3-dimethylaminopropyl)carbodiimide hydrochloride, or the like can be used.

### (References)

"Organic Functional Group Preparations", S. R. Sandler and W. Karo, 2nd Ed, Vol. 1, Academic Press Inc., 1991
"Courses in Experimental Chemistry (5th Ed.)", edited by The Chemical Society of Japan, Vol. 14 (2005) (Maruzen)

### (Starting Material Synthesis 1)

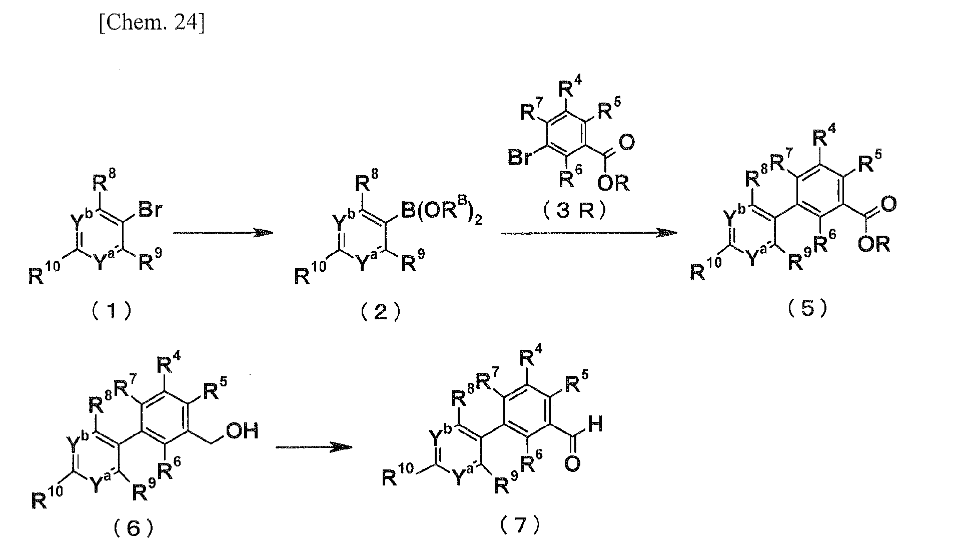

(wherein R represents lower alkyl, R^{B} represents H or lower alkyl, or two R^{B,}s are combined with each other to form C₂₋₇ alkylene).
A compound (7) can be prepared from the compound (1).
First, the compound (2) can be obtained by subjecting the compound (1) to a boronate ester-synthesizing reaction.
In this reaction, a mixture of the compound (1) and a boronate ester-synthesizing reagent in equivalent amounts, or with either thereof in an excess amount is stirred under any temperature condition from cooling to heating, and preferably -20°C to 60°C, usually for 0.1 hours to 5 days, in a solvent which is inert to the reaction, in the presence of an organometallic compound. The solvent as used herein is not particularly limited, but examples thereof include aromatic hydrocarbons such as benzene, toluene or xylene, and the like, halogenated hydrocarbons such as dichloromethane, 1,2-dichloroethane, chloroform, and the like, ethers such as diethyl ether, tetrahydrofuran, dioxane, dimethoxyethane, and the like, DMF, DMSO, EtOAc, acetonitrile, water, and a mixture thereof. Examples of the boronate ester-synthesizing reagent include triisopropyl borate, tributyl borate, and the like. Examples of the organometallic compound used in the present reaction include organic lithium compounds such as n-butyllithium and the like.
Furthermore, a compound in which R^{B} is H, among the compounds (2), can be obtained by subjecting the compound (2) to a hydrolysis reaction with reference to Reference, P. G. M. Wuts, et al.
Moreover, the compound (5) can be obtained by subjecting the compound (2) and the compound (3R) to a coupling reaction.
In this reaction, a mixture of the compound (2) and an equivalent amount or an excess amount of the compound (3R) is stirred under any temperature condition from cooling to heating and refluxing, and preferably at 0°C to 80°C, usually for 0.1 hours to 5 days, in a solvent which is inert to the reaction or without a solvent. The solvent as used herein is not particularly limited, but examples thereof include aromatic hydrocarbons such as benzene, toluene, xylene, and the like, ethers such as dimethyl ether, diethyl ether, tetrahydrofuran, dioxane, dimethoxyethane, and the like, halogenated hydrocarbons such as dichloromethane, 1,2-dichloroethane, chloroform, and the like, N,N-dimethylformamide, dimethylsulfoxide, ethyl acetate, acetonitrile, and a mixture thereof. It may be advantageous in some cases for the smooth progress of the reaction to carry out the reaction in the presence of an organic base such as triethylamine, N,N-diisopropylethylamine, N-methylmorpholine, and the like, or an inorganic base such as potassium carbonate, sodium carbonate, potassium phosphate, potassium hydroxide, and the like.
Furthermore, the reaction may be carried out using a catalyst which is not particularly limited, but includes catalysts used for a Suzuki-Miyaura cross-coupling reaction. The catalyst as used herein is not particularly limited, but tetrakis(triphenylphosphine)palladium(0), palladium(II) acetate, dichloro[1,1'-bis(diphenylphosphenylphosphino)ferrocene]palladium(II), bistriphenylphosphinepalladium(II) chloride, or the like can be used. In addition, metal palladium(0) can also be used to carry out the coupling reaction.
The compound (6) can be obtained by subjecting the compound (5) to a reduction reaction.
In this reaction, the compound (5) is treated with an equivalent amount or an excess amount of a reducing agent under any temperature condition from cooling to heating, and preferably at -20°C to 80°C, usually for 0.1 hours to 3 days, in a solvent which is inert to the reaction. Examples of the solvent as used herein are not particularly limited, but include ethers such as diethyl ether, tetrahydrofuran, dioxane, dimethoxyethane, and the like, alcohols such as methanol, ethanol, 2-propanol, and the like, aromatic hydrocarbons such as benzene, toluene, xylene, and the like, N,N-dimethylformamide, dimethylsulfoxide, ethyl acetate, and a mixture thereof. As the reducing agent, a hydrogenation reducing agent such as lithium aluminum hydride, sodium borohydride, diisobutyl aluminum hydride, and the like, a metal reducing agent such as sodium, zinc, iron, platinum, and the like, or another reducing agent in the following References is suitably used.
Finally, the compound (7) can be prepared by subjecting the compound (6) to an oxidation reaction. Here, the oxidation reaction can be carried out using the reaction conditions described in (Production Process 2). Further, for the present reaction, DMSO oxidation such as Swern oxidation and the like or oxidation using a Dess-Martin reagent is suitably used.

### (References)

"Reductions in Organic Chemistry, 2nd Ed. (ACS Monograph: 188)", M. Hudlicky, ACS, 1996
"Comprehensive Organic Transformations", R. C. Larock, 2nd Ed., VCH Publishers, Inc., 1999
"Oxidation and Reduction in Organic Synthesis (Oxford Chemistry Primers 6)", T. J. Donohoe, Oxford Science Publications, 2000
"Courses in Experimental Chemistry (5th Ed.)", edited by The Chemical Society of Japan, Vol. 14 (2005) (Maruzen)

### (Starting Material Synthesis 2)

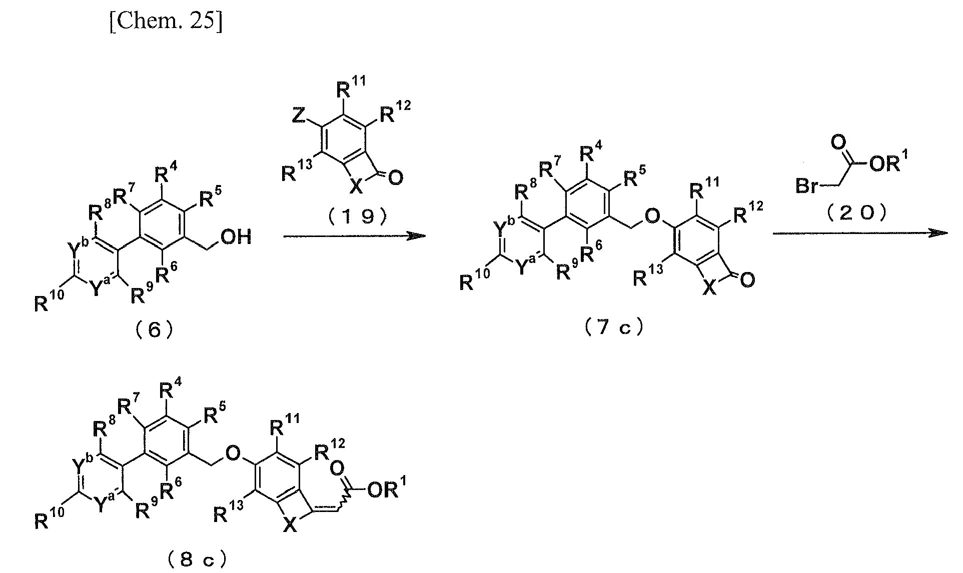

A compound (8c) can be prepared from the compound (6).
First, the compound (7c) can be obtained by subjecting the compound (6) to a substitution reaction. In this reaction, the compound can be prepared by the method described in (Production Process 2) of (Other Production Processes).
Next, the compound (8c) can be obtained by subjecting the compound (7c) to a Reformatsky reaction.
In this reaction, the compound (7c) and an equivalent amount or an excess amount of the compound (20) are used, and a mixture thereof is stirred under any temperature condition from cooling to heating and refluxing, preferably at 0°C to 200°C, and still more preferably at 20°C to 120°C, usually for 0.1 hours to 5 days, in a solvent which is inert to the reaction or without a solvent, in the presence of zinc powder. The solvent as used herein is not particularly limited, but examples thereof include aromatic hydrocarbons such as benzene, toluene, xylene, and the like, ethers such as diethyl ether, tetrahydrofuran, dioxane, dimethoxyethane, and the like, halogenated hydrocarbons such as dichloromethane, 1,2-dichloroethane, chloroform, and the like, N,N-dimethylformamide, dimethylsulfoxide, and a mixture thereof. Further, the zinc powder and the compound (20) may also be treated in advance, and then used as a Reformatsky reagent in the reaction.

### (References)

"Organic Functional Group Preparations", S. R. Sandler and W. Karo, 2nd Ed., Vol. 1, Academic Press Inc., 1991
"Courses in Experimental Chemistry (5th Ed.)", edited by The Chemical Society of Japan, Vol. 14 (2005) (Maruzen)
Synthesis 2006, 4, 629-632

### (Starting Material Synthesis 3)

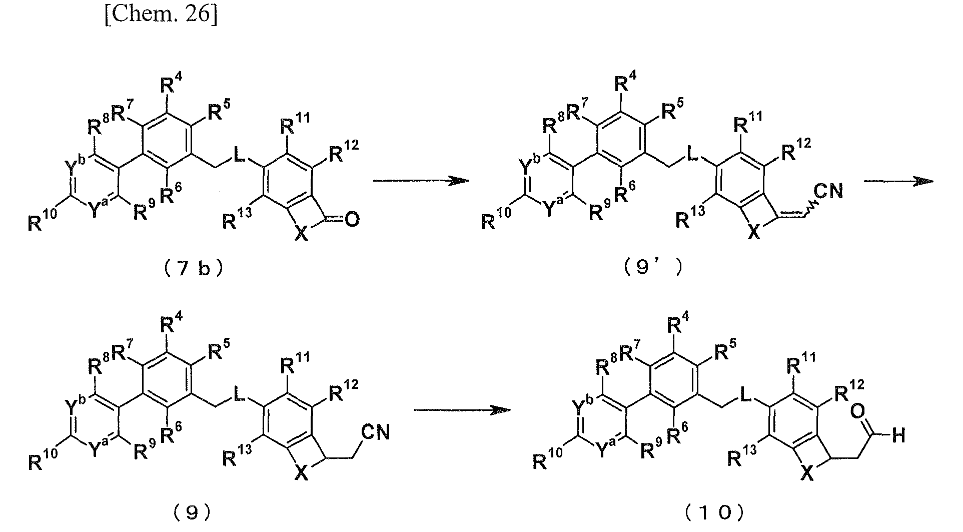

A compound (10) can be prepared from a compound (7b).
First, a compound (9) can be obtained by subjecting the compound (7b) to a coupling reaction and a hydrogenation reaction. Here, the coupling reaction can be carried out under the reaction conditions described in (Starting Material Synthesis 4) as described later, and the hydrogenation reaction can be carried out using the reaction conditions described in the aforementioned (Production Process 1).
Next, the compound (10) can be obtained by subjecting the compound (9) to a reduction reaction. In the present reaction, a hydrogenation reducing agent such as lithium aluminum hydride, sodium borohydride, diisobutyl aluminum hydride, and the like, a metal reducing agent such as sodium, zinc, iron, platinum, and the like, or a reducing agent in the References described in the aforementioned (Starting Material Synthesis 1).

### (Starting Material Synthesis 4)

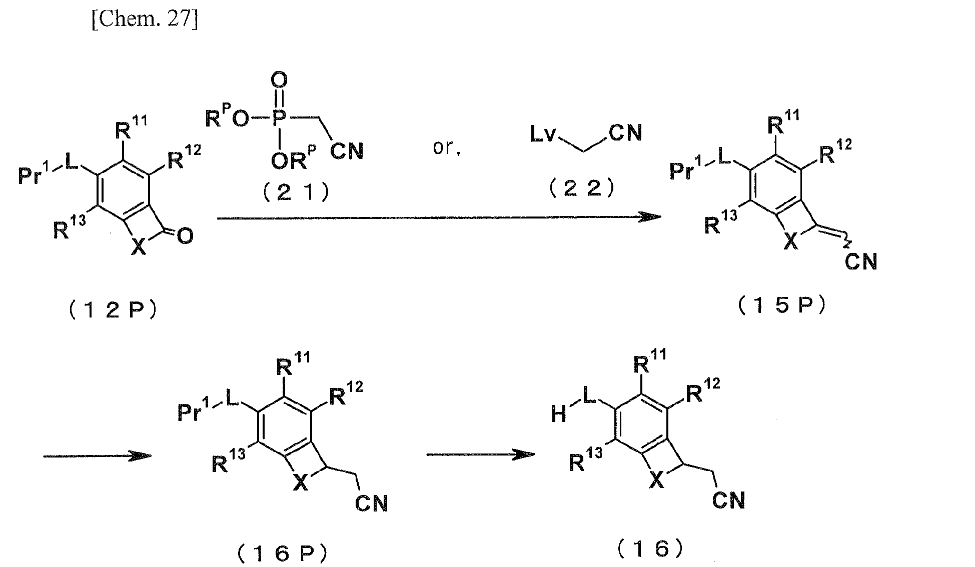

(wherein Pr¹ represents a protecting group and R^{p} represents lower alkyl).
A compound (16) can be prepared from a compound (12P).
First, a compound (15P) can be obtained by subjecting the compound (12P) and a phosphoric ester to a coupling reaction. The present reaction may be carried out by a Horner-Emmons reaction or a Wittig reaction although it is not particularly limited.
In this reaction, the compound (12P) is treated under any temperature condition from cooling to heating, and preferably -20°C to 80°C, usually for 0.1 hours to 3 days, in a solvent which is inert to the reaction, in the presence of an equivalent amount or an excess amount of a phosphoric ester compound (21). Examples of the solvent as used herein are not particularly limited, but include ethers such as diethyl ether, tetrahydrofuran, dioxane, dimethoxyethane, and the like, aromatic hydrocarbons such as benzene, toluene, xylene, and the like, N,N-dimethylformamide, dimethylsulfoxide, and a mixture thereof. It may be advantageous in some cases for the smooth progress of the reaction to carry out the reaction in the presence of a base such as sodium bis(trimethylsilyl)amide, n-butyllithium, potassium tert-butoxide, sodium ethoxide, sodium methoxide, sodium hydride, and the like. Examples of the phosphoric ester compound (21) include diethyl (cyanomethyl)phosphate and the like. Further, the present reaction may also be carried out using the compound (22) in the presence of a phosphorous compound instead of the phosphoric ester compound (21). As the phosphorous compound, an alkyltriphenylphosphonium salt is suitably used, and more specific examples thereof include (methoxymethyl)triphenylphosphonium chloride, (methylthiomethyl)triphenylphosphonium, and the like.
Next, the compound (16) can be obtained by subjecting the compound (15P) to a hydrogenation reaction and a deprotection reaction. Here, the hydrogenation reaction can be carried out with reference to the reaction conditions described in the preparation method (Production Process 1) and the deprotection reaction can be carried out with reference to the References such as the aforementioned P. G. M. Wuts, et al.

### (Starting Material Synthesis 5)

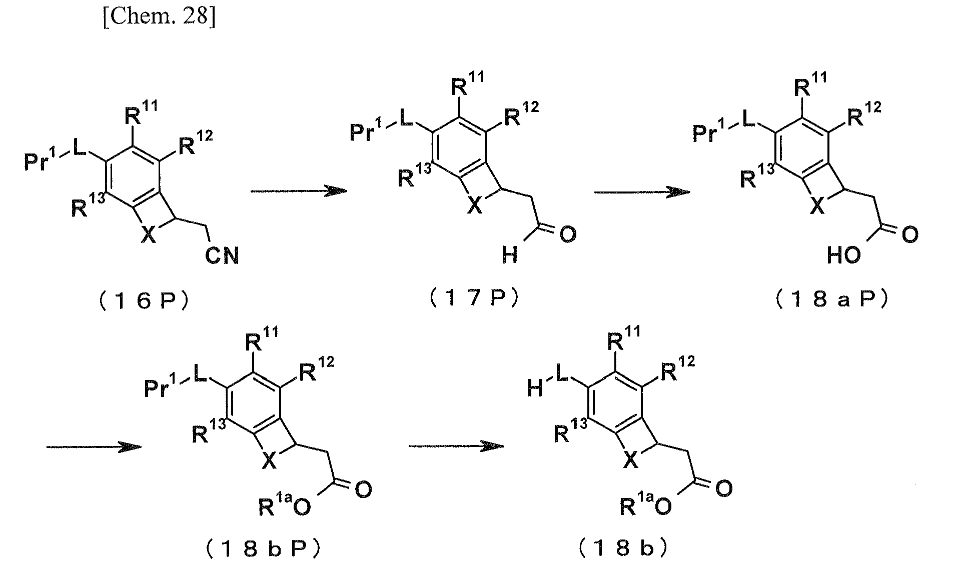

A compound (18b) can be obtained by subjecting a compound (16P) to a reduction reaction, an oxidation reaction, a substitution reaction, and a deprotection reaction. For the reduction reaction, the reaction conditions described in (Starting Material Synthesis 1) can be used; for the oxidation reaction, the reaction conditions described in (Production Process 2) can be used; and for the substitution reaction, the reaction conditions described in (Production Process 5) can be used.

The compounds of the formula (I) can be isolated and purified as their free compounds, salts, hydrates, solvates, or polymorphic crystalline substances thereof. The salts of the compound of the formula (I) can be prepared by carrying out the treatment of a conventional salt forming reaction.
Isolation and purification are carried out by employing ordinary chemical operations such as extraction, fractional crystallization, various types of fractional chromatography, and the like.
Various isomers can be prepared by selecting an appropriate starting compound or separated by using the difference in the physicochemical properties between the isomers. For example, the optical isomers can be obtained by means of a general method for designing optical resolution of racemic products (for example, fractional crystallization for inducing diastereomer salts with optically active bases or acids, chromatography using a chiral column or the like, and others), and further, the isomers can also be prepared from an appropriate optically active starting compound.

The pharmacological activity of the compound of the formula (I) was confirmed by the tests shown below.

### Test Method 1: Measurement of GPR40 Agonistic Activity

### i) Cloning of Human GPR40

A full-length sequence of GPR40 was obtained by PCR method using human genomic DNA (Clontech) as a template in accordance with the procedure shown below.
An oligonucleotide consisting of the base sequence represented by SEQ ID NO: 1 was used as the forward primer, and an oligonucleotide consisting of the base sequence represented by SEQ ID NO: 2 as the reverse primer. In this connection, a base sequence comprising a XbaI recognition region was added to the respective 5'-termini of the aforementioned forward primer and reverse primer. PCR was carried out in the presence of 5% dimethylsulfoxide (DMSO) using a Taq DNA polymerase (Ex Taq DNA polymerase; Takara Bio), by repeating 30 times of a cycle consisting of 94°C (15 seconds)/55°C (30 seconds)/72°C (1 minute). As a result, a DNA fragment of about 0.9 kbp was amplified. This DNA fragment was digested with XbaI and then inserted into the XbaI site of a plasmid pEF-BOS-dhfr (Nucleic acids Research, 18, 5322, 1990), thereby obtaining a plasmid pEF-BOS-dhfr-GPR40.
The base sequence of the GPR40 gene in the pEF-BOS-dhfr-GPR40 was determined by the dideoxy terminator method using a DNA sequencer (ABI 377 DNA Sequencer, Applied Biosystems). The base sequence of the GPR40 gene was represented by the base sequence SEQ ID NO: 3. The base sequence represented by SEQ ID NO: 3 had an open reading frame (ORF) of 903 bases, and the amino acid sequence deduced from this ORF (300 amino acids) was represented by the amino acid sequence SEQ ID NO: 4.

### ii) Preparation of GPR40 Stable Expression Cell

As the cell for expressing GPR40 protein, a CHO dhfr cell (a dihydrofolate reductase (dhfr) gene-deficient CHO cell) was used. Also, as the plasmid for expressing GPR40 protein, the plasmid pEF-BOS-dhfr-GPR40 obtained in the aforementioned i) was used. The CHO dhfr cell was inoculated into an αMEM medium containing 10% fetal calf serum (FCS) using a 6 well plate (Asahi Techno Glass) and cultured overnight to a confluence of 80 to 90%, and then 2 µg per well of the plasmid pEF-BOS-dhfr-GPR40 was gene-transferred using a transfection reagent (Lipofectamine 2000; Invitrogen). After 24 hours of culturing from the gene transfer, the cells were diluted and inoculated again. In this time, the αMEM medium containing 10% FCS was changed to an αMEM medium which contained 10% FCS but did not contain nucleic acid. After 20 days of culturing, the formed colonies of cells were individually recovered and cultured to obtain CHO cells stably expressing GPR40. From these, cells having high reactivity for intrinsic ligands oleic acid and linoleic acid were selected.

### iii) Measurement of GPR40 Agonistic Activity

The present test was measured by FLIPR (registered trademark, Molecular Devices) using a change in intracellular calcium concentration as the index. Hereinafter, the test method will be shown.
A CHO cell strain in which human GPR40 was expressed was inoculated into a 384 well black plate (Becton Dickinson) at 6×10³ cells per well portion and cultured overnight in a CO₂ incubator.
Using a Calcium-3 assay kit (Molecular Devices), one bottle of the phosphorescent pigment was dissolved in 10 ml of HBSS-HBEPES buffer (pH 7.4, 1×HBSS, 20 mM HEPES, Invitrogen). 35.68 mg of Probenecid (Sigma) was dissolved in 250 µ1 of 1 M NaOH and adjusted by adding 250 µl of the HBSS-HEPES buffer. A phosphorescent pigment solution was prepared by mixing 16 ml of HBSS-HEPES buffer, 640 µl of the phosphorescent pigment and 32 µl of probenecid per one plate. The medium was discarded from the plate, and the phosphorescent pigment solution was dispensed at 40 µl per well portion and then incubated at room temperature for 2 hours. Each compound to be tested was dissolved in DMSO and then diluted with HBSS-HEPES buffer and dispensed in 10 µl portions into the plate, thereby starting the reaction, and changes in the intracellular calcium concentration were measured by FLIPR. The EC₅₀ value of each compound to be tested was calculated by a dose-response curve of changes in fluorescence intensity after 1 minute of the measurement.
As a result, the compound of the present invention exhibits a GPR40 agonistic activity. The EC₅₀ values of the representative compounds of the compound of the present invention are shown in Table 1. Ex denotes the Example Compound No. as described later.

**[Table 1]**

| Ex | EC₅₀ (µM) | Ex | EC₅₀ (µM) | Ex | EC₅₀ (µM) |
|---|---|---|---|---|---|
| 2 | 0.52 | 21 | 0.81 | 23 | 0.52 |
| 2-2 | 0.79 | 21-1 | 0.75 | 25 b | 0.21 |
| 4 | 0.25 | 21-2 | 0.81 | 25-3 a | 0.56 |
| 6 | 0.78 | 21-3 | 0.71 | 25-3 b | 0.19 |
| 8 | 0.61 | 22 | 0.25 | 25-5 a | 0.35 |
| 8-3 | 0.61 | 22-1 | 0.27 | 25-5 b | 0.26 |
| 8-12 | 0.59 | 22-2 | 0.43 | 25-7 a | 0.49 |
| 20 | 0.49 | 22-3 | 0.47 | 25-7 b | 0.33 |

### Test Method 2: Insulin Secretion-Promoting Action Using MIN6 Cell

The present test was to examine the insulin secretion promoting action of a test compound using a mouse pancreas β cell strain, MIN6 cell. Hereinafter, the test method will be shown.
The MIN6 cell was dispensed in 5×10⁴ cells/well (200 µl) portions into a 96 well plate. DMEM (25 mM glucose) containing 10% FBS, 55 µM 2-mercaptoethanol, 100 U/ml penicillin and 100 µg/ml streptomycin was used as the medium. The medium was discarded 2 days thereafter using an aspirator, followed by washing once with 200 µl of KRB-HEPES (116 mM NaCl, 4.7 mM KCl, 1.2 mM KH₂PO₄, 1.2 mM MgSO₄, 0.25 mM CaCl₂; 25 mM NaHCO₃, 0.005% FFA Free BSA, 24 mM HEPES (pH 7.4)) containing 2.8 mM glucose, which was warmed up to 37°C, and subsequent incubation again at 37°C for 1 hour by adding 200 µl of the same buffer. After discarding the above-mentioned buffer using an aspirator and again washing with the buffer (200 µl), a predetermined concentration of a compound to be tested was added to the KRB-HEPES containing 2.8 mM or 22.4 mM glucose and added to respective wells in 100 µl portions and incubated at 37°C for 2 hours. The above-mentioned samples were fractioned and diluted 100 times, and the insulin concentration was determined using an insulin RIA kit (Amersham RI).
As a result, it was confirmed that the compound of the present invention has an excellent insulin secretion promoting action.

### Test Method 3: Normal Mice Single Oral Glucose Tolerance Test

The present test was to examine the blood glucose increase inhibiting action of the test compound after glucose loading, using normal mice. Hereinafter, the test method will be shown.
Male ICR mice (6 weeks of age) after 1 week of acclimatization were subjected to overnight fasting and used as test animals. The test compound was made into a 0.01 M aqueous sodium hydroxide solution and orally administered at a dose of 10 mg/kg 30 minutes before the glucose loading (2 g/kg). A 0.01 M aqueous sodium hydroxide solution was administered to the control group. The blood glucose increase inhibitory ratio (%) after 30 minutes of glucose loading was calculated, relative to the control group.
The test results of the representative compounds are shown in Table 2. Ex denotes the Example Compound No. as described later. As a result, it was confirmed that the compound of the present invention has an excellent blood glucose increase inhibiting action.

**[Table 2]**

| Ex | Blood glucose increase inhibition rate (%) | Ex | Blood glucose increase inhibition rate (%) | Ex | Blood glucose increase inhibition rate (%) |
|---|---|---|---|---|---|
| 2 | 37 | 8-12 | 24 | 22 | 34 |
| 2-2 | 34 | 20 | 40 | 22-1 | 35 |
| 4 | 20 | 21 | 43 | 22-2 | 34 |
| 6 | 35 | 21-1 | 30 | 22-3 | 38 |
| 8 | 25 | 21-2 | 23 | 23 | 20 |
| 8-3 | 21 | 21-3 | 32 | 25 b | 33 |

### (Comparative Experiment)

When the compound of Example 72 described in Pamphlet of International Publication W02005/087710 was orally administered at 10 mg/kg by the same method as the test method 3 above, the blood glucose increase inhibition rate was measured and found to be 9%, whereas when the compound was orally administered at 30 mg/kg, the blood glucose increase inhibition rate was 20%. On the other hand, among the compounds of the present invention, there were the compounds exhibiting a blood glucose increase inhibiting action of 20% or more when orally administered at 0.3 mg/kg. Therefore, it became apparent that the compound of the present invention effectively exhibits a blood glucose increase inhibiting action at a low dose, as compared with the corresponding compounds. Further, in the present test, a minimum administration amount showing a blood glucose increase inhibition rate of 20% or more or a minimum administration amount showing a significance (Dunnet multiple comparison test) relative to a control was taken as a minimum effective dose (MED).

As described above, it was confirmed that the compound of the formula (I) has an excellent GPR40 agonistic activity, and thus has effects of a potent insulin secretion promoting action and a blood glucose increase inhibiting action. The compound can be therefore used as an insulin secretion promoter or an agent for preventing/treating diabetes.

A pharmaceutical composition containing one or two or more kinds of the compound of the formula (I) or a salt thereof as an active ingredient can be prepared in accordance with a generally used method, using a pharmaceutical carrier, an a pharmaceutical excipient, a pharmaceutical carrier, or the like, that is usually used in the art.
The administration can be carried out through any mode of oral administration via tablets, pills, capsules, granules, powders, liquid preparations, or the like, or parenteral administration via injections such as intraarticular, intravenous, intramuscular, or others, suppositories, eye drops, eye ointments, transdermal liquid preparations, ointments, transdermal patches, transmucosal liquid preparations, transmucosal patches, inhalers, and the like.

The solid composition for use in the oral administration according to the present invention is used in the form of tablets, powders, granules, or the like. In such a solid composition, one or more active ingredient(s) are mixed with at least one inactive excipient. According to a conventional method, the composition may contain inactive additives, such as a lubricant, a disintegrating agent such as and the like, a stabilizer, or a solubilization assisting agent. If necessary, tablets or pills may be coated with sugar or a film of a gastric or enteric coating substance.
The liquid composition for oral administration contains pharmaceutically acceptable emulsions, solutions, suspensions, syrups, elixirs, or the like, and also contains generally used inert diluents, for example, purified water or ethanol. In addition to the inert diluent, the liquid composition may also contain auxiliary agents, such as a solubilization assisting agent, a moistening agent, and a suspending agent, sweeteners, flavors, aromatics, and antiseptics.

The injections for parenteral administration include sterile aqueous or non-aqueous solution preparations, suspensions and emulsions. The aqueous solvent includes, for example, distilled water for injection and physiological saline. Examples of the non-aqueous solvent include alcohols such as ethanol. Such a composition may further contain a tonicity agent, an antiseptic, a moistening agent, an emulsifying agent, a dispersing agent, a stabilizing agent, or a solubilizing aid. These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, poultices, sprays, lotions, eye drops, eye ointments, and the like. The agents contain generally used ointment bases, lotion bases, aqueous or non-aqueous liquid preparations, suspensions, emulsions, and the like.

As the transmucosal agents such as an inhaler, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate ejection agent, for example, a suitable gas such as chlorofluoroalkane, hydrofluoroalkane, carbon dioxide, and the like, or other forms.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

The compound of the formula (I) can be used in combination with various therapeutic or prophylactic agents for the diseases, in which the compound of the formula (I) is considered effective, as described above. The combined preparation may be administered simultaneously or separately and continuously, or at a desired time interval. The preparations to be co-administered may be a blend or prepared individually.

Hereinbelow, the preparation methods for the compound of the formula (I) will be described in more detail with reference to Examples. Further, the present invention is not limited to the preparation methods of the specific Examples and Preparation Examples shown below, but the compound of the formula (I) can be prepared by any combination of such the preparation methods or the methods that are apparent to a person skilled in the art.

### Preparation Example 1

Under nitrogen air flow, to a solution of 4-bromo-3,5-dimethylphenol (150.00 g) in acetonitrile (1200 mL) was added potassium carbonate (257.80 g). Subsequently, chloromethyl methyl ether (68 mL) was added dropwise thereto, followed by stirring at room temperature for 1 hour. Next, to the reaction mixture was added potassium carbonate (25.80 g), followed by stirring at room temperature for 15 minutes. Subsequently, to the reaction mixture was added dropwise chloromethyl methyl ether (5.6 mL), followed by stirring at room temperature for 1.5 hours. Finally, to the reaction mixture was added dropwise chloromethyl methyl ether (2.8 mL) at room temperature, followed by stirring at room temperature for 0.5 hours. The reaction mixture was filtered and washed with acetonitrile. The filtrate was concentrated under reduced pressure, and the resulting residue was diluted with diethyl ether, and then washed with a 1 M aqueous sodium hydroxide solution and a saturated aqueous sodium chloride solution. The organic layer was separated, dried over anhydrous magnesium sulfate, and filtered to remove the desiccant, and the solvent was evaporated under reduced pressure to obtain 2-bromo-5-(methoxymethoxy)-1,3-dimethylbenzene (180.30 g) as a pale yellow solid.

In the same manner as in the method of Preparation Example 1, the compound of Preparation Example 1-1 shown in Tables below was prepared.

### Preparation Example 2

Under nitrogen air flow, to a solution of 2-bromo-5-(methoxymethoxy)-1,3-dimethylbenzene (124.36 g) in THF (845 mL) was added dropwise a 1.55 M n-butyllithium solution in hexane (360 mL) under cooling in a dry ice-acetone bath, followed by stirring at the same temperature for 0.5 hours. Next, to the reaction mixture was added dropwise a solution of triisopropyl borate (135 mL) in THF (150 mL), followed by stirring at the same temperature for 0.5 hours. The dry ice-acetone bath was removed, followed by stirring for 1 hour while slowly warming to about 5°C. To the reaction mixture was added a saturated aqueous ammonium chloride solution (400 mL), followed by stirring at room temperature for 1 hour. The reaction mixture was concentrated under reduced pressure, and to the resulting residue were added water (300 mL) and heptane (200 mL), followed by stirring at room temperature for 5 minutes. Thereafter, the mixture was stirred for 0.5 hours under ice-cooling, and the solid was collected by filtration and washed with water (100 mL) and heptane (100 mL). The resulting solid was heated and dried under reduced pressure to obtain [4-(methoxymethoxy)-2,6-dimethylphenyl]boronic acid (100.53 g) as a white solid.

### Preparation Example 3

To a mixture of 3-bromo-2-methylbenzoic acid (112.0 g) and methanol (1000 mL) was added concentrated sulfuric acid (31 mL) under stirring. This reaction mixture was stirred for 22 hours under heating and refluxing. The solvent was evaporated under reduced pressure, and the resulting residue was adjusted to pH 7 to 8 by adding a saturated aqueous sodium hydrogen carbonate solution (110 mL) and sodium hydrogen carbonate (50 g) portionwise. Water (200 mL) was added thereto, followed by extraction with ethyl acetate. The organic layer was washed with water and a saturated aqueous sodium chloride solution, and then dried over anhydrous magnesium sulfate. The desiccant was removed by filtration, and the solvent was evaporated under reduced pressure to obtain methyl 3-bromo-2-methylbenzoate (116.4 g) as a pale yellow solid.

### Preparation Example 4

A mixture of {5'-[(tert-butoxycarbonyl)amino]-1',3'-dihydrospiro[cyclopropan-1,2'-inden]-1'-yl}acetic acid (56.25 g), potassium hydrogen carbonate (20.00 g), methyl iodide (12.7 mL), and DMF (850 mL) was stirred at room temperature for 4 hours. To the reaction mixture were added potassium hydrogen carbonate (5.30 g) and methyl iodide (3.3 mL), followed by stirring at room temperature for 2 hours. To the reaction mixture was added acetic acid (10 mL), followed by stirring at room temperature for 0.5 hours. The solvent was evaporated under reduced pressure, and then to the resulting residue was added water (1000 mL), followed by extraction with a toluene-ethyl acetate solution. The organic layer was washed with water and a saturated aqueous sodium chloride solution, and then dried over anhydrous magnesium sulfate. The desiccant was removed by filtration, and the solvent was evaporated under reduced pressure. The resulting residue was purified by silica gel column chromatography (hexane-ethyl acetate) to obtain methyl {5'-[(tert-butoxycarbonyl)amino]-1',3'-dihydrospiro[cyclopropan-1,2'-inden]-1'-yl}acetate (26.50 g) as a pale brown gummy syrup.

### Preparation Example 5

Under nitrogen air flow, [4-(methoxymethoxy)-2,6-dimethylphenyl]boronic acid (86.00 g), methyl 3-bromo-2-methylbenzoate (86.00 g), tripotassium phosphate (239.07 g), dicyclohexyl(2',6'-dimethoxybiphenyl-2-yl)phosphine (1.55 g), and palladium(II) acetate (0.85 g) were mixed, and then toluene (1290 mL) and water (129 mL) were added thereto. The reaction mixture was warmed to 70°C, followed by stirring at the same temperature for 2 hours. The reaction mixture was cooled to room temperature, and water (300 mL) was added thereto, followed by filtration through Celite and addition of ethyl acetate for liquid-separation. The organic layer was washed with a saturated aqueous sodium chloride solution, and then dried over anhydrous magnesium sulfate. The desiccant was removed by filtration and the solvent was evaporated under reduced pressure. The resulting residue was purified by silica gel column chromatography (hexane-toluene-ethyl acetate) to obtain methyl 4'-(methoxymethoxy)-2,2',6'-trimethylbiphenyl-3-carboxylate (105.93 g) as a pale yellow crystal.

In the same manner as in the method of Preparation Example 5, the compounds of Preparation Examples 5-1 to 5-2 shown in Tables below were prepared.

### Preparation Example 6

A mixture of 5-bromo-2-(2-{[tert-butyl(dimethyl)silyl]oxy}ethoxy)-4,6-dimethylpyrimidine (7.21 g), methyl 2-methyl-3-(4,4,5,5-tetramethyl-1,3,2-dioxaborolan-2-yl)benzoate (6.10 g), palladium(II) acetate (242 mg), dicyclohexyl(2',6'-dimethoxybiphenyl-2-yl)phosphine (848 mg), tripotassium phosphate (12.7 g), toluene (100 mL), and water (10 mL) was stirred at 80°C for 24 hours under a nitrogen atmosphere. The reaction mixture was cooled to room temperature, then water and ethyl acetate were added thereto, and the insoluble materials were removed by filtration through Celite. The filtrate was subjected to liquid-separation, and then the aqueous layer was extracted with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain methyl 3-[2-(2-{[tert-butyl(dimethyl)silyl]oxy}ethoxy)-4,6-dimethylpyrimidin-5-yl]-2-methylbenzoate (7.30 g) as a pale yellow oil.

In the same manner as in the method of Preparation Example 6, the compounds of Preparation Examples 6-1 to 6-4 shown in Tables below were prepared.

### Preparation Example 7

Under nitrogen air flow, to THF (300 mL) was added lithium aluminum hydride (4.00 g) under ice-cooling, and then a solution of methyl 4'-(methoxymethoxy)-2,2',6'-trimethylbiphenyl-3-carboxylate (25.00 g) in THF (100 mL) was added dropwise thereto. The reaction mixture was stirred for 10 minutes under ice-cooling, and then the ice-bath was removed. The mixture was stirred for 40 minutes while warming to room temperature. To the reaction mixture was added sodium sulfate decahydrate (35.00 g) portionwise under ice-cooling, and then the ice-bath was removed. The mixture was stirred for 0.5 hours while warming to room temperature. The insoluble materials were separated by filtration through Celite, and then the solvent was evaporated under reduced pressure. The resulting residue was purified by silica gel column chromatography (hexane-ethyl acetate) to obtain [4'-(methoxymethoxy)-2,2',6'-trimethylbiphenyl-3-yl]methanol (21.88 g) as a colorless gummy syrup.

In the same manner as in the method of Preparation Example 7, the compounds of Preparation Examples 7-1 to 7-6 shown in Tables below were prepared.

### Preparation Example 8

To a mixture of [4'-(methoxymethoxy)-2,2',6'-trimethylbiphenyl-3-yl]methanol (1.13 g), 3-fluoro-9H-fluoren-9-one (785 mg), and DMF (10 mL) was added sodium hydride (about 40% of mineral oil added, 210 mg), followed by stirring at room temperature for 1 hour, and then further stirring at 50°C for 1 hour. To the reaction mixture was added water (40 mL), followed by extraction with ethyl acetate, and then the organic layer was washed with a saturated aqueous sodium chloride solution and dried over anhydrous magnesium sulfate. The desiccant was removed by filtration and the solvent was evaporated under reduced pressure. The resulting residue was purified by silica gel column chromatography (hexane-ethyl acetate) to obtain 3-{[4'-(methoxymethoxy)-2,2',6'-trimethylbiphenyl-3-yl]methoxy}-9H-fluoren-9-one (1.36 g) as a yellow amorphous solid.

In the same manner as in the method of Preparation Example 8, the compounds of Preparation Examples 8-1 to 8-6 shown in Tables below were prepared.

### Preparation Example 9

Under nitrogen air flow, to a solution of 3-{[4'-(methoxymethoxy)-2,2',6'-trimethylbiphenyl-3-yl]methoxy}-9H-fluoren-9-one (1.35 g) in THF (20 mL) was added zinc powder (570 mg), and then about 2 mL of a soltuion of ethyl bromoacetate (0.78 mL) in THF (10 mL) was added thereto. This mixture was stirred at 80°C for 10 minutes, and then the whole remaining amount of the solution of ethyl bromoacetate (0.78 mL) in THF (10 mL) was added dropwise thereto. After completion of dropwise addition, the mixture was stirred for 45 minutes while slowly leaving to be cooled to room temperature. To the reaction mixture was added 1 M hydrochloric acid (10 mL), followed by stirring at room temperature for 1.5 hours, and further stirring at 50°C for 1 hour. To the reaction mixture was added 1 M hydrochloric acid (10 mL), followed by further stirring for 1 hour. The reaction mixture was extracted with ethyl acetate, and then this organic layer was washed with a saturated aqueous sodium chloride solution and dried over anhydrous magnesium sulfate. The desiccant was removed by filtration and the solvent was evaporated under reduced pressure. The resulting residue was purified by silica gel column chromatography (hexane-ethyl acetate) to obtain ethyl (3-{[4'-(methoxymethoxy)-2,2',6'-trimethylbiphenyl-3-yl]methoxy}-9H-fluoren-9-ylidene)acetate (1.22 g) as a yellow amorphous solid.

In the same manner as in the method of Preparation Example 9, the compounds of Preparation Examples 9-1 to 9-2 shown in Tables below were prepared.

### Preparation Example 10

A mixture of ethyl (3-{[4'-(methoxymethoxy)-2,2',6'-trimethylbiphenyl-3-yl]methoxy}-9H-fluoren-9-ylidene)acetate (1.20 g), 1 M hydrochloric acid (5 mL), ethanol (10 mL), and THF (2 mL) was stirred at room temperature for 1.5 hours, and then concentrated hydrochloric acid (1 mL) was added thereto, followed by stirring at room temperature for 16 hours and then at 50°C for 4 hours. To the reaction mixture was added water, followed by extraction with ethyl acetate, and then the organic layer was washed with a saturated aqueous sodium chloride solution and dried over anhydrous magnesium sulfate. The desiccant was removed by filtration and the solvent was evaporated under reduced pressure. To the resulting yellow amorphous solid (1.17 g) were added DMF (10 mL), 2-bromoethyl acetate (0.37 mL), and cesium carbonate (1.6 g), followed by stirring at 50°C for 4 hours. To the reaction mixture was added water (50 mL), followed by extraction with a toluene-ethyl acetate solution, and then the organic layer was washed with a saturated aqueous sodium chloride solution and dried over anhydrous magnesium sulfate. The desiccant was removed by filtration and the solvent was evaporated under reduced pressure. The resulting residue was purified by silica gel column chromatography (hexane-ethyl acetate) to obtain ethyl (3-{[4'-(2-acetoxyethoxy)-2,2',6'-trimethylbiphenyl-3-yl]methoxy}-9H-fluoren-9-ylidene)acetate (950 mg) as a yellow oil.

### Preparation Example 11

A mixture of 4-bromo-3,5-dimethylphenol (50.00 g), 3,4-dihydro-2H-pyran (47.00 mL), pyridine 4-methylbenzene sulfonate (12.00 g), and dichloromethane (500 mL) was stirred at room temperature for 17.5 hours. The solvent was evaporated under reduced pressure, and to the residue was added water, followed by extraction with ethyl acetate. The organic layer was washed with water and a saturated aqueous sodium chloride solution, and then dried over anhydrous magnesium sulfate. The desiccant was removed and the solvent was evaporated under reduced pressure. The resulting residue was purified by silica gel column chromatography (hexane-ethyl acetate) to obtain 2-(4-bromo-3,5-dimethylphenoxy)tetrahydro-2H-pyran (67.57 g) as a colorless oil.

### Preparation Example 12

Under nitrogen air flow, a solution of 2-(4-bromo-3,5-dimethylphenoxy)tetrahydro-2H-pyran(67.57 g) in THF (850 mL) was cooled in a dry ice-acetone bath. A 1.66 M n-butyllithium solution in hexane (160 mL) was added dropwise thereto, followed by stirring at the same temperature for 1.5 hours. To the reaction mixture was added a solution of 2-isopropoxy-4,4,5,5-tetramethyl-1,3,2-dioxaborolane (55 mL) in THF (150 mL). The dry ice-acetone bath was removed, followed by stirring for 2 hours while warming to room temperature. The solvent was evaporated under reduced pressure, and to the residue was added water (400 mL), followed by extraction with ethyl acetate (500 mL). The organic layer was washed with a saturated aqueous sodium chloride solution (300 mL), and then dried over anhydrous magnesium sulfate. The desiccant was removed by filtration and the solvent was evaporated under reduced pressure. To the residue was added methanol (75 mL), followed by stirring for 0.5 hours under cooling in an ice-methanol bath. The solid was collected by filtration and dried under reduced pressure to obtain 2-[3,5-dimethyl-4-(4,4,5,5-tetramethyl-1,3,2-dioxaborolan-2-yl)phenoxy]tetrahydro-2H-pyran (58.53 g) as a white solid. Further, the filtrate was concentrated under reduced pressure and the resulting residue was purified by silica gel column chromatography (hexane-ethyl acetate) to obtain 2-[3,5-dimethyl-4-(4,4,5,5-tetramethyl-1,3,2-dioxaborolan-2-yl)phenoxy]tetrahydro-2H-pyran (9.16 g) as a white solid.

### Preparation Example 13

Under a nitrogen atmosphere, a mixture of methyl 3-bromo-2-methylbenzoate (53.00 g), 4,4,4',4',5,5,5',5'-octamethyl-2,2'-bi-1,3,2-dioxaborolane (88.10 g), bistriphenylphosphine palladium chloride (8.12 g), triphenylphosphine (6.07 g), potassium acetate (68.10 g), and dioxane (530 mL) was heated and stirred at 100°C for 29 hours, and then cooled to room temperature. The reaction mixture was filtered through Celite and washed with ethyl acetate. The resulting filtrate was concentrated under reduced pressure and the residue was purified by silica gel column chromatography (hexane-ethyl acetate) to obtain methyl 2-methyl-3-(4,4,5,5-tetramethyl-1,3,2-dioxaborolan-2-yl)benzoate (54.00 g) as a colorless oil.

### Preparation Example 14

To a solution of [2,2',6'-trimethyl-4'-(tetrahydro-2H-pyran-2-yloxy)biphenyl-3-yl]methanol (35.35 g) in chloroform (300 mL) was added manganese dioxide (70.00 g), and the reaction mixture was stirred at 60°C for 19 hours. The reaction mixture was cooled to room temperature and filtered through Celite. The insoluble materials were separated by filtration and then washed with chloroform. To the filtrate were added anhydrous magnesium sulfate and activated carbon (3.00 g). The desiccant and activated carbon were removed by filtration, and the solvent was evaporated under reduced pressure to obtain 2,2',6'-trimethyl-4'-(tetrahydro-2H-pyran-2-yloxy)biphenyl-3-carbaldehyde (37.82 g) as a brown gummy syrup.

In the same manner as in the method of Preparation Example 14, the compounds of Preparation Examples 14-1 to 14-11 shown in Tables below were prepared.

### Preparation Example 15

To a solution of 2,2',6'-trimethyl-4'-(tetrahydro-2H-pyran-2-yloxy)biphenyl-3-carbaldehyde (35.13 g) in THF (350 mL) was added 1 M hydrochloric acid (350 mL), followed by stirring at room temperature for 3.5 hours. The solvent was evaporated under reduced pressure, and to the residue was added water, followed by extraction with ethyl acetate. The organic layer was washed with a saturated aqueous sodium chloride solution, and then anhydrous magnesium sulfate and activated carbon (3.00 g) were added thereto. The mixture was filtered through Celite to remove the desiccant and activated carbon, and the solvent was evaporated under reduced pressure. To the residue was added heptane (100 mL), followed by stirring for 0.5 hours under ice-cooling. The solid was collected by filtration, washed with heptane (20 mL), and then heated and dried under reduced pressure to obtain 4'-hydroxy-2,2',6'-trimethylbiphenyl-3-carbaldehyde (22.46 g) as a pale yellow solid.

### Preparation Example 16

To a solution of 4'-hydroxy-2,2',6'-trimethylbiphenyl-3-carbaldehyde (2.00 g) in DMF (20 mL) were added cesium carbonate (8.13 g) and 2-bromoethyl benzoate (1.70 mL). The reaction mixture was warmed to 70°C and stirred for 14 hours. The reaction mixture was cooled to room temperature, and water (100 mL) was added thereto, followed by extraction with ethyl acetate. The organic layer was washed with a saturated aqueous sodium chloride solution and dried over anhydrous magnesium sulfate. Then, the desiccant was removed by filtration and the solvent was evaporated under reduced pressure. The resulting residue was purified by silica gel column chromatography (hexane-ethyl acetate) to obtain 2-[(3'-formyl-2,2',6-trimethylbiphenyl-4-yl)oxy]ethyl benzoate (1.56 g) as a colorless oil.

In the same manner as in the method of Preparation Example 16, the compounds of Preparation Examples 16-1 to 16-13 shown in Tables below were prepared.

### Preparation Example 17

To a mixture of methyl 4-fluoro-2-hydroxybenzoate (29.55 g), potassium carbonate (31.00 g), and acetone (280 mL) was added 3-bromodihydrofuran-2(3H)-one (25 mL). The reaction mixture was warmed to 60°C and stirred for 12 hours. The reaction mixture was cooled to room temperature and filtered. The filtrate was concentrated under reduced pressure, and the resulting residue was purified by silica gel column chromatography (hexane-ethyl acetate) to obtain methyl 4-fluoro-2-[(2-oxotetrahydrofuran-3-yl)oxy]benzoate (31.90 g) as a white solid.

### Preparation Example 18

A mixture of 4'-hydroxy-2,2',6'-trimethylbiphenyl-3-carbaldehyde (500 mg), (3-bromopropoxy)(tert-butyl) dimethylsilane (0.53 mL), potassium phosphate (1.30 g), and DMF (8 mL) was stirred at 65°C for 15.5 hours. To the reaction mixture was added water, followed by extraction with a toluene-ethyl acetate solution. Further, the aqueous layer was extracted with a toluene-ethyl acetate solution. The organic layer was combined, washed with water and a saturated aqueous sodium chloride solution, and dried over anhydrous magnesium sulfate. The desiccant was removed by filtration and the solvent was evaporated under reduced pressure. The resulting residue was purified by silica gel column chromatography (hexane-ethyl acetate) to obtain 4'-(3-{[tert-butyl(dimethyl)silyl]oxy}propoxy)-2,2',6'-trimethylbiphenyl-3-carbaldehyde (775 mg) as a colorless gummy syrup.

In the same manner as in the method of Preparation Example 18, the compounds of Preparation Examples 18-1 to 18-6 shown in Tables below were prepared.

### Preparation Example 19

A mixture of 4'-hydroxy-2,2',6'-trimethylbiphenyl-3-carbaldehyde (800 mg), ethyl bromoacetate (0.45 mL), potassium carbonate (1.30 g), and acetone (15 mL) was stirred at room temperature for 5 hours. To the reaction mixture was added ethyl bromoacetate (0.30 mL), followed by stirring at room temperature for 13.5 hours. To the reaction mixture was added water, followed by extraction with ethyl acetate. The organic layer was washed with a saturated aqueous sodium chloride solution and dried over anhydrous magnesium sulfate. The desiccant was removed by filtration and the solvent was evaporated under reduced pressure. The resulting residue was purified by silica gel column chromatography (hexane-ethyl acetate) to obtain ethyl [(3'-formyl-2,2',6-trimethylbiphenyl-4-yl)oxy]acetate (962 mg) as a colorless gummy syrup.

### Preparation Example 20

To a mixture of 2-[(3'-formyl-2,2',6-trimethylbiphenyl-4-yl)oxy]ethyl benzoate (740 mg) and methanol (7.5 mL) was added sodium borohydride (80 mg) under ice-cooling, followed by stirring at room temperature for 0.5 hours. To the reaction mixture was added water, followed by extraction with ethyl acetate. The organic layer was washed with a saturated aqueous sodium chloride solution and then dried over anhydrous magnesium sulfate. The desiccant was removed by filtration, and the solvent was evaporated under reduced pressure to obtain 2-{[3'-(hydroxymethyl)-2,2',6-trimethylbiphenyl-4-yl]oxy}ethyl benzoate (670 mg) as a colorless gummy syrup.

In the same manner as in the method of Preparation Example 20, the compounds of Preparation Examples 20-1 to 20-3 shown in Tables below were prepared.

### Preparation Example 21

To a mixture of sodium hydride (about 40% mineral oil was added, 2.10 g) and bis(2-methoxyethyl)ether (50 mL) was added ethyl diethylphosphonoacetate (11.0 mL) under ice-cooling, followed by stirring at room temperature for 0.5 hours. To the reaction mixture was added a solution of 3-(benzyloxy)-9H-fluoren-9-one (5.03 g) in bis(2-methoxyethyl) ether (50 mL), and the reaction mixture was stirred at 150°C for 0.5 hours. The reaction mixture was cooled to room temperature, and then water (300 mL) was added thereto, followed by extraction with an ethyl acetate solution. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate), and the resulting yellow oil (12.3 g) was diluted with toluene (60 mL), and then washed with water and a saturated aqueous sodium chloride solution. The organic layer was dried over anhydrous magnesium sulfate, and then the solvent was evaporated under reduced pressure to obtain ethyl [3-(benzyloxy)-9H-fluoren-9-ylidene]acetate (6.13 g) as a yellow oil.

### Preparation Example 22

Under nitrogen air flow, to a mixture of sodium hydride (about 40% mineral oil was added, 1.30 g) and DMF (70 mL) was added diethyl (cyanomethyl)phosphonate (4.80 mL) portionwise under ice-cooling, followed by stirring at the same temperature for 0.5 hours. To the reaction mixture was added a solution of 5'-{[4'-(methoxymethoxy)-2,2',6'-trimethylbiphenyl-3-yl]methoxy}spiro[cyclopropane-1,2'-inden]-1'(3'H)-one (4.83 g) in DMF (30 mL), followed by stirring at 60°C for 17 hours. The reaction mixture was cooled to room temperature, and water was added thereto, followed by extraction with a toluene-ethyl acetate solution. The organic layer was washed with water and a saturated aqueous sodium chloride solution, and then dried over anhydrous magnesium sulfate. The desiccant was removed by filtration and the solvent was evaporated under reduced pressure. The resulting residue was purified by silica gel column chromatography (hexane-ethyl acetate) to obtain [5'-{[4'-(methoxymethoxy)-2,2',6'-trimethylbiphenyl-3-yl]methoxy}spiro[cyclopropan-1,2'-inden]-1'(3'H)-ylidene]acetonitrile (3.69 g) as a white amorphous solid.

In the same manner as in the method of Preparation Example 22, the compounds of Preparation Examples 22-1 to 22-3 shown in Tables below were prepared.

### Preparation Example 23

To a mixture of sodium hydride (about 40% of mineral oil added, 15.0 g) and DMF (230 mL) was added dropwise diethyl cyanomethylphosphonate (59.0 mL) under ice-cooling. The reaction mixture was stirred for 45 minutes under ice-cooling, and then a solution of methyl 1'-oxo-1',3'-dihydrospiro[cyclopropan-1,2'-indene]-5'-carboxylate (26.4 g) in DMF (230 mL) was added thereto, followed by stirring at room temperature for 2.5 hours. To the reaction mixture was added a 5 M aqueous sodium hydroxide solution (50 mL), followed by stirring at room temperature for 0.5 hours. Thereafter, water (500 mL) was added, and 1 M hydrochloric acid (300 mL) was further added thereto under ice-cooling. Moreover, water (500 mL) was added thereto, followed by stirring at room temperature for 0.5 hours. The resulting solid was collected by filtration, washed with water, and then heated and dried under reduced pressure to obtain 1'-(cyanomethylene)-1',3'-dihydrospiro[cyclopropan-1,2'-indene]-5'-carboxylic acid (30.9 g) as a green brown solid.

### Preparation Example 24

To a mixture of ethyl [3-(benzyloxy)-9H-fluoren-9-ylidene]acetate (6.13 g), ethanol (60 mL), and ethyl acetate (15 mL) was added 10% palladium on activated carbon (900 mg) under a nitrogen atmosphere. Next, the mixture was stirred at room temperature for 8 hours under 3.0 to 4.0 kg/cm² of hydrogen atmosphere. The catalyst was removed by filtration through Celite, and then the solvent was evaporated under reduced pressure. The resulting residue was purified by silica gel column chromatography (hexane-ethyl acetate) to obtain ethyl (3-hydroxy-9H-fluoren-9-yl)acetate (4.49 g) as a colorless gummy syrup.

### Preparation Example 25

Under nitrogen air flow, to NMP (250 mL) was added sodium hydride (about 40% of mineral oil added, 21.00 g) under ice-cooling, followed by stirring at the same temperature for 10 minutes. Thereafter, a solution of 5-fluoroindan-1-one (15.00 g) and 1,2-dibromoethane (30 mL) in NMP (50 mL) was added dropwise thereto. After completion of addition dropwise, the mixture was stirred at the same temperature for 10 minutes. Moreover, 1,2-dibromoethane (10 mL) was added dropwise thereto, and the reaction mixture was stirred at the same temperature for 0.5 hours. To the reaction mixture were added water and a saturated aqueous sodium chloride solution, followed by extraction with a toluene-ethyl acetate solution. The organic layer was washed with a saturated aqueous sodium chloride solution and dried over anhydrous magnesium sulfate. Then, the desiccant was removed by filtration and the solvent was evaporated under reduced pressure. The resulting residue was purified by silica gel column chromatography (hexane-ethyl acetate) to obtain 5'-fluorospiro[cyclopropane-1,2'-inden]-1'(3'H)-one (10.06 g) as a pale yellow solid.

In the same manner as in the method of Preparation Example 25, the compounds of Preparation Examples 25-1 to 25-2 shown in Tables below were prepared.

### Preparation Example 26

To a mixture of [5'-{[4'-(methoxymethoxy)-2,2',6'-trimethylbiphenyl-3-yl]methoxy}spiro[cyclopropan-1,2'-inden]-1'(3'H)-ylidene]acetonitrile (3.69 g) and methanol (65 mL) was added magnesium (turnings, 1.70 g). To the reaction mixture were added 3 droplets of a mixture of magnesium (cut flake-shaped, 0.10 g), iodine (1 piece), and methanol (5 mL), which had been stirred at room temperature for 0.5 hours, followed by stirring at room temperature for 1 hour. To the reaction mixture were added ethyl acetate and 1 M hydrochloric acid, followed by stirring at room temperature for 0.5 hours and then extracting with ethyl acetate. The organic layer was washed with a saturated aqueous sodium chloride solution and then dried over anhydrous magnesium sulfate. The desiccant was removed by filtration and the solvent was evaporated under reduced pressure. The resulting residue was purified by silica gel column chromatography (hexane-ethyl acetate) to obtain (5'-{[4'-(methoxymethoxy)-2,2',6'-trimethylbiphenyl-3-yl]methoxy}-1',3'-dihydrospiro[cyclopropan-1,2'-inden]-1'-yl)acetonitrile (2.59 g) as a yellow gummy syrup.

In the same manner as in the method of Preparation Example 26, the compounds of Preparation Examples 26-1 to 26-4 shown in Tables below were prepared.

### Preparation Example 27

To a solution of (5'-{[4'-(methoxymethoxy)-2,2',6'-trimethylbiphenyl-3-yl]methoxy}-1',3'-dihydrospiro[cyclopropan-1,2'-inden]-1'-yl)acetonitrile (2.59 g) in THF (30 ml) and methanol (13 mL) was added 1 M hydrochloric acid (24 mL), followed by stirring at 55°C for 15.5 hours. The reaction mixture was cooled to room temperature, and the solvent was evaporated under reduced pressure. To the resulting residue was added water, followed by extraction with ethyl acetate. The organic layer was washed with a saturated aqueous sodium chloride solution and then dried over anhydrous magnesium sulfate. The desiccant was removed by filtration and the solvent was evaporated under reduced pressure to obtain {5'-[(4'-hydroxy-2,2',6'-trimethylbiphenyl-3-yl)methoxy]-1',3'-dihydrospiro[cyclopropan-1,2'-inden]-1'-yl}acetonitrile (2.36 g) as a pale yellow amorphous solid.

In the same manner as in the method of Preparation Example 27, the compound of Preparation Example 27-2 shown in Tables below was prepared.

### Preparation Example 28

Under nitrogen air flow, a solution of {5'-[(4'-{[(4S)-2,2-dimethyl-1,3-dioxolan-4-yl]methoxy}-2,2',6'-trimethylbiphenyl-3-yl)methoxy]-1',3'-dihydrospiro[cyclopropan-1,2'-inden]-1'-yl}acetonitrile (400 mg) in toluene (10 mL) was cooled in a dry ice-acetone bath. To the reaction mixture was added dropwise a 0.99 M diisobutylaluminum hydride solution in toluene (1.00 mL), followed by stirring at the same temperature for 15 minutes. To the reaction mixture were added ethyl acetate and a saturated aqueous potassium sodium (+)-tartrate solution, followed by stirring for 1 hour while warming to room temperature. The reaction mixture was filtered through Celite and washed with ethyl acetate. The resulting filtrate was concentrated under reduced pressure to obtain {5'-[(4'-{[(4S)-2,2-dimethyl-1,3-dioxolan-4-yl]methoxy}-2,2',6'-trimethylbiphenyl-3-yl)methoxy]-1',3'-dihydrospiro[cyclopropan-1,2'-inden]-1'-yl}acetaldehyde (383 mg) as a colorless gummy syrup.

In the same manner as in the method of Preparation Example 28, the compounds of Preparation Examples 28-1 to 28-4 shown in Tables below were prepared.

### Preparation Example 29

To a solution of methyl 2,2',6'-trimethylbiphenyl-3-benzoate (2.21 g) in dichloromethane (20 mL) was added dropwise a 1.0 M diisobutylaluminum hydride solution in toluene (22 mL) at 0°C, followed by stirring at the same temperature for 0.5 hours. To the reaction mixture was added a saturated aqueous potassium sodium (+)-tartrate solution (25 mL), followed by filtration through Celite and then extraction with an ethyl acetate-diethyl ether solution. The organic layer was washed with a saturated aqueous sodium chloride solution and dried over anhydrous magnesium sulfate. Then, the desiccant was removed by filtration and the solvent was evaporated under reduced pressure to obtain (2,2',6'-trimethylbiphenyl-3-yl)methanol (1.90 g) as a colorless oil.

### Preparation Example 30

Under nitrogen air flow, a solution of 4'-{[(4S)-2,2-dimethyl-1,3-dioxolan-4-yl]methoxy}-N-methoxy-N, 2,2',6'-tetramethylbiphenyl-3-carboxamide (131.00 g) in toluene (1000 mL) was cooled in a dry ice-acetone bath. To the reaction mixture was added dropwise a 0.99 M diisobutylaluminum hydride solution in toluene (352 mL), followed by stirring at the same temperature for 1 hour. To the reaction mixture was added a saturated aqueous potassium sodium (+)-tartrate solution, followed by warming to room temperature and stirring for 1.5 hours. To the reaction mixture was added ethyl acetate, followed by filtrating through Celite and washing ethyl acetate. The resulting filtrate was subjected to liquid-separation, and the organic layer was washed with water and a saturated aqueous sodium chloride solution, and dried over anhydrous magnesium sulfate. The desiccant was removed by filtration and the filtrate was concentrated under reduced pressure. The resulting residue was purified by silica gel column chromatography (hexane-ethyl acetate) to obtain 4'-{[(4S)-2,2-dimethyl-1,3-dioxolan-4-yl]methoxy}-2,2',6'-trimethylbiphenyl-3-carbaldehyde (111.51 g) as a white solid.

### Preparation Example 31

To a mixture of methyl 4'-{[(4S)-2,2-dimethyl-1,3-dioxolan-4-yl]methoxy}-2,2',6'-trimethylbiphenyl-3-carboxylate (168.40 g), methanol (500 mL), and THF (500 mL) was added a 5 M aqueous sodium hydroxide solution (135 mL), followed by stirring at 65°C for 4 hours and then cooled to room temperature. The reaction mixture was concentrated under reduced pressure, and to the resulting residue was added water (500 mL). Further, 1 M hydrochloric acid (600 mL) was added dropwise under ice-cooling, and a 10% aqueous citric acid solution (350 mL) was further added thereto. The mixture was extracted with ethyl acetate, and the organic layer was washed with a saturated aqueous sodium chloride solution and then dried over anhydrous magnesium sulfate. The desiccant was removed by filtration and the solvent was evaporated under reduced pressure to obtain 4'-{[(4S)-2,2-dimethyl-1,3-dioxolan-4-yl]methoxy}-2,2',6'-trimethylbiphenyl-3-carboxylic acid (160.08 g) as a yellow solid.

### Preparation Example 32

To a solution of ethyl [(3'-formyl-2,2',6-trimethylbiphenyl-4-yl)oxy]acetate (1.31 g) in THF (13 mL) and methanol (13 mL) was added a 1 M aqueous sodium hydroxide solution (8 mL), followed by stirring at room temperature for 0.5 hours. The reaction mixture was acidified (pH 1) by the addition of 1 M hydrochloric acid, and extracted with chloroform. The organic layer was dried over anhydrous magnesium sulfate. The desiccant was removed by filtration and the solvent was evaporated under reduced pressure to obtain [(3'-formyl-2,2',6-trimethylbiphenyl-4-yl)oxy]acetic acid (1.19 g) as a white amorphous solid.

### Preparation Example 33

To a solution of 4'-(3-hydroxy-3-methylbutoxy)-2,2',6'-trimethylbiphenyl-3-carbaldehyde (763 mg) in dichloromethane (10 mL) were added triethylamine (0.80 mL), acetic anhydride (0.50 mL), and N,N-dimethylpyridin-4-amine (40 mg) under ice-cooling, and the ice bath was removed, followed by stirring for 8 hours while warming to room temperature. To the reaction mixture were added triethylamine (0.80 mL), acetic anhydride (0.50 mL), and N,N-dimethylpyridin-4-amine (40 mg), followed by stirring at 55°C for 17 hours. The reaction mixture was cooled to room temperature, and a saturated aqueous sodium hydrogen carbonate solution was added thereto, followed by extraction with ethyl acetate. The organic layer was washed with a 1 M hydrochloric acid and a saturated aqueous sodium chloride solution, and then dried over anhydrous magnesium sulfate. The desiccant was removed by filtration and the solvent was evaporated under reduced pressure. The resulting residue was purified by silica gel column chromatography (hexane-ethyl acetate) to obtain 3-[(3'-formyl-2,2',6-trimethylbiphenyl-4-yl)oxy]-1,1-dimethylpropyl acetate (676 mg) as a colorless gummy syrup.

In the same manner as in the method of Preparation Example 33, the compounds of Preparation Examples 33-1 to 33-2 shown in Tables below were prepared.

### Preparation Example 34

A mixture of 5'-bromospiro[cyclopropan-1,2'-indene]-1'(3'H)-one (41.00 g), palladium(II) acetate (3.88 g), 1,3-bis(diphenylphosphino)propane (7.13 g), triethylamine (48.2 mL), DMF (230 mL), and methanol (115 mL) was stirred at room temperature for 15 minutes under carbon monoxide air flow. The reaction mixture was stirred at 70°C for 13 hours under carbon monoxide atmosphere. The reaction mixture was cooled to room temperature, and then water, ethyl acetate, and toluene were added thereto. The insoluble materials were removed by filtration through Celite. The filtrate was subjected to liquid-separation and the aqueous layer was extracted with a toluene-ethyl acetate solution. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (chloroform-methanol) to obtain methyl 1'-oxo-1',3'-dihydrospiro[cyclopropan-1,2'-indene]-5'-carboxylate (35.85 g) as a pale yellow solid.

### Preparation Example 35

To a mixture of 1'-(cyanomethylene)-1',3'-dihydrospiro[cyclopropan-1,2'-indene]-5'-carboxylic acid (57.60 g), triethylamine (70.0 mL), tert-butanol (350 mL), and toluene (700 mL) was added diphenyl phosphoryl azide (75.0 mL), followed by stirring at room temperature for 0.5 hours. Thereafter, the reaction mixture was further stirred at 100°C for 24 hours. The reaction mixture was cooled to room temperature, and water was added thereto, followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain tert-butyl [1'-(cyanomethylene)-1',3'-dihydrospiro[cyclopropan-1,2'-inden]-5'-yl]carbamate (53.00 g) as a pale yellow solid.

### Preparation Example 36

To a mixture of tert-butyl [1'-(2-oxoethyl)-1',3'-dihydrospiro[cyclopropan-1,2'-inden]-5'-yl]carbamate (2.07 g), 2-methyl-2-butene (2.4 mL), and dioxane (40 mL) was added a mixture of sodium chlorite (1.20 g), sodium dihydrogen phosphate (3.30 g), and water (10 mL) under ice-cooling. The reaction mixture was ice-cooled and sitrred for 0.5 hours. To the reaction mixture was added water, followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain {5'-[(tert-butoxycarbonyl)amino]-1',3'-dihydrospiro[cyclopropan-1,2'-inden]-1'-yl}acetic acid (1.25 g) as a pale brown gummy syrup.

### Preparation Example 37

A mixture of methyl {5'-[(tert-butoxycarbonyl)amino]-1',3'-dihydrospiro[cyclopropan-1,2'-inden]-1'-yl}acetate (26.50 g) and a 4 M hydrogen chloride solution in dioxane (250 mL) was stirred at room temperature for 0.5 hours, and then the solvent was evaporated under reduced pressure. The resulting residue was diluted with THF (100 mL), and a saturated aqueous sodium hydrogen carbonate solution (150 mL) was added thereto, followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain methyl (5'-amino-1',3'-dihydrospiro[cyclopropan-1,2'-inden]-1'-yl)acetate (16.50 g) as an orange solid.

In the same manner as in the method of Preparation Example 37, the compounds of Preparation Examples 37-1 to 37-2 shown in Tables below were prepared.

### Preparation Example 38

To a mixture of 4'-{[(4S)-2,2-dimethyl-1,3-dioxolan-4-yl]methoxy}-2,2',6'-trimethylbiphenyl-3-carboxylic acid (160.08 g), 1H-benzotriazol-1-ol (64.30 g), and DMF (800 mL) were sequentially added triethylamine (70 mL), N-methoxymethane amine hydrochloride (46.40 g), and N-[3-(dimethylamino)propyl]-N'-ethylcarbodiimide hydrochloride (91.20 g), and the reaction mixture was stirred at room temperature for 3 hours. To the reaction mixture was added water (1000 mL), followed by extraction with toluene. The organic layer was sequentially washed with water, a 5% aqueous citric acid solution, a saturated aqueous sodium hydrogen carbonate solution, and a saturated aqueous sodium chloride solution, and then dried over anhydrous magnesium sulfate. The desiccant was removed by filtration and the solvent was evaporated under reduced pressure. The resulting residue was warmed to about 70°C, and heptane (1000 mL) was added dropwise thereto. After cooling to room temperature, the mixture was stirred for 0.5 hours under ice-cooling. The solid was collected by filtration, washed with heptane, and dried under reduced pressure to obtain 4'-{[(4S)-2,2-dimethyl-1,3-dioxolan-4-yl]methoxy}-N-methoxy-N,2,2',6'-tetramethylbiphenyl-3-carboxamide (154.05 g) as a white solid.

### Preparation Example 39

To a solution of methyl [4'-(3-aminopropoxy)-2,2',6'-trimethylbiphenyl-3-yl]acetate (1.40 g) in dichloromethane (20 mL) were added triethylamine (0.86 mL), propanoic anhydride (0.60 mL), and N,N-dimethylpyridin-4-amine (60 mg) under ice-cooling, and the ice-bath was removed, followed by stirring for 14.5 hours while warming to room temperature. To the reaction mixture was added water, followed by extraction with chloroform. The organic layer was dried over anhydrous magnesium sulfate. The desiccant was removed by filtration and the solvent was evaporated under reduced pressure. The resulting residue was purified by silica gel column chromatography (chloroform-methanol) to obtain methyl {2,2',6'-trimethyl-4'-[3-(propionylamino)propoxy]biphenyl-3-yl}acetate (1.57 g) as a colorless gummy syrup.

In the same manner as in the method of Preparation Example 39, the compound of Preparation Example 39-1 shown in Tables below was prepared.

### Preparation Example 40

A mixture of 5-bromo-4,6-dimethylpyrimidin-2-ol (1.00 g), (2-bromoethoxy)(tert-butyl) dimethylsilane (1.59 mL), potassium carbonate (1.36 g), and DMF (10 mL) was stirred at 100°C for 3 hours. Thereafter, the reaction mixture was returned to room temperature, (2-bromoethoxy)(tert-butyl)dimethylsilane (0.50 mL) was added thereto, and then the mixture was stirred at 100°C for 1 hour. The reaction mixture was cooled to room temperature, and then water was added thereto, followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain 5-bromo-2-(2-{[tert-butyl(dimethyl)silyl]oxy}ethoxy)-4,6-dimethylpyrimidine (1.01 g) as a colorless oil.

In the same manner as in the method of Preparation Example 40, the compounds of Preparation Examples 40-1 to 40-4 shown in Tables below were prepared.

### Preparation Example 41

To a solution of 5-bromo-2-[(2,2-dimethyl-1,3-dioxan-5-yl)methoxy]-4,6-dimethylpyrimidine (3.62 g) in THF (30 mL) was added 1 M hydrochloric acid (30 mL), followed by stirring at room temperature for 2.5 hours. To the reaction mixture was added a 1 M aqueous sodium hydroxide solution (30 mL), followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure to obtain 2-{[(5-bromo-4,6-dimethylpyrimidin-2-yl)oxy]methyl}propane-1,3-diol (3.08 g) as a white solid.

### Preparation Example 42

A mixture of 2-{[(5-bromo-4,6-dimethylpyrimidin-2-yl)oxy]methyl} profane-1,3-diol (3.08 g), tert-butyl(chloro) dimethylsilane (4.80 g), imidazole (2.90 g), and DMF (25 mL) was stirred at room temperature for 2 days. To the reaction mixture was added water (100 mL), followed by extraction with a toluene-ethyl acetate solution. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain 5-bromo-2-[3-{[tert-butyl(dimethyl)silyl]oxy}-2-({[tert-butyl(dimethyl)silyl]oxy}methyl)propoxy]-4,6-dimethylpyrimidine (5.40 g) as a colorless oil.

### Preparation Example 43

To a solution of methyl 4-fluoro-2-[(2-oxotetrahydrofuran-3-yl)oxy]benzoate (31.78 g) in methanol (500 mL) was added a 5 M aqueous sodium hydroxide solution (125 mL), followed by stirring at 60°C for 2 hours and then cooling to room temperature. To the reaction mixture was added 1 M hydrochloric acid (650 mL), followed by concentrating under reduced pressure. The residue was extracted with a 2-propanol-chloroform solution, and the organic layer was dried over anhydrous magnesium sulfate. The desiccant was removed by filtration and the solvent was evaporated under reduced pressure to obtain a pale yellow solid (36.15 g).
The resulting solid (36.15 g) was dissolved in dioxane (320 mL), and toluene (320 mL) and 4-methylbenzene sulfonic acid monohydrate (9.00 g) were added thereto, to which a Dean-Stark device was installed, followed by stirring for 5 hours under heating and refluxing. The reaction mixture was concentrated under reduced pressure, and to the resulting residue was added water, followed by extraction with a THF-chloroform solution, and further extraction with a 2-propanol-chloroform solution. The organic layer formed by combination thereof was dried over anhydrous magnesium sulfate. The desiccant was removed by filtration and the solvent was evaporated under reduced pressure to obtain 4-fluoro-2-[(2-oxotetrahydrofuran-3-yl)oxy]benzoic acid (27.90 g) as a pale brown solid.

### Preparation Example 44

To 4-fluoro-2-[(2-oxotetrahydrofuran-3-yl)oxy]benzoic acid (27.90 g) were added acetic anhydride (350 mL) and triethylamine (80 mL), followed by stirring for 4.5 hours under heating and refluxing. The reaction mixture was cooled to room temperature and concentrated under reduced pressure. To the resulting residue was added water, followed by extraction with ethyl acetate. The organic layer was washed with a saturated aqueous sodium chloride solution and dried over anhydrous magnesium sulfate. Then, the desiccant was removed by filtration and the solvent was evaporated under reduced pressure. The resulting residue was purified by silica gel column chromatography (hexane-ethyl acetate) to obtain 6-fluoro-4',5'-dihydro-3H-spiro[1-benzofuran-2,3'-furan]-2',3-dione (19.49 g) as a pale yellow solid.

### Preparation Example 45

A mixture of 6-fluoro-4',5'-dihydro-3H-spiro[1-benzofuran-2,3'-furan]-2',3-dione (19.48 g), sodium chloride (1.13 g), and DMSO (200 mL) was warmed to 150°C, followed by stirring at the same temperature for 1 hour. The reaction mixture was cooled to room temperature, and water was added thereto, followed by extraction with a toluene-ethyl acetate solution and ethyl acetate. The organic layer was washed with water and a saturated aqueous sodium chloride solution, and dried over anhydrous magnesium sulfate, and then the desiccant was removed. The solvent was evaporated under reduced pressure and the resulting residue was purified by silica gel column chromatography (hexane-ethyl acetate) to obtain 6-fluoro-3H-spiro[1-benzofuran-2,1'-cyclopropan]-3-one (13.18 g) as a pale yellow solid.

### Preparation Example 46

Under nitrogen air flow, to a solution of [5'-(methoxymethoxy)-1',3'-dihydrospiro[cyclopropan-1,2'-inden]-1'-yl]acetonitrile (12.23 g) in toluene (180 mL) was added dropwise a 1.01 M diisobutylaluminum hydride solution in toluene (150 mL) under cooling in a dry ice-acetone bath. The reaction mixture was slowly warmed to 0°C, and a saturated aqueous potassium sodium (+)-tartrate solution (400 mL) was added portionwise thereto under ice-cooling. To the reaction mixture was added ethyl acetate, followed by stirring at room temperature for a while, and then filtering through Celite. The filtrate was subjected to liquid-separation and the aqueous layer was further extracted with ethyl acetate. The organic layer was combined, washed with a saturated aqueous sodium chloride solution, and then dried over anhydrous magnesium sulfate. The desiccant was removed and the solvent was evaporated under reduced pressure to obtain a yellow oil (12.33 g).
To a solution of the resulting yellow oil (12.23 g) and 2-methyl-2-butene (17.6 mL) in dioxane (280 mL) were added dropwise a mixture of sodium chlorite (7.02 g), sodium dihydrogen phosphate (23.70 g), and water (70 ml) under ice-cooling. After completion of addition dropwise, the mixture was stirred at the same temperature for 0.5 hours. To the reaction mixture were added ethyl acetate and water, followed by performing liquid-separation. The aqueous layer was further extracted with ethyl acetate. The organic layer was combined, washed with a saturated aqueous sodium chloride solution, and then dried over anhydrous magnesium sulfate. The desiccant was removed by filtration and the solvent was evaporated under reduced pressure. The resulting residue was purified by silica gel column chromatography (hexane-ethyl acetate) to obtain a yellow syrup (8.46 g).
To a solution of the resulting yellow syrup (8.46 g) in DMF (130 mL) were addded potassium hydrogen carbonate (6.46 g) and methyl iodide (6.0 mL), followed by stirring at room temperature for 2 hours. To the reaction mixture was added water, followed by extraction with a toluene-ethyl acetate solution. The organic layer was wased with water and a saturated aqueous sodium chloride solution, and dried over anhydrous magnesium sulfate. The desiccant was removed by filtration and the solvent was evaporated under reduced pressure. The resulting residue was purified by silica gel column chromatography (hexane-ethyl acetate) to obtain methyl [5'-(methoxymethoxy)-1',3'-dihydrospiro[cyclopropan-1,2'-inden]-1'-yl]acetate (5.82 g) as a yellow oil.

### Preparation Example 47

To methyl [4'-(3-aminopropoxy)-2,2',6'-trirnethylbiphenyl-3-yl]acetate (600 mg) in dichloromethane (10 mL) was added triethylamine (0.30 mL) under ice-cooling, and methanesulfonyl chloride (0.15 mL) was added thereto. The ice-bath was removed, followed by stirring for 14.5 hours while warming to room temperature. To the reaction mixture was added water, followed by extraction with chloroform. The organic layer was dried over anhydrous magnesium sulfate. The desiccant was removed by filtration and the solvent was evaporated under reduced pressure. The resulting residue was purified by silica gel column chromatography (chloroform-methanol) to obtain methyl (2,2',6'-trimethyl-4'-{3-[(methylsulfonyl)amino]propoxy}biphenyl-3-yl)acetate (725 mg) as a colorless gummy syrup.

In the same manner as in the method of Preparation Example 47, the compound of Preparation Example 47-1 shown in Tables below was prepared.

### Preparation Example 48

To a solution of methyl (2,2',6'-trimethyl-4'-{3-[(methylsulfonyl)amino]propoxy}biphenyl-3-yl)acetate (725 mg) in THF (6 mL) and methanol (6 mL) was added a 1 M aqueous sodium hydroxide solution (3 mL), followed by stirring at room temperature for 1.5 hours. To the reaction mixture was added a 10% aqueous citric acid solution, followed by extraction with chloroform. The organic layer was dried over anhydrous magnesium sulfate. The desiccant was removed by filtration and the solvent was evaporated under reduced pressure to obtain N-(3-{[3'-(hydroxymethyl)-2,2',6-trimethylbiphenyl-4-yl]oxy}propyl)methanesulfonamide (668 mg) as a colorless gummy syrup.

In the same manner as in the method of Preparation Example 48, the compounds of Preparation Examples 48-1 to 48-3 shown in Tables below were prepared.

### Preparation Example 49

A mixture of N-(3-{[3'-(hydroxymethyl)-2,2',6-trimethylbiphenyl-4-yl]oxy}propyl)propane amide (801 mg), triethylamine (0.35 mL), and dichloromethane (12 mL) was ice-cooled, and chloro(trimethyl)silane (0.30 mL) was added thereto, followed by stirring for 10 minutes. The ice-bath was removed, followed by stirring for 1 hour while warming to room temperature. The solvent was evaporated under reduced pressure, and to the resulting residue was added THF. The insoluble materials were separated by filtration and washed with THF, and the filtrate was concentrated under reduced pressure. The resulting residue was dissolved in THF (10 mL), and sodium hydride (about 40% mineral oil was added, 100 mg) was added under ice-cooling, followed by stirring at the same temperature for 15 minutes. To the reaction mixture was added methyl iodide (0.15 mL) under ice-cooling, and the ice-bath was removed, followed by stirring for 40 minutes while warming to room temperature. Methyl iodide (0.20 mL) was further added thereto, followed by stirring at room temperature for 1 hour. To the reaction mixture was added a 1 M aqueous sodium hydroxide solution, followed by stirring at room temperature for 10 minutes, and 1 M hydrochloric acid was further added thereto, followed by stirring at room temperature for 0.5 hours. To the reaction mixture was added water, followed by extraction with ethyl acetate. The organic layer was dried over anhydrous magnesium sulfate. The desiccant was removed by filtration and the solvent was evaporated under reduced pressure. The resulting residue was purified by silica gel column chromatography (hexane-ethyl acetate) to obtain N-(3-{[3'-(hydroxymethyl)-2,2',6-trimethylbiphenyl-4-yl]oxy}propyl)-N-methylpropanamide (243 mg) as a colorless gummy syrup.

### Preparation Example 50

To a solution of tetrahydro-2H-pyran-4-ol (1.00 g) in pyridine (10 mL) was added 4-methylbenzenesulfonyl chloride under ice-cooling, and the reaction mixture was stirred at room temperature for 3 days. To the reaction mixture was added water, followed by extraction with ethyl acetate. The organic layer was washed with 1 M hydrochloric acid, a saturated aqueous sodium hydrogen carbonate solution, and a saturated aqueous sodium chloride solution, and dried over anhydrous magnesium sulfate. The desiccant was removed by filtration and the solvent was evaporated under reduced pressure to obtain tetrahydro-2H-pyran-4-yl 4-methylbenzenesulfonate (2.72 g) as a pale orange oil.

In the same manner as in the method of Preparation Example 50, the compound of Preparation Example 50-1 shown in Tables below was prepared.

### Preparation Example 51

In the same manner as in the method of Example 12 as described later, the compounds of Preparation Examples 51 and Preparation Examples 51-1 to 51-3 shown in Tables below were prepared.

### Preparation Example 52

In the same manner as in the method of Example 10 as described later, the compounds of Preparation Example 52 and Preparation Examples 52-1 to 52-3 shown in Tables below were prepared.

For the Preparation Example Compounds, the structures are shown in Tables 3 to 20 and Table 57 and the physicochemical data are shown in Tables 21 to 25.

**[Table 3]**

| **Pr** | **Structure** |
|---|---|
| **1** | 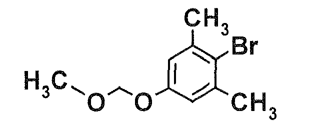 |
| **1-1** | 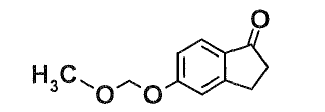 |
| **2** | 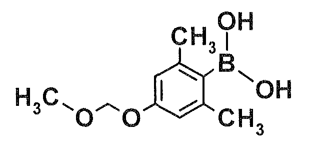 |
| **3** | 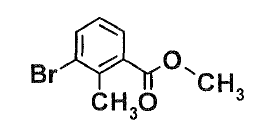 |
| **4** | 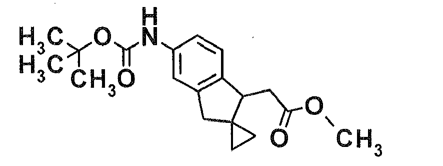 |
| **5** | 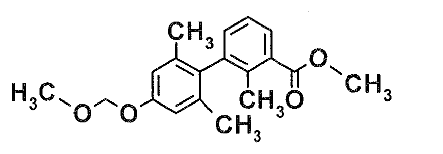 |
| **5-1** | 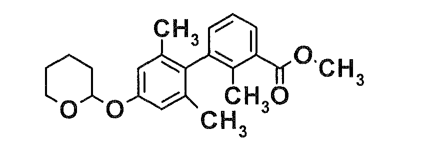 |
| **5-2** | 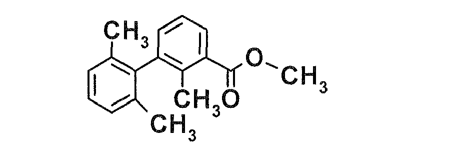 |
| **6** | 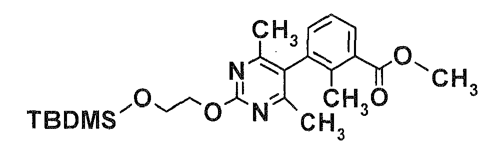 |

**[Table 4]**

| **Pr** | **Structure** |
|---|---|
| **6-1** | 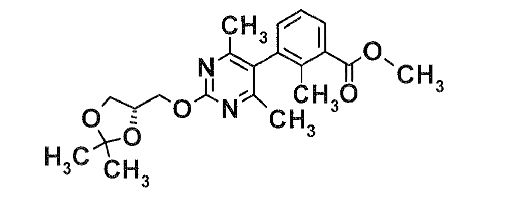 |
| **6-2** | 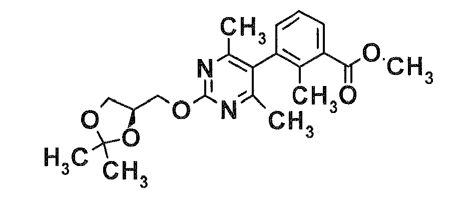 |
| **6-3** | 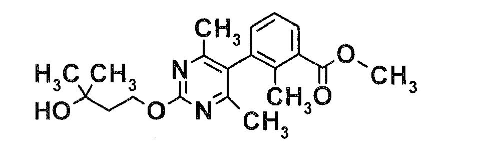 |
| **6-4** | 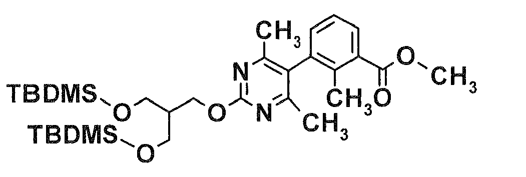 |
| **7** | 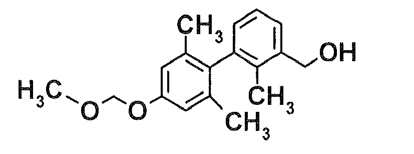 |
| **7-1** | 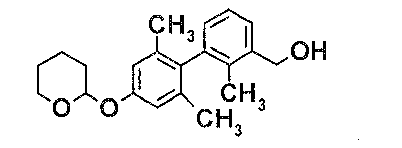 |
| **7-2** | 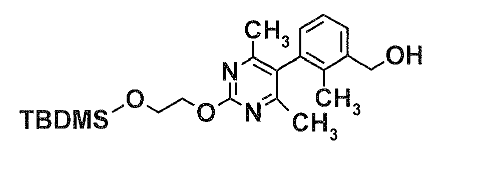 |

**[Table 5]**

| **Pr** | **Structure** |
|---|---|
| **7-3** | 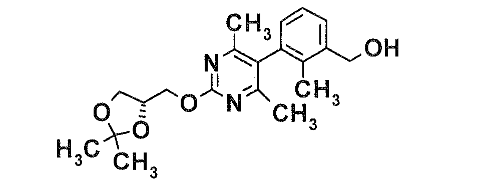 |
| **7-4** | 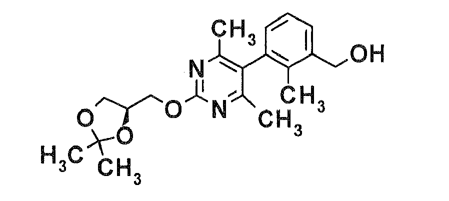 |
| **7-5** | 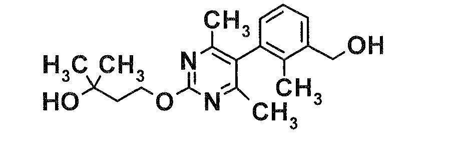 |
| **7-6** | 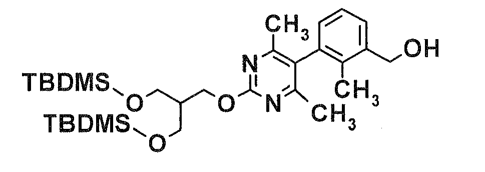 |
| **8** | 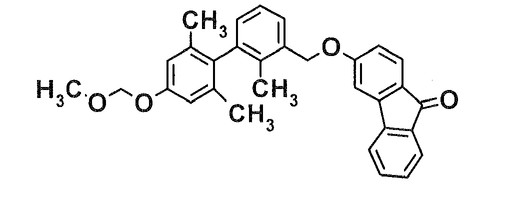 |
| **8-1** | 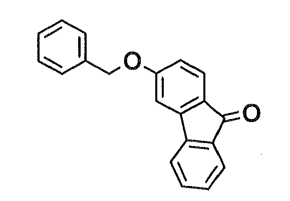 |
| **8-2** | 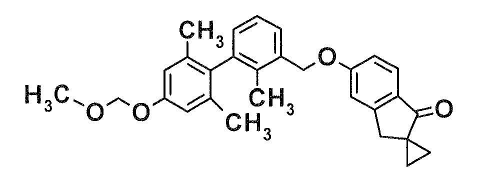 |

**[Table 6]**

| **Pr** | **Structure** |
|---|---|
| **8-3** | 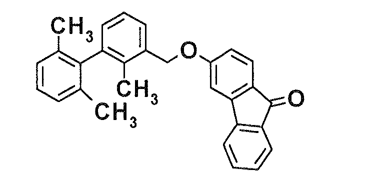 |
| **8-4** | 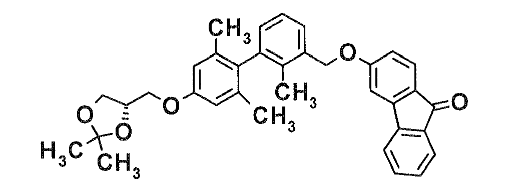 |
| **8-5** | 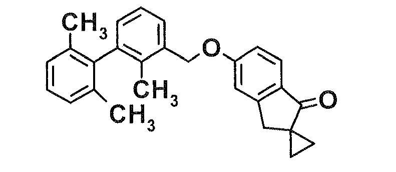 |
| **8-6** | 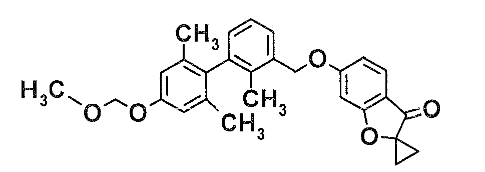 |
| **9** | 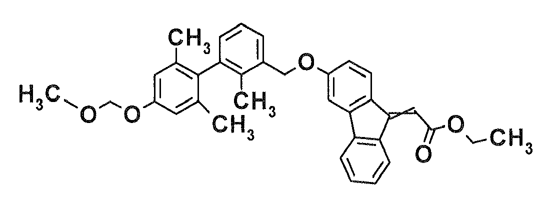 |
| **9-1** | 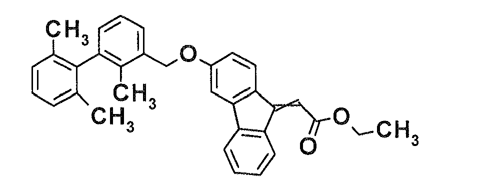 |
| **9-2** | 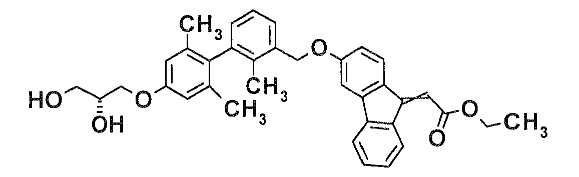 |

**[Table 7]**

| **Pr** | **Structure** |
|---|---|
| **10** | 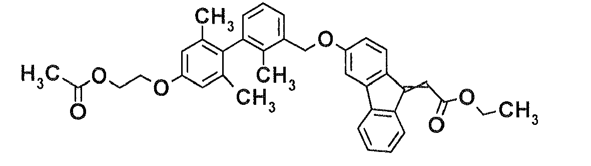 |
| **11** | 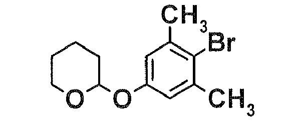 |
| **12** | 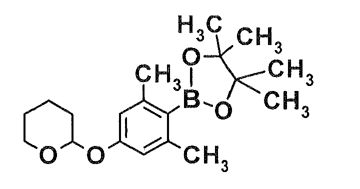 |
| **13** | 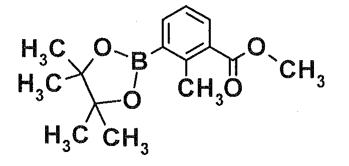 |
| **14** | 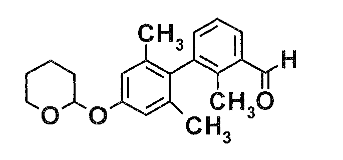 |
| **14-1** | 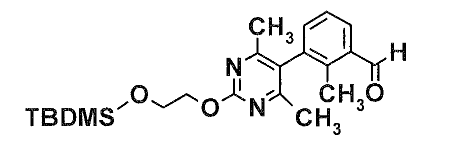 |
| **14-2** | 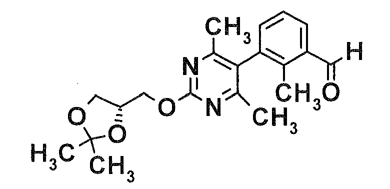 |

**[Table 8]**

| **Pr** | **Structure** |
|---|---|
| **14-3** | 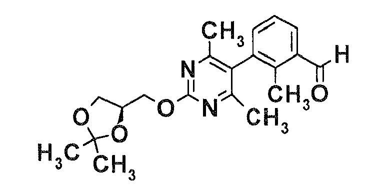 |
| **14-4** | 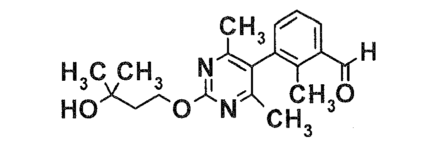 |
| **14-5** | 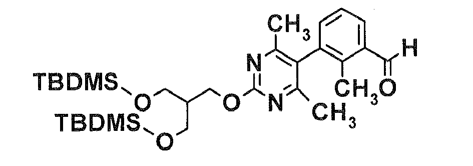 |
| **14-6** | 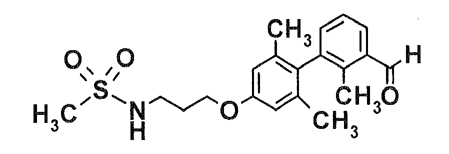 |
| **14-7** | 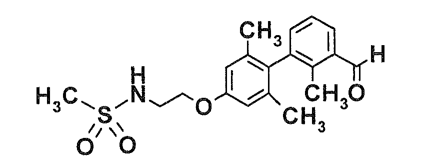 |
| **14-8** | 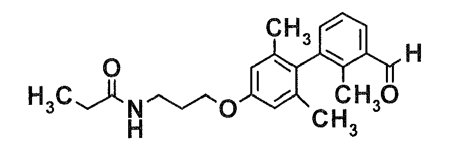 |
| **14-9** | 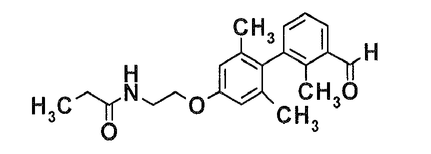 |
| **14-10** | 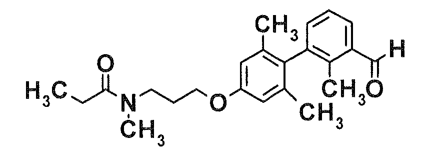 |

**[Table 9]**

| **Pr** | **Structure** |
|---|---|
| **14-11** | 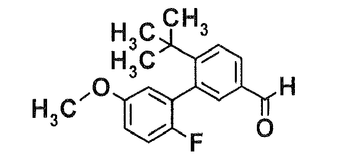 |
| **15** | 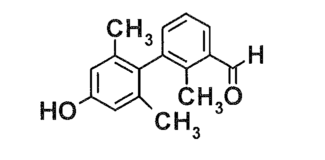 |
| **16** | 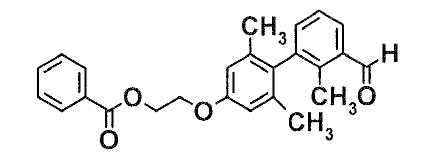 |
| **16-1** | 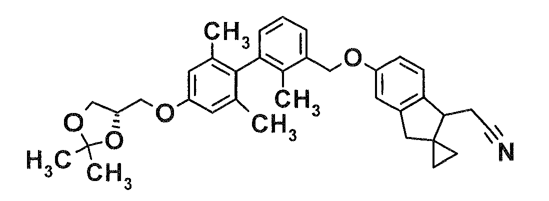 |
| **16-2** | 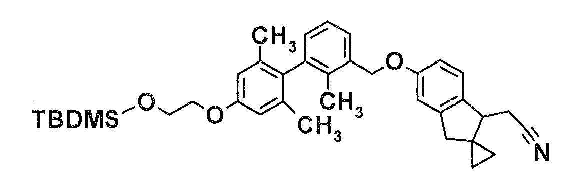 |
| **16-3** | 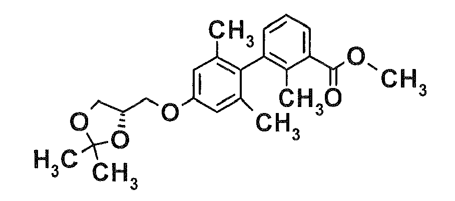 |
| **16-4** | 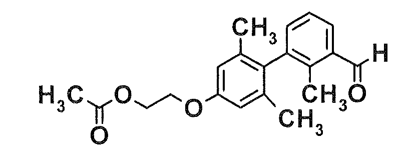 |

**[Table 10]**

| **Pr** | **Structure** |
|---|---|
| **16-5** | 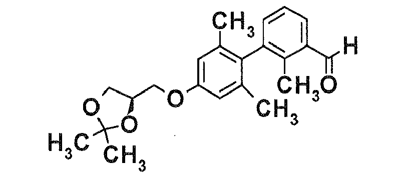 |
| **16-6** | 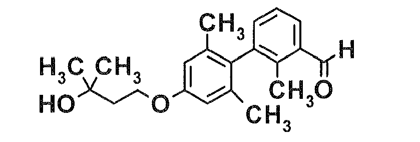 |
| **16-7** | 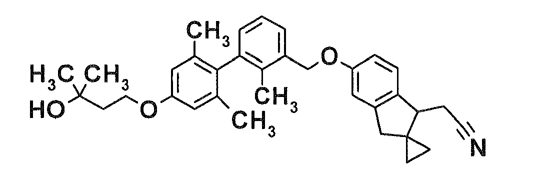 |
| **16-8** | 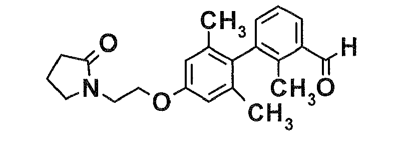 |
| **16-9** | 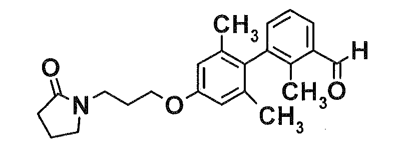 |
| **17** | 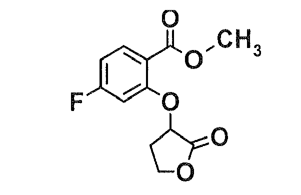 |
| **18** | 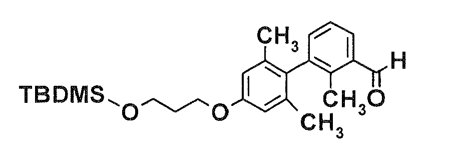 |
| **18-1** | 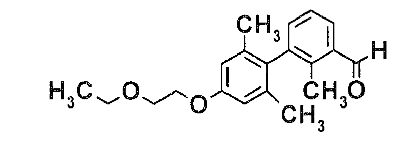 |

**[Table 11]**

| **Pr** | **Structure** |
|---|---|
| **18-2** | 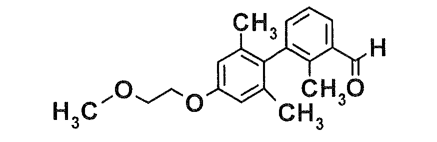 |
| **18-3** | 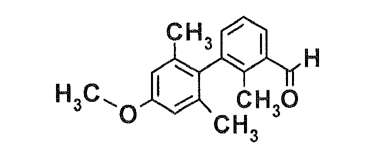 |
| **18-4** | 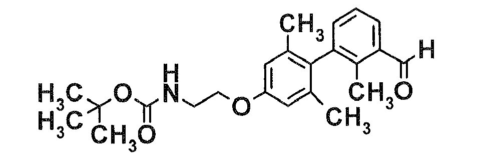 |
| **18-5** | 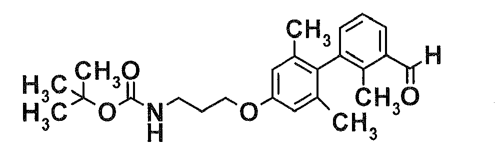 |
| **18-6** | 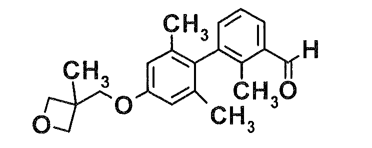 |
| **19** | 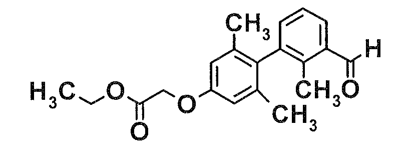 |
| **20** | 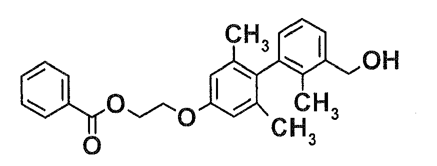 |
| **20-1** | 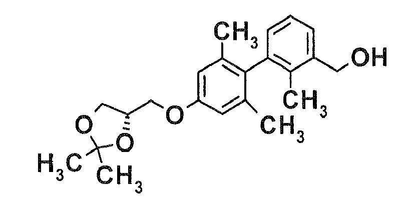 |

**[Table 12]**

| **Pr** | **Structure** |
|---|---|
| **20-2** | 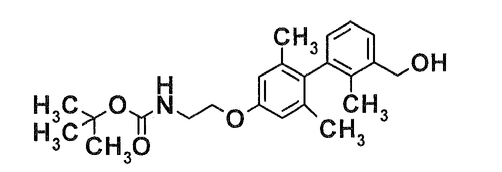 |
| **20-3** | 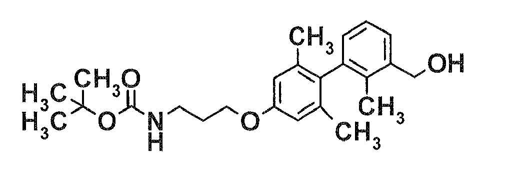 |
| **21** | 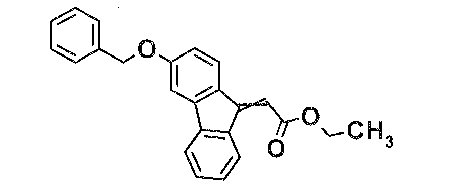 |
| **22** | 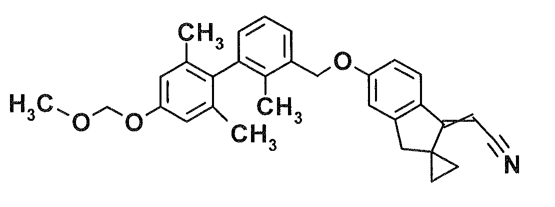 |
| **22-1** | 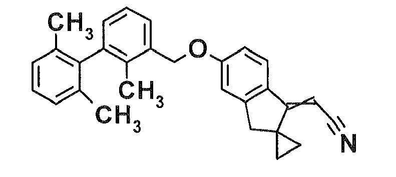 |
| **22-2** | 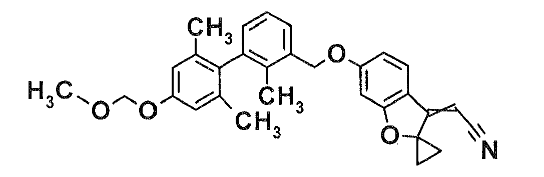 |
| **23** | 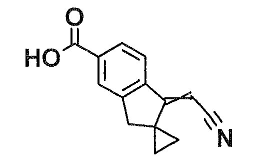 |

**[Table 13]**

| **Pr** | **Structure** |
|---|---|
| **24** | 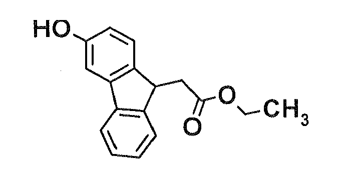 |
| **25** | 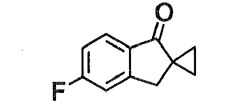 |
| **25-1** | 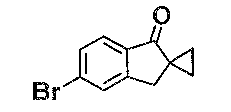 |
| **25-2** | 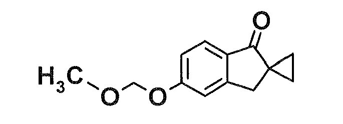 |
| **26** | 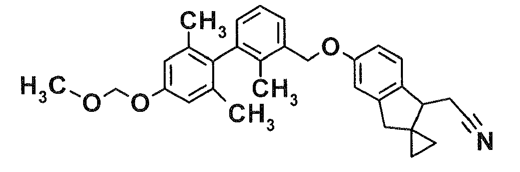 |
| **26-1** | 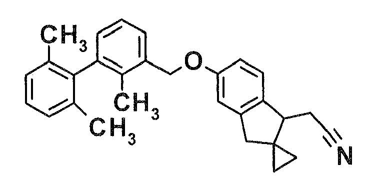 |
| **26-2** | 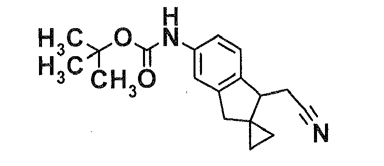 |
| **26-3** | 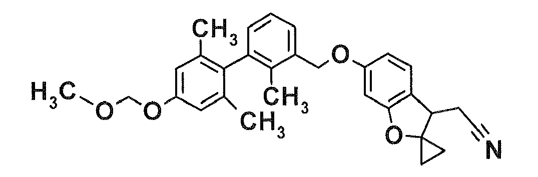 |

**[Table 14]**

| **Pr** | **Structure** |
|---|---|
| **26-4** | 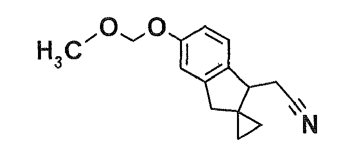 |
| **27-2** | 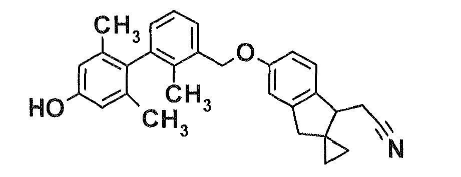 |
| **27-1** | 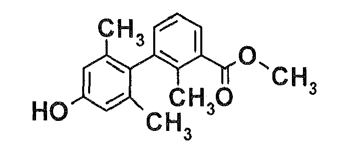 |
| **28** | 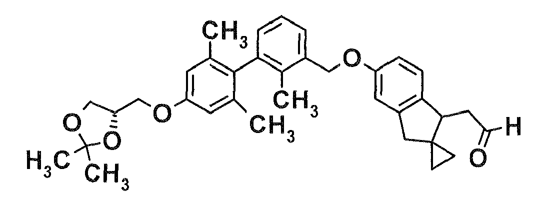 |
| **28-1** | 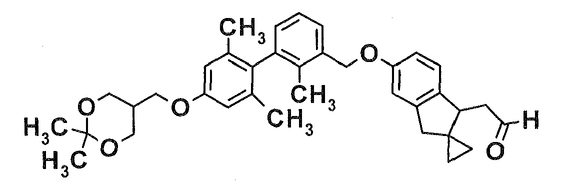 |
| **28-2** | 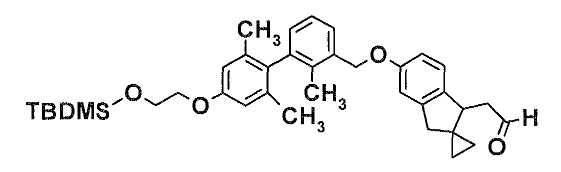 |
| **28-3** | 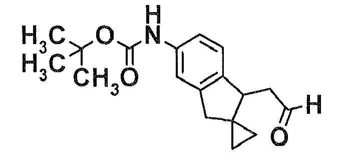 |

**[Table 15]**

| **Pr** | **Structure** |
|---|---|
| **28-4** | 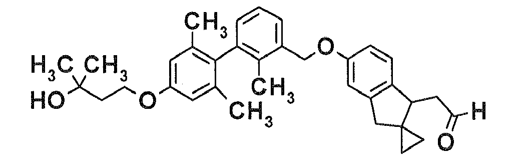 |
| **29** | 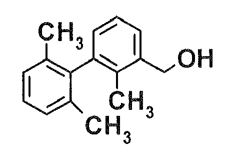 |
| **30** | 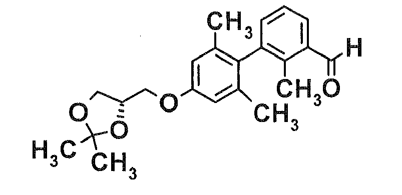 |
| **31** | 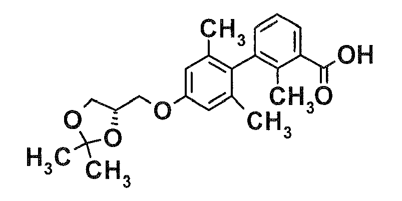 |
| **32** | 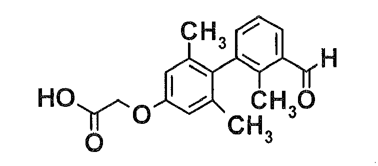 |
| **33** | 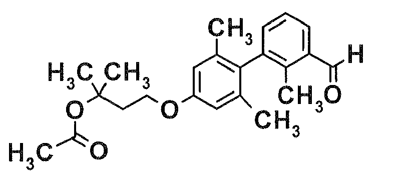 |
| **33-1** | 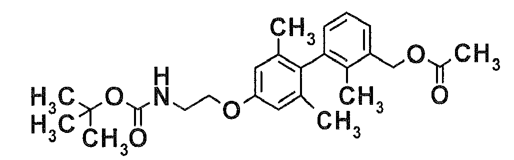 |

**[Table 16]**

| **Pr** | **Structure** |
|---|---|
| **33-2** | 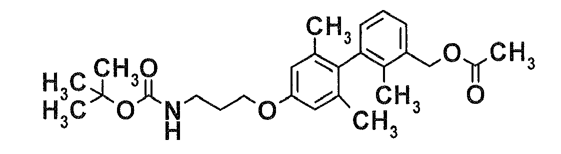 |
| **34** | 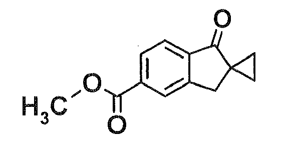 |
| **35** | 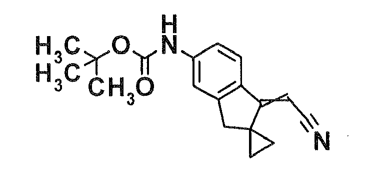 |
| **36** | 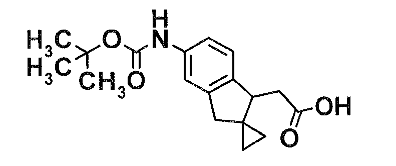 |
| **37** | 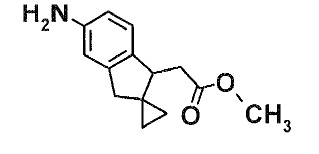 |
| **37-1** | 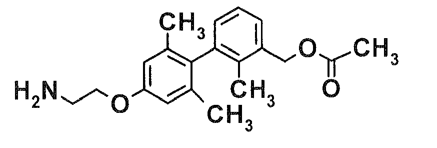 |
| **37-2** | 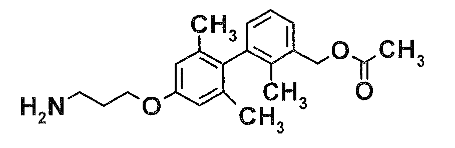 |
| **38** | 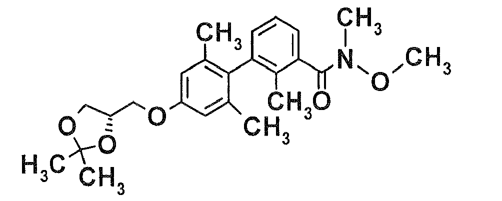 |

**[Table 17]**

| **Pr** | **Structure** |
|---|---|
| **39** | 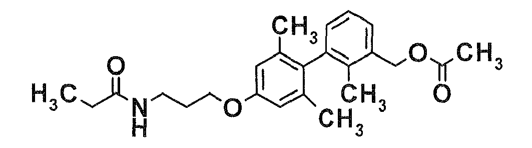 |
| **39-1** | 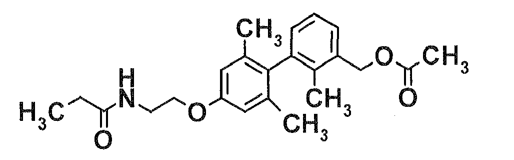 |
| **40** | 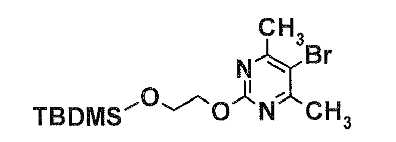 |
| **40-1** | 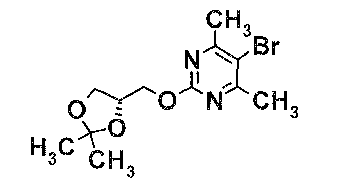 |
| **40-2** | 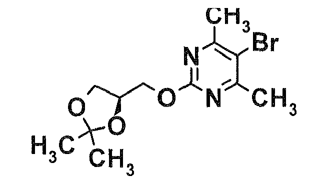 |
| **40-3** | 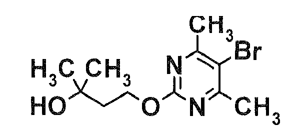 |
| **40-4** | 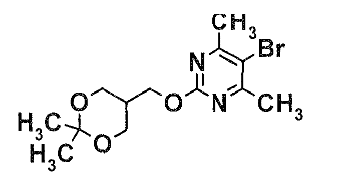 |
| **41** | 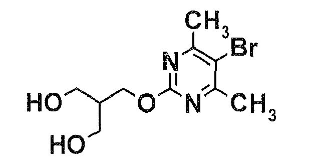 |

**[Table 18]**

| **Pr** | **Structure** |
|---|---|
| **42** | 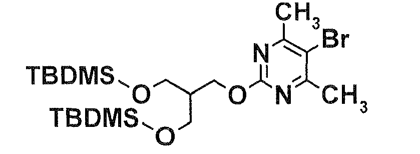 |
| **43** | 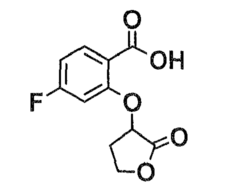 |
| **44** | 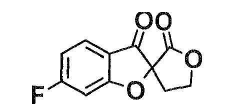 |
| **45** | 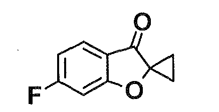 |
| **46** | 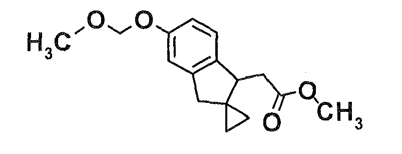 |
| **47** | 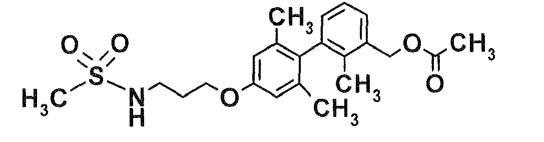 |
| **47-1** | 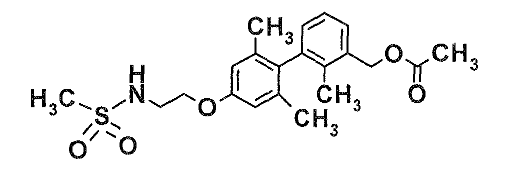 |
| **48** | 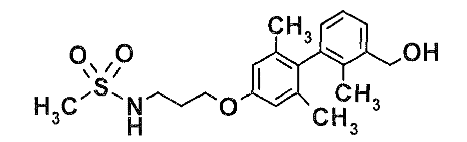 |

**[Table 19]**

| **Pr** | **Structure** |
|---|---|
| **48-1** | 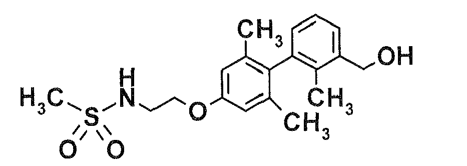 |
| **48-2** | 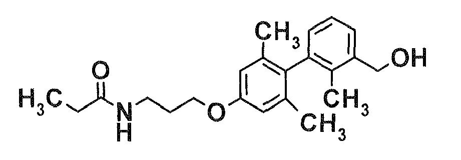 |
| **48-3** | 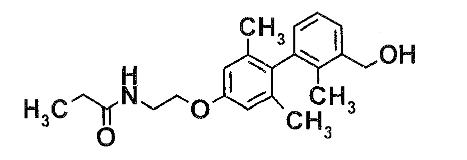 |
| **49** | 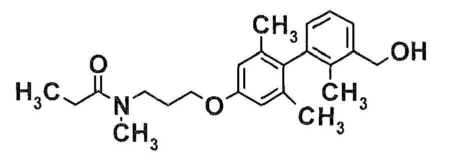 |
| **50** | 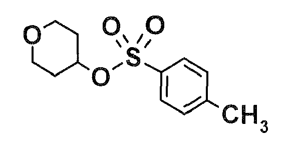 |
| **50-1** | 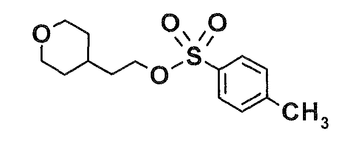 |
| **51** | 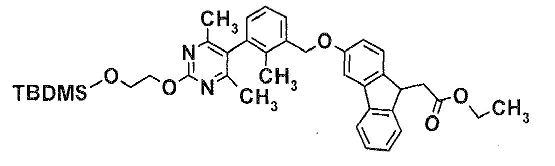 |
| **51-1** | 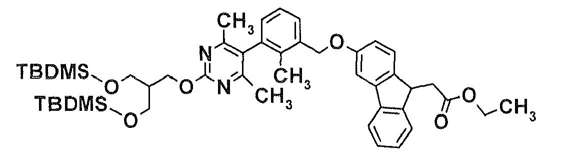 |

**[Table 20]**

| **Pr** | **Structure** |
|---|---|
| **51-2** | 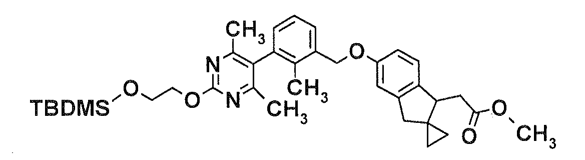 |
| **51-3** | 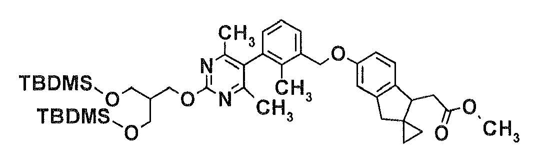 |
| **52** | 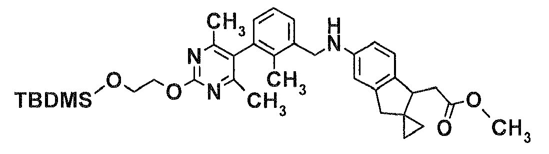 |
| **52-1** | 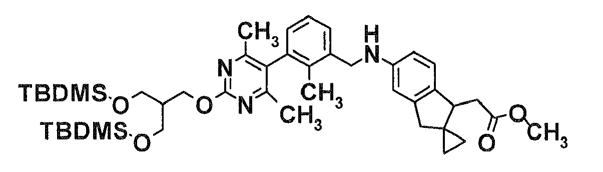 |
| **52-2** | 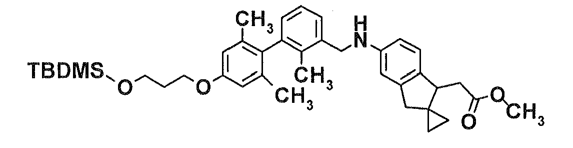 |
| **22-3** | 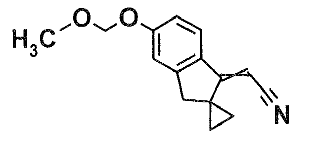 |

**[Table 21]**

| Pr | Data |
|---|---|
| 1 | EI : 244, 246 |
| 1-1 | ESI- : 191 |
| 2 | ESI- : 208 |
| 3 | EI : 228, 230 |
| 4 | ESI+ : 332 |
| 5 | EI : 314 |
| 5-1 | ESI+ : 355 |
| 5-2 | NMR1 : 1.86 (6H, s), 2.07 (3H, s), 3.85 (3H, s), 7.12-7.28 (4H, m), 7.40 (1H, t, J = 7.6 Hz), 7.70-7.84 (1H, m) |
| 6 | ESI+ : 431 |
| 6-1 | ESI+ : 387 |
| 6-2 | ESI+ : 387 |
| 6-3 | ESI+ : 359 |
| 6-4 | ESI+ : 589 |
| 7 | NMR2 : 1.61 (1H, t, J = 5.8 Hz), 1.90 (6H, s), 1.97 (3H, s), 3.52 (3H, s), 4.76 (2H, d, J = 5.7 Hz), 5.20 (2H, s), 6.80 (2H, s), 6.93-7.02 (1H, m), 7.17-7.29 (1H, m), 7.31-7.42 (1H, m) |
| 7-1 | ESI+ : 327 |
| 7-2 | ESI+ : 403 |
| 7-3 | ESI+ : 359 |
| 7-4 | ESI+ : 359 |
| 7-5 | ESI+ : 331 |
| 7-6 | ESI+ : 561 |
| 8 | ESI- : 463 |
| 8-1 | ESI+ : 287 |
| 8-2 | ESI+ : 443 |
| 8-3 | ESI- : 403 |
| 8-4 | NMR1 : 1.32 (3H, s), 1.38 (3H, s), 1.86 (6H, s), 1.96 (3H, s), 3.74-3.80 (1H, m), 3.97-4.05 (2H, m), 4.08-4.14 (1H, m), 4.38-4.45 (1H, m), 5.31 (2H, s), 6.76 (2H, s), 6.97-7.04 (2H, m), 7.30 (1H, t, J = 7.6 Hz), 7.38 (1H, t, J = 7.4 Hz), 7.49 (1H, d, J = 7.0 Hz), 7.55-7.63 (4H, m), 7.81 (1H, d, J = 7.4 Hz) |
| 8-5 | ESI+ : 383 |
| 8-6 | ESI+ : 445 |
| 9 | ESI+ : 535 |
| 9-1 | ESI+ : 475 |
| 9-2 | ESI+ : 565 |
| 10 | ESI+ : 577 |
| 11 | EI : 284, 286 |

**[Table 221**

| Pr | Data |
|---|---|
| 12 | FAB+: 333 |
| 13 | ESI+ : 277 |
| 14 | FAB+ : 325 |
| 14-1 | ESI+ : 401 |
| 14-2 | ESI+ : 357 |
| 14-3 | ESI+ : 357 |
| 14-4 | ESI+ : 311 [(M-OH)+] |
| 14-5 | ESI+ : 559 |
| 14-6 | ESI- : 374 |
| 14-7 | ESI- : 360 |
| 14-8 | ESI+ : 354 |
| 14-9 | ESI+ : 340 |
| 14-10 | ESI+ : 368 |
| 14-11 | NMR2 : 1.26 (9H, s), 3.80 (3H, s), 6.76 (1H, dd, J = 3.0, 6.0 Hz), 6.88 (1H, m), 7.03 (1H, dd, J = 8.0, 9.0 Hz), 7.53 (1H, d, J = 1.9 Hz), 7.73 (1H, d, J = 8.0 Hz), 7.84 (1H, dd, J 1.9, 8.0 Hz), 9.97 (1H, s) |
| 15 | ESI+ : 241 |
| 16 | ESI+ : 389 |
| 16-1 | FAB+ : 537 |
| 16-2 | FAB+ : 582 |
| 16-3 | EI : 385 |
| 16-4 | ESI+ : 327 |
| 16-5 | ESI+ : 355 |
| 16-6 | ESI+ : 327 |
| 16-7 | ESI- : 508 |
| 16-8 | ESI+ : 352 |
| 16-9 | ESI+ : 366 |
| 17 | ESI- : 253 |
| 18 | ESI+ : 413 |
| 18-1 | ESI+ : 313 |
| 18-2 | EI:298 |
| 18-3 | EI : 254 |
| 18-4 | ESI+ : 384 |
| 18-5 | ESI+ : 398 |
| 18-6 | ESI+ : 325 |
| 19 | ESI+ : 327 |

**[Table 23]**

| Pr | Data |
|---|---|
| 20 | NMR2 : 1.91 (6H, s), 1.97 (3H, s), 4.30-4.38 (2H, m), 4.65-4.82 (4H, m), 6.72 (2H, s), 6.95-7.00 (1H, m), 7.22-7.28 (4H, m), 7.34-7.39 (1H, m), 7.41-7.48 (2H, m), 7.54-7.61 (1H, m), 8.05-8.11 (2H, m) |
| 20-1 | NMR1 : 1.32 (3H, s), 1.37 (3H, s), 1.83 (9H, s), 3.72-4.45 (5H, m), 4.54 (2H, d, J = 5.2 Hz), 5.11 (1H, t, J = 5.3 Hz), 6.73 (2H, s), 6.84 (1H, d, J = 6.6 Hz), 7.22 (1H, t, J = 7.5 Hz), 7.37 (1H, d, J = 7.4 Hz) |
| 20-2 | ESI+ : 386 |
| 20-3 | ESI+ : 400 |
| 21 | ESI- : 355 |
| 22 | ESI- : 464 |
| 22-1 | ESI+ : 406 |
| 22-2 | ESI- : 466 |
| 23 | ESI- : 224 |
| 24 | ESI- : 267 |
| 25 | ESI+ : 177 |
| 25-1 | ESI+ : 237, 239 |
| 25-2 | ESI+ : 219 |
| 26 | ESI- : 466 |
| 26-1 | ESI- : 406 |
| 26-2 | ESI+ : 299 |
| 26-3 | ESI- : 468 |
| 26-4 | NMR2 : 0.58-0.78 (3H, m), 0.78-0.94 (1H, m), 2.34-2.64 (3H, m), 2.93 (1H, dd, J = 5.6, 7.6 Hz), 3.26 (1H, d, J = 16.3 Hz), 3.49 (3H, s), 5.17 (2H, s), 6.88-6.96 (2H, m), 7.24-7.32 (1H, m) |
| 27-2 | ESI- : 422 |
| 27-1 | ESI- : 269 |
| 28 | ESI+ : 541 |
| 28-1 | FAB+ : 554 |
| 28-2 | FAB- : 583 |
| 28-3 | ESI+ : 302 |
| 28-4 | ESI+ : 513 |
| 29 | NMR1 : 1.83 (3H, s), 1.86 (6H, s), 4.55 (2H, d, J = 5.3 Hz), 5.13 (1H, t, J = 5.4 Hz), 6.84-6.88 (1H, m), 7.08-7.20 (3H, m), 7.25 (1H, t, J = 7.5 Hz), 7.39 (1H, d, J = 6.9 Hz) |
| 30 | ESI+ : 355 |
| 31 | ESI- : 369 |
| 32 | ESI- : 297 |
| 33 | FAB+ : 368 |
| 33-1 | ESI+ : 428 |

**[Table 24]**

| Pr | Data |
|---|---|
| 33-2 | FAB- : 440 |
| 34 | ESI+ : 217 |
| 35 | ESI- : 295 |
| 36 | ESI- : 316 |
| 37 | ESI- : 232 |
| 37-1 | ESI+ : 328 |
| 37-2 | ESI+ : 342 |
| 38 | ESI+ : 414 |
| 39 | ESI+ : 398 |
| 39-1 | ESI+ : 384 |
| 40 | ESI+ : 361, 363 |
| 40-1 | ESI+ : 317, 319 |
| 40-2 | NMR2 : 1.39 (3H, s), 1.47 (3H, s), 2.57 (6H, s), 3.94 (1H, dd, J = 5.4, 8.5 Hz), 4.16 (1H, dd, J = 6.3, 8.6 Hz), 4.27 (1H, dd, J = 6.6, 10.3 Hz), 4.42-4.53 (2H, m) |
| 40-3 | NMR2 : 1.31 (6H, s), 1.62 (1H, s), 2.01 (2H, t, J = 6.6 Hz), 2.56 (6H, s), 4.51 (2H, t, J = 6.7 Hz) |
| 40-4 | NMR2 : 1.43 (3H, s), 1.46 (3H, s), 2.13-2.23 (1H, m), 2.56 (6H, s), 3.88 (1H, dd, J = 5.7, 12.1 Hz), 4.09 (1H, dd, J = 4.1, 12.1 Hz), 4.41 (2H, d, J = 6.9 Hz) |
| 41 | ESI+ : 291, 293 |
| 42 | ESI+ : 519, 521 |
| 43 | ESI- : 239 |
| 44 | ESI+: 223 |
| 45 | ESI+ : 179 |
| 46 | NMR1 : 0.44-0.61 (3H, m), 0.61-0.74 (1H, m), 2.36 (1H, dd, J = 8.6, 15.4 Hz), 2.46-2.58 (2H, m), 3.02 (1H, dd, J = 6.2, 8.5 Hz), 3.09 (1H, d, J = 16.3 Hz), 3.36 (3H, s), 3.60 (3H, s), 5.14 (2H, s), 6.79 (1H, dd, J = 2.4, 8.2 Hz), 6.85-6.90 (1H, m), 7.04 (1H, d, J = 8.3 Hz) |
| 47 | ESI- : 419 |
| 47-1 | ESI- : 404 |
| 48 | ESI- : 376 |
| 48-1 | ESI- : 362 |
| 48-2 | ESI+ : 356 |
| 48-3 | ESI+ : 342 |
| 49 | ESI+ : 370 |
| 50 | NMR2 : 1.60-1.92 (4H, m), 2.45 (3H, s), 3.41-3.53 (2H, m), 3.82-3.92 (2H, m), 4.63-4.78 (1H, m), 7.35 (2H, d, J = 8.0 Hz), 7.81 (2H, d, J = 8.0 Hz) NMR2 : 1.13-1.30 (2H, m), 1.42-1.70 (5H, m), 2.46 (3H, s), 3.31 (2H, dt, J = 2.0, 12.0 Hz), 3.85-3.94 (2H, m), 4.08 (2H, d, J = 6.0 Hz), 7.36 (2H, d, J = 8.5 Hz), 7.79 (2H, d, J = 8.5 Hz) |

**[Table 25]**

| Pr | Data |
|---|---|
| 50-1 | NMR2 : 1.13-1.30 (2H, m), 1.42-1.70 (5H, m), 2.46 (3H, s), 3.31 (2H, dt, J = 12.0, 2.0 Hz), 3.85-3.94 (2H, m), 4.08 (2H, d, J = 6.0 Hz), 7.36 (2H, d, J = 8.5 Hz), 7.79 (2H, d, J = 8.5 Hz) |
| 51 | ESI+ : 653 |
| 51-1 | ESI+ : 811 |
| 51-2 | ESI+ : 617 |
| 51-3 | ESI+ : 775 |
| 52 | ESI+ : 616 |
| 52-1 | ESI+ : 774 |
| 52-2 | ESI+ : 628 |
| 22-3 | ESI+ : 242 |
| 16-10 | FAB+ : 551 |
| 16-11 | ESI+ : 325 |
| 16-12 | EI : 338 |
| 16-13 | FAB+ : 353 |
| 27 | NMR1 :0.40-0.59 (3H, m), 0.59-0.70 (1H, m), 2.32 (1H, dd, J = 8.6, 15.3 Hz), 2.40-2.55 (2H, m), 2.97 (1H, dd, J = 6.2,8. 6 Hz), 3.02 (1H, d, J = 16.1 Hz), 3.59 (3H, s), 6.52 (1H, dd, J = 2.3, 8.1 Hz), 6.55-6.62 (1H, m), 6.90 (1H, d, J=8.2Hz), 9.15 (1H, s) |

### Example 1

A mixture of ethyl (3-{[4'-(2-acetoxyethoxy)-2,2',6'-trimethylbiphenyl-3-yl]methoxy}-9H-fluoren-9-ylidene)acetate (470 mg), 10% palladium on activated carbon (wetted with 50% H₂O, 100 mg), and ethanol (5 mL) was stirred at room temperature for 4 hours under a hydrogen (1.94 atm) atmosphere. The reaction mixture was filtered through Celite, and then the solvent was evaporated under reduced pressure. The resulting residue was purified by silica gel column chromatography (hexane-ethyl acetate) to obtain ethyl (3-{[4'-(2-acetoxyethoxy)-2,2',6'-trimethylbiphenyl-3-yl]methoxy}-9H-fluoren-9-yl)acetate (368 mg) as a colorless oil.

In the same manner as in the method of Example 1, the compound of Example 1-1 shown in Tables below was prepared.

### Example 2

A mixture of ethyl (3-{[4'-(2-acetoxyethoxy)-2,2',6'-trimethylbiphenyl-3-yl]methoxy}-9H-fluoren-9-yl)acetate (358 mg), a 1 M aqueous sodium hydroxide solution (1.8 mL), ethanol (5 mL) and THF (5 mL) was stirred at 50°C for 18 hours. The reaction mixture was cooled to room temperature, and 1 M hydrochloric acid (2 mL) was added thereto, followed by extraction with ethyl acetate. The organic layer was washed with a saturated aqueous sodium chloride solution and dried over anhydrous magnesium sulfate. The desiccant was removed by filtration and the solvent was evaporated under reduced pressure. The residue was purified by silica gel column chromatography (hexane-ethyl acetate). To the resulting yellow oil (113 mg) were added THF and a 1 M aqueous sodium hydroxide solution (0.22 mL), and the solvent was evaporated under reduced pressure. The residue was purified by ODS column chromatography (acetonitrile-water). To the resulting residue (95 mg) was added diethyl ether, and the solid was collected by filtration, washed with diethyl ether, and then heated and dried under reduced pressure to obtain sodium (3-{[4'-(2-hydroxyethoxy)-2,2',6'-trimethylbiphenyl-3-yl]methoxy}-9H-fluoren-9-yl)acetate (71 mg) as a white solid.

In the same manner as in the method of Example 2, the compounds of Examples 2-1 to 2-2 shown in Tables below were prepared.

### Example 3

A mixture of 2-{[3'-({[9-(2-ethoxy-2-oxoethyl)-9H-fluoren-3-yl]oxy}methyl)-2,2',6-trimethylbiphenyl-4-yl]oxy} ethyl benzoate (835 mg), a 1 M aqueous sodium hydroxide solution (7.0 mL), THF (4.5 mL), and ethanol (4.5 mL) was stirred at room temperature for 5 hours. To the reaction mixture was added 1 M hydrochloric acid (8 mL), followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain (3-{[4'-(2-hydroxyethoxy)-2,2',6'-trimethylbiphenyl-3-yl]methoxy}-9H-fluoren-9-yl)acetic acid (501 mg) as a pale yellow amorphous solid.

In the same manner as in the method of Example 3, the compound of Example 3-1 shown in Tables below was prepared.

### Example 4

To a mixture of {5'-[(4'-{[(4S)-2,2-dimethyl-1,3-dioxolan-4-yl]methoxy}-2,2',6'-trimethylbiphenyl-3-yl)methoxy]-1',3'-dihydrospiro[cyclopropan-1,2'-inden]-1'-yl}acetaldehyde (383 mg), 2-methyl-2-butene (0.23 mL), and dioxane (8 mL) was added a mixture of sodium chlorite (120 mg), sodium dihydrogen phosphate (340 mg), and water (2 mL) under ice-cooling, followed by stirring at room temperature for 0.5 hours. To the reaction mixture was added water, followed by extraction with chloroform. The organic layer was dried over anhydrous magnesium sulfate. The desiccant was removed by filtration and the solvent was evaporated under reduced pressure. The resulting residue was dissolved in THF (5 mL), and 1 M hydrochloric acid (4 mL) was added thereto, followed by stirring at room temperature for 3.5 hours. To the reaction mixture was added water, followed by extraction with chloroform. The organic layer was dried over anhydrous magnesium sulfate. The desiccant was removed by filtration and the solvent was evaporated under reduced pressure. The resulting residue was purified by silica gel column chromatography (chloroform-methanol) to obtain a pale brown oil (316 mg). The resulting pale brown oil (316 mg) was dissolved in acetonitrile (5 mL), and a 1 M aqueous sodium hydroxide solution (0.65 mL) was added thereto, followed by stirring at room temperature for 0.5 hours. Then, the solvent was evaporated under reduced pressure and the resulting residue was purified by ODS column chromatography (acetonitrile-water) to obtain a white amorphous solid. To this was added diethyl ether (10 mL), followed by stirring at room temperature for 0.5 hours. The solid was collected by filtration, washed with diethyl ether, and then heated and dried under reduced pressure to obtain sodium {5'-[(4'-{[(2R)-2,3-dihydroxypropyl]oxy}-2,2',6'-trimethylbiphenyl-3-yl)methoxy]-1',3'-dihydrospiro[cyclopropan-1,2'-inden]-1'-yl}acetate (60 mg) as a white solid.

In the same manner as in the method of Example 4, the compound of Example 4-1 shown in Tables below was prepared.

### Example 5

To a mixture of (5'-{[4'-(2-{[tert-butyl(dimethyl)silyl]oxy}ethoxy)-2,2',6'-trimethylbiphenyl-3-yl]methoxy}-1',3'-dihydrospiro[cyclopropan-1,2'-inden]-1'-yl)acetaldehyde (374 mg), 2-methyl-2-butene (0.22 mL), and dioxane (8 mL) was added a mixture of sodium chlorite (110 mg), sodium dihydrogen phosphate (300 mg), and water (2 mL) under ice-cooling, followed by stirring at room temperature for 1 hour. To the reaction mixture was added water, followed by extraction with ethyl acetate. The organic layer was dried over anhydrous magnesium sulfate. The desiccant was removed by filtration and the solvent was evaporated under reduced pressure. The resulting residue was dissolved in THF (5 mL), and 1 M hydrochloric acid (3 mL) was added thereto, followed by stirring at room temperature for 1 hour. To the reaction mixture was added a 1 M aqueous sodium hydroxide solution, followed by washing with diethyl ether. The aqueous layer was acidifed (pH 1) by the addition of 1 M hydrochloric acid, and extracted with chloroform. The organic layer was dried over anhydrous magnesium sulfate. The desiccant was removed by filtration and the solvent was evaporated under reduced pressure. The resulting residue was dissolved in THF (5 mL), and a 1 M aqueous sodium hydroxide solution (0.3 mL) was added thereto, followed by stirring at room temperature for 0.5 hours, and then the solvent was evaporated under reduced pressure. The resulting residue was purified by ODS column chromatography (acetonitrile-water) to obtain a white amorphous solid. To the resulting white amorphous solid was added diethyl ether (10 mL), followed by stirring at room temperature for 0.5 hours. The solid was collected by filtration, washed with diethyl ether, and then heated and dried under reduced pressure to obtain sodium (5'-{[4'-(2-hydroxyethoxy)-2,2',6'-trimethylbiphenyl-3-yl]methoxy}-1',3'-dihydrospiro[cyclopropan-1,2'-inden]-1'-yl)acetate (54 mg) as a white solid.

### Example 6

A mixture of ethyl {3-[(2,2',6'-trimethylbiphenyl-3-yl)methoxy]-9H-fluoren-9-yl}acetate (717 mg), a 1 M aqueous sodium hydroxide solution (3 mL), ethanol (3 mL), and THF (3 mL) was stirred at room temperature for 17 hours. To the reaction mixture were added water (15 mL) and 1 M hydrochloric acid (3 mL), followed by extraction with ethyl acetate. The organic layer was washed with a saturated aqueous sodium chloride solution and dried over anhydrous magnesium sulfate. The desiccant was removed by filtration and the solvent was evaporated under reduced pressure. The resulting residue was purified by silica gel column chromatography (hexane-ethyl acetate), and to the resulting yellow oil (672 mg) were added THF and a 1 M aqueous sodium hydroxide solution (1.5 mL). The solvent was evaporated under reduced pressure, then ethanol and a 1 M aqueous calcium chloride solution (0.75 mL) was added thereto, and the solvent was evaporated under reduced pressure. The resulting residue was purified by ODS column chromatography (acetonitrile-diluted hydrochloric acid) to obtain a yellow amorphous solid (241 mg). To the resulting yellow amorphous solid were added THF and a 1 M aqueous sodium hydroxide solution (0.54 mL), followed by concentration under reduced pressure. To the residue were added acetonitrile, water, and a 1 M aqueous calcium chloride solution (0.27 mL). The precipitated solid was collected by filtration, washed with water, and then heated and dried under reduced pressure to obtain 0.5 calcium {3-[(2,2',6'-trimethylbiphenyl-3-yl)methoxy]-9H-fluoren-9-yl}acetate (216 mg) as a light yellow solid.

### Example 7

To a mixture of ethyl {3-[(4'-{[(2R)-2,3-dihydroxypropyl]oxy}-2,2',6'-trimethylbiphenyl-3-yl)methoxy]-9H-fluoren-9-ylidene}acetate (600 mg), methanol (10 mL), and THF (1 mL) was added magnesium (turnings, 250 mg), followed by stirring at room temperature for 1 hour. To the reaction mixture were added 1 M hydrochloric acid (20 mL) and ethyl acetate, followed by stirring for a while, followed by extraction with ethyl acetate. The organic layer was washed with a saturated aqueous sodium chloride solution, and dried over anhydrous magnesium sulfate. The desiccant was removed by filtration and the solvent was evaporated under reduced pressure. The resulting residue was purified by silica gel column chromatography (hexane-ethyl acetate) to obtain ethyl {3-[(4'-{[(2R)-2,3-dihydroxypropyl]oxy} -2,2',6'-trimethylbiphenyl-3-yl)methoxy]-9H-fluoren-9-yl}acetate (192 mg) as a colorless oil.

### Example 8

A mixture of ethyl {3-[(4'-{[(2R)-2,3-dihydroxypropyl]oxy}-2,2',6'-trimethylbiphenyl-3-yl)methoxy]-9H-fluoren-9-yl}acetate (182 mg), a 1 M aqueous sodium hydroxide solution (0.65 mL), ethanol (2 mL), and THF (2 mL) was stirred at room temperature for 19 hours. To the reaction mixture were added water (10 mL) and 1 M hydrochloric acid (0.7 mL), followed by extraction with ethyl acetate. The organic layer was washed with a saturated aqueous sodium chloride solution and dried over anhydrous magnesium sulfate. The desiccant was removed by filtration and the solvent was evaporated under reduced pressure. The resulting residue was purified by silica gel column chromatography (chloroform-methanol). To the resulting yellow oil (172 mg) were added THF and a 1 M aqueous sodium hydroxide solution (0.32 mL), the solvent was evaporated under reduced pressure, and then the residue was purified by ODS column chromatography (acetonitrile-water). The solvent was evaporated under reduced pressure, and to the resulting residue (153 mg) were added acetonitrile and diethyl ether. The solid was collected by filtration, and heated and dried under reduced pressure to obtain sodium {3-[(4'-{[(2R)-2,3-dihydroxypropyl]oxy}-2,2',6'-trimethylbiphenyl-3-yl)methoxy]-9H-fluoren-9-yl}acetate (73 mg) as a white solid.

In the same manner as in the method of Example 8, the compounds of Examples 8-1 to 8-23 shown in Tables below were prepared.

### Example 9

To a mixture of {5'-[(2,2',6'-trimethylbiphenyl-3-yl)methoxy]-1',3'-dihydrospiro[cyclopropan-1,2'-inden]-1'-yl}acetaldehyde (342 mg), 2-methyl-2-butene (0.30 ml), and dioxane (8 mL) was added a mixture of sodium chlorite (150 mg), sodium dihydrogen phosphate (400 mg), and water (2 mL) under ice-cooling, followed by stirring at room temperature for 2 hours. To the reaction mixture was added water, followed by extraction with ethyl acetate, and then the organic layer was washed with a saturated aqueous sodium chloride solution and dried over anhydrous magnesium sulfate. The desiccant was removed by filtration and the solvent was evaporated under reduced pressure. The residue was dissovled in THF, a 1 M aqueous sodium hydroxide solution (0.80 mL) was added thereto, followed by concentration under reduced pressure, and then the residue was purified by ODS column chromatography (acetonitrile-water). To the resulting oil (73 mg) were added methanol and a 1 M aqueous calcium chloride solution (0.10 mL), followed by concentrating under reduced pressure. Then, to the residue was added water, and the solid was collected by filtration, washed with water, and then heated and dried under reduced pressure to obtain 0.5 calcium {5'-[(2,2',6'-trimethylbiphenyl-3-yl)methoxy]-1',3'-dihydrospiro[cyclopropan-1,2'-inden]-1'-yl}acetate (57 mg) as a white solid.

### Example 10

A mixture of 4'-{[(4S)-2,2-dimethyl-1,3-dioxolan-4-yl]methoxy}-2,2',6'-trimethylbiphenyl-3-carbaldehyde (300 mg), methyl (5'-amino-1',3'-dihydrospiro[cyclopropan-1,2'-inden]-1'-yl)acetate (200 mg), acetic acid (0.25 mL), and THF (7 mL) was stirred at room temperature for 4.5 hours. To the reaction mixture was added sodium triacetoxyborohydride (300 mg) under ice-cooling, followed by stirring for 1 hour under ice-cooling. Thereafter, the ice-bath was removed, followed by stirring for 17 hours while warming to room temperature. To the reaction mixture was added water (10 mL), followed by extraction with ethyl acetate. The organic layer was washed with a saturated aqueous sodium chloride solution and dried over anhydrous magnesium sulfate. The desiccant was removed by filtration and the solvent was evaporated under reduced pressure. The resulting residue was purified by silica gel column chromatography (hexane-ethyl acetate) to obtain methyl (5'-{[(4'-{[(4S)-2,2-dimethyl-1,3-dioxolan-4-yl]methoxy}-2,2',6'-trimethylbiphenyl-3-yl)methyl]amino}-1',3'-dihydrospiro[cyclopropan-1,2'-inden]-1'-yl)acetate (426 mg) as a brown oil.

In the same manner as in the method of Example 10, the compounds of Examples 10-1 to 10-22 shown in Tables below were prepared.

### Example 11

To a solution of methyl (5'-{[(4'-{[(4S)-2,2-dimethyl-1,3-dioxolan-4-yl]methoxy}-2,2',6'-trimethylbiphenyl-3-yl)methyl]amino}-1',3'-dihydrospiro[cyclopropan-1,2'-inden]-1'-yl)acetate (426 mg) in THF (3 mL) and methanol (3 mL) was added 1 M hydrochloric acid (3 mL), followed by stirring at room temperature for 4.5 hours. To the reaction mixture was added a 1 M aqueous sodium hydroxide solution (6 mL), followed by stirring at room temperature for 1.5 hours. The reaction mixture was warmed to 50°C and stirred for 3 hours. The reaction mixture was left to be cooled to room temperature, and a 10% aqueous citric acid solution (30 mL) was added thereto, followed by extraction with chloroform. The organic layer was dried over anhydrous magnesium sulfate. The desiccant was removed by filtration and the solvent was evaporated under reduced pressure. The resulting residue was dissolved in methanol (5 mL), and a 1 M aqueous sodium hydroxide solution (0.82 mL) was added thereto, followed by stirring at room temperature for 0.5 hours, and then the solvent was evaporated under reduced pressure. The resulting residue was purified by ODS column chromatography (acetonitrile-water) to obtain a white amorphous solid. To the resulting white amorphous solid was added diethyl ether (10 mL), followed by stirring at room temperature for 0.5 hours. The solid was collected by filtration, washed with diethyl ether, and then heated and dried under reduced pressure to obtain sodium (5'-{[(4'-{[(2R)-2,3-dihydroxypropyl]oxy} -2,2',6'-trimethylbiphenyl-3-yl)methyl] amino}- 1',3'-dihydrospiro[cyclopropan-1,2'-inden]-1'-yl)acetate (220 mg) as a white solid.

In the same manner as in the method of Example 11, the compound of Examples 11-1 shown in Tables below was prepared.

### Example 12

To a mixed solution of [3-(2-{[(4S)-2,2-dimethyl-1,3-dioxolan-4-yl]methoxy}-4,6-dimethylpyrimidin-5-yl)-2-methylphenyl]methanol (1.14 g), ethyl (3-hydroxy-9H-fluoren-9-yl)acetate (0.92 g), tributylphosphine (1.2 mL), and THF (12 mL) was added 1,1'-(azodicarbonyl)dipiperidine (1.20 g) under ice-cooling, and the reaction mixture was stirred at room temperature for 2 days. The insoluble materials were separated by filtration, and then the solvent was evaporated under reduced pressure. The resulting residue was purified by silica gel column chromatography (hexane-ethyl acetate) to obtain ethyl (3-{[3-(2-{[(4S)-2,2-dimethyl-1,3-dioxolan-4-yl]methoxy}-4,6-dimethylpyrimidin-5-yl)-2-methylbenzyl]oxy}-9H-fluoren-9-yl)acetate (1.30 g) as a pale yellow amorphous solid.

In the same manner as in the method of Example 12, the compounds of Examples 12-1 to 12-6 shown in Tables below were prepared.

### Example 13

A mixture of ethyl (3-{[3-(2-{[(4S)-2,2-dimethyl-1,3-dioxolan-4-yl]methoxy}-4,6-dimethylpyrimidin-5-yl)-2-methylbenzyl]oxy}-9H-fluoren-9-yl)acetate (1.30 g), 1 M hydrochloric acid (6 mL), and THF (6 mL) was stirred at room temperature for 1.5 hours. To the reaction mixture was added 1 M hydrochloric acid (6 mL), followed by stirring at room temperature for 3 hours. To the reaction mixture was added a saturated aqueous sodium hydrogen carbonate solution (20 ml), followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was dried under reduced pressure to obtain ethyl (3-{[3-(2-{[(2R)-2,3-dihydroxypropyl]oxy}-4,6-dimethylpyrimidin-5-yl)-2-methylbenzyl]oxy}-9H-fluoren-9-yl)acetate (1.15 g) as a pale yellow amorphous solid.

In the same manner as in the method of Example 13, the compounds of Examples 13-1 to 13-3 shown in Tables below were prepared.

### Example 14

To a mixture of (5'-{[4'-(3-hydroxy-3-methylbutoxy)-2,2',6'-trimethylbiphenyl-3-yl]methoxy}-1',3'-dihydrospiro[cyclopropan-1,2'-inden]-1'-yl)acetaldehyde (457 mg), 2-methyl-2-butene (0.30 mL), and dioxane (10 mL) was added a mixture of sodium chlorite (150 mg), sodium dihydrogen phosphate (420 mg), and water (3 mL) under ice-cooling, followed by stirring at room temperature for 1 hour. To the reaction mixture was added water, followed by extraction with ethyl acetate. The organic layer was dried over anhydrous magnesium sulfate. The desiccant was removed by filtration and the solvent was evaporated under reduced pressure. The resulting residue was dissolved in methanol (6 mL), a 1 M aqueous sodium hydroxide solution (2 mL) was added thereto, followed by stirring at room temperature for 0.5 hours, and then the solvent was evaporated under reduced pressure. The resulting residue was purified by ODS column chromatography (acetonitrile-water) to obtain a white amorphous solid. To the resulting white amorphous solid was added diisopropyl ether (10 mL), followed by stirring at room temperature for 0.5 hours. The solid was collected by filtration, washed with diisopropyl ether, and then heated and dried under reduced pressure to obtain sodium (5'-{[4'-(3-hydroxy-3-methylbutoxy)-2,2',6'-trimethylbiphenyl-3-yl]methoxy}-1',3'-dihydrospiro[cyclopropan-1,2'-inden]-1'-yl)acetate (252 mg) as a pale blue solid.

### Example 15

To a solution of methyl [5'-({[4'-(3-{[tert-butyl(dimethyl)silyl]oxy}propoxy)-2,2',6'-trimethylbiphenyl-3-yl]methyl}amino)-1',3'-dihydrospiro[cyclopropan-1,2'-inden]-1'-yl]acetate (1.11 g) in THF (6 mL) and methanol (6 mL) was added 1 M hydrochloric acid (5 mL), followed by stirring at room temperature for 2 hours, and then a 1 M aqueous sodium hydroxide solution (10 mL) was added thereto, followed by stirring at 50°C for 2 hours. Thereafter, a 1 M aqueous sodium hydroxide solution (1 mL) was added thereto, followed by further stirring at 50°C for 1 hour. The reaction mixture was cooled to room temperature, a 10% aqueous citric acid solution (50 mL) was added thereto, followed by extraction with chloroform. To the organic layer were added anhydrous magnesium sulfate and activated carbon (0.5 g). The desiccant and activated carbon were removed by filtration, and the solvent was evaporated under reduced pressure. The resulting residue was purified by silica gel column chromatography (chloroform-methanol) to obtain a white amorphous solid (789 mg).
The resulting white amorphous solid was dissolved in methanol (5 mL), a 1 M aqueous sodium hydroxide solution (1.6 mL) was added thereto, followed by stirring at room temperature for 0.5 hours, and then the solvent was evaporated under reduced pressure. To the resulting residue was added diethyl ether (10 mL), followed by stirring at room temperature for 0.5 hours. The solid was collected by filtration, washed with diethyl ether, and then heated and dried under reduced pressure to obtain sodium [5'-({[4'-(3-hydroxypropoxy)-2,2',6'-trimethylbiphenyl-3-yl]methyl}amino)-1',3'-dihydrospiro[cyclopropan-1,2'-inden]-1'-yl]acetate (670 mg) as a white solid.

### Example 16

To a solution of (6-{[4'-(methoxymethoxy)-2,2',6'-trimethylbiphenyl-3-yl]methoxy}-3H-spiro[1-benzofuran-2,1'-cyclopropan]-3-yl)acetonitrile (6.08 g) in ethanol (125 mL) were added water (30 mL) and potassium hydroxide (60.00 g), followed by stirring at 150°C for 8 hours in a stainless steel-made autoclave, and then cooled to room temperature. To the reaction mixture was added 1 M hydrochloric acid (1100 mL), followed by extraction with ethyl acetate. The organic layer was washed with a saturated aqueous sodium chloride solution and dried over anhydrous magnesium sulfate. The desiccant was removed by filtration and the solvent was evaporated under reduced pressure to obtain a pale brown amorphous solid (6.95 g).
To a solution of the resulting pale brown amorphous solid (6.95 g) in DMF (100 mL) were added potassium hydrogen carbonate (2.60 g) and methyl iodide (2.40 mL), and the reaction mixture was stirred at room temperature for 2 hours. To the reaction mixture were added potassium hydrogen carbonate (2.60 g) and methyl iodide (2.40 mL), and the reaction mixture was stirred at room temperature for 2 hours. To the reaction mixture were added potassium hydrogen carbonate (2.60 g) and methyl iodide (2.40 mL), and the reaction mixture was stirred at room temperature for 1 hour. To the reaction mixture was added water, followed by extraction with a toluene-ethyl acetate solution. The organic layer was washed with water and a saturated aqueous sodium chloride solution, and dried over anhydrous magnesium sulfate. The desiccant was removed by filtration and the solvent was evaporated under reduced pressure. The resulting residue was purified by silica gel column chromatography (hexane-ethyl acetate) to obtain methyl (6-{[4'-(methoxymethoxy)-2,2',6'-trimethylbiphenyl-3-yl]methoxy}-3H-spiro[1-benzofuran-2,1'-cyclopropan]-3-yl)acetate (5.60 g) as a colorless syrup.

### Example 17

To a solution of methyl (6-{[4'-(methoxymethoxy)-2,2',6'-trimethylbiphenyl-3-yl]methoxy}-3H-spiro[1-benzofuran-2,1'-cyclopropan]-3-yl)acetate (5.59 g) in methanol (100 mL) and THF (15 mL) was added concentrated hydrochloric acid (2.50 mL) at room temperature, followed by stirring at 55°C for 1 hour. The reaction mixture was cooled to room temperature, and water was added thereto, followed by extraction with ethyl acetate. The organic layer was washed with a saturated aqueous sodium chloride solution and dried over anhydrous magnesium sulfate. The desiccant was removed by filtration and the solvent was evaporated under reduced pressure to obtain methyl (6-{[4'-hydroxy-2,2',6'-trimethylbiphenyl-3-yl]methoxy}-3H-spiro[1-benzofuran-2,1'-cyclopropan]-3-yl)acetate (5.02 g) as a white amorphous solid.

### Example 18

To a solution of methyl [5'-({3-[2-(2-{[tert-butyl(dimethyl)silyl]oxy}ethoxy)-4,6-dimethylpyrimidin-5-yl]-2-methylbenzyl}oxy)-1',3'-dihydrospiro[cyclopropan-1,2'-inden]-1'-yl]acetate (843 mg) in THF (8 mL) was added a 1 M tetrabutyl ammonium fluoride solution in THF (4.0 mL) under ice-cooling, followed by stirring at room temperature for 1 hour. To the reaction mixture were added a 10% aqueous citric acid solution (10 mL) and water (10 mL), followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (hexane-ethyl acetate) to obtain methyl [5'-({3-[2-(2-hydroxyethoxy)-4,6-dimethylpyrimidin-5-yl]-2-methylbenzylloxy)-1',3'-dihydrospiro[cyclopropan-1,2'-inden]-1'-yl]acetate (589 mg) as a white amorphous solid.

In the same manner as in the method of Example 18, the compound of Example 18-1 shown in Tables below was prepared.

### Example 19

To a solution of {[3'-({[1'-(2-methoxy-2-oxoethyl)-1',3'-dihydrospiro[cyclopropan-1,2'-inden]-5'-yl]amino}methyl)-2,2',6-trimethylbiphenyl-4-yl]oxy}acetic acid in DMF (8 mL) were added 1H-benzotriazol-1-ol (120 mg), triethylamine (0.13 mL), methylamine hydrochloride (60 mg), and N-[3-(dimethylamino)propyl]-N'-ethylcarbodiimide hydrochloride (170 mg), followed by stirring at room temperature for 14.5 hours. To the reaction mixture was added water, followed by extraction with ethyl acetate. The organic layer was washed with water and a saturated aqueous sodium chloride solution, and dried over anhydrous magnesium sulfate. The desiccant was removed by filtration and the solvent was evaporated under reduced pressure. The resulting residue was purified by silica gel column chromatography (hexane-ethyl acetate) to obtain methyl {5'-[({2,2',6'-trimethyl-4'-[2-(methylamino)-2-oxoethoxy]biphenyl-3-yl}methyl)amino]-1',3'-dihydrospiro[cyclopropan-1,2'-inden]-1'-yl}acetate (320 mg) as a white amorphous solid.

In the same manner as in the method of Example 19, the compounds of Examples 19-1 to 19-2 shown in Tables below were prepared.

### Example 20

To a solution of methyl (6-{[4'-hydroxy-2,2',6'-trimethylbiphenyl-3-yl]methoxy}-3H-spiro[1-benzofuran-2,1'-cyclopropan]-3-yl)acetate (206 mg) in DMF (4 mL) were added cesium carbonate (220 mg) and 2-bromoethyl acetate (0.06 mL), and the reaction mixture was stirred at 65°C for 15 hours. The reaction mixture was cooled to room temperature, and water and a saturated aqueous sodium chloride solution was added thereto, followed by extraction with ethyl acetate. The organic layer was washed with a saturated aqueous sodium chloride solution and dried over anhydrous magnesium sulfate. Then, the desiccant was removed by filtration and the solvent was evaporated under reduced pressure.
The resulting residue was dissolved in methanol (3 mL) and THF (3 mL), and a 1 M aqueous sodium hydroxide solution (2.5 mL) was added thereto. The reaction mixture was warmed to 50°C, followed by stirring for 2 hours. The reaction mixture was cooled to room temperature, and 1 M hydrochloric acid (3.0 mL) and water (30 mL) were added thereto, followed by extraction with chloroform. The organic layer was dried over anhydrous magnesium sulfate, then the desiccant was removed by filtration, and the solvent was evaporated under reduced pressure. The resulting residue was purified by silica gel column chromatography (chloroform-methanol) to obtain a light yellow amorphous solid (191 mg). The resulting light yellow amorphous solid (191 mg) was dissolved in methanol (2 mL), and a 1 M aqueous sodium hydroxide solution (0.39 mL) was added thereto, followed by concentration under reduced pressure. To the resulting residue was added diethyl ether (10 mL), followed by stirring at room temperature for 0.5 hours. The solid was collected by filtration, and heated and dried under reduced pressure to obtain sodium (6-{[4'-(2-hydroxyethoxy)-2,2',6'-trimethylbiphenyl-3-yl]methoxy}-3H-spiro[1-benzofuran-2,1'-cyclopropan]-3-yl)acetate (170 mg)) as a pale yellow solid.

### Example 21

To a solution of methyl (6-{[4'-hydroxy-2,2',6'-trimethylbiphenyl-3-yl]methoxy}-3H-spiro[1-benzofuran-2,1'-cyclopropan]-3-yl)acetate (274 mg) in DMF (5 mL) were added cesium carbonate (293 mg) and 3-hydroxy-3-methylbutyl 4-methylbenzenesulfate (185 mg), and the reaction mixture was stirred at 65°C for 15 hours. The reaction mixture was cooled to room temperature, and water and a saturated aqueous sodium chloride solution were added thereto, followed by extraction with ethyl acetate. The organic layer was washed with a saturated aqueous sodium chloride solution and dried over anhydrous magnesium sulfate. Then, the desiccant was removed by filtration and the solvent was evaporated under reduced pressure.
The resulting residue was dissolved in methanol (3 mL) and THF (3 mL), and a 1 M aqueous sodium hydroxide solution (3.0 mL) was added thereto. The reaction mixture was warmed to 50°C, followed by stirring for 2 hours. The reaction mixture was cooled to room temperature, and 1 M hydrochloric acid (3.5 mL) and water (30 mL) were added thereto, followed by extraction with chloroform. The organic layer was dried over anhydrous magnesium sulfate, then the desiccant was removed by filtration, and the solvent was evaporated under reduced pressure. The resulting residue was purified by silica gel column chromatography (chloroform-methanol) to obtain a brown syrup (331 mg). The resulting brown syrup (331 mg) was dissolved in methanol (1 mL), and a 1 M aqueous sodium hydroxide solution (0.60 mL) was added thereto. This solution was purified by ODS column chromatography (acetonitrile-water) to obtain a light yellow amorphous solid (221 mg). To the resulting solid (221 mg) was added diisopropyl ether (10 mL), followed by stirring at room temperature for 0.5 hours. The solid was collected by filtration, and heated and dried under reduced pressure to obtain sodium (6-{[4'-(3-hydroxy-3-methylbutoxy)-2,2',6'-trimethylbiphenyl-3-yl]methoxy}-3H-spiro[1-benzofuran-2,1'-cyclopropan]-3-yl)acetate (175 mg) as a light yellow solid.

In the same manner as in the method of Example 21, the compounds of Examples 21-1 to 21-6 shown in Tables below were prepared.

### Example 22

To a solution of methyl {6-[(4'-hydroxy-2,2',6'-trimethylbiphenyl-3-yl)methoxy]-3H-spiro[1-benzofuran-2,1'-cyclopropan]-3-yl}acetate (356 mg) in DMF (6 mL) were added cesium carbonate (380 mg) and [(4 R)-2,2-dimethyl-1,3-dioxolan-4-yl]methyl 4-methylbenzenesulfonate (250 mg), and the reaction mixture was stirred at 65°C for 15 hours. The reaction mixture was cooled to room temperature, and water and a saturated aqueous sodium chloride solution were added thereto, followed by extraction with ethyl acetate. The organic layer was washed with a saturated aqueous sodium chloride solution and dried over anhydrous magnesium sulfate. The desiccant was removed by filtration and the solvent was evaporated under reduced pressure.
The resulting residue was dissolved in methanol (4 mL) and and THF (4 mL), and 1 M hydrochloric acid (4 mL) was added thereto, followed by stirring at 50°C for 1.5 hours. To the reaction mixture was added a 5 M aqueous sodium hydroxide solution (2 mL), followed by stirring at 50°C for 3 hours. The reaction mixture was cooled to room temperature, and 1 M hydrochloric acid (10 mL) and water (20 mL) were added thereto, followed by extraction with chloroform. The organic layer was dried over anhydrous magnesium sulfate, then the desiccant was removed by filtration, and the solvent was evaporated under reduced pressure. The resulting residue was dissolved in methanol (1 mL), and a 1 M aqueous sodium hydroxide solution (0.80 mL) was added thereto, followed by purification by ODS column chromatography (acetonitrile-water) to obtain a light yellow amorphous solid (305 mg). To the resulting light yellow amorphous solid (305 mg) was added diethyl ether (10 mL), followed by stirring at room temperature for 0.5 hours. The solid was collected by filtration, washed with diethyl ether, and then heated and dried under reduced pressure to obtain sodium {6-[(4'-{[(2R)-2,3-dihydroxypropyl]oxy}-2,2',6'-trimethylbiphenyl-3-yl)methoxy]-3H-spiro[1-benzofuran-2,1'-cyclopropan]-3-yl}acetate (266 mg)) as a pale yellow solid.

In the same manner as in the method of Example 22, the compounds of Examples 22-1 to 22-3 shown in Tables below were prepared.

### Example 23

A mixture of methyl [5'-({3-[2-(2-hydroxyethoxy)-4,6-dimethylpyrimidin-5-yl]-2-methylbenzyl}oxy)-1',3'-dihydrospiro[cyclopropan-1,2'-inden]-1'-yl]acetate (589 mg), a 1 M aqueous sodium hydroxide solution (6 mL), THF (3 mL), and ethanol (3 mL) was stirred at room temperature for 14 hours. To the reaction mixture was added a 10% aqueous citric acid solution (12 mL), followed by extraction with ethyl acetate. The organic layer was washed with a saturated aqueous sodium chloride solution and then dried over anhydrous magnesium sulfate. The desiccant was removed by filtration, and then the solvent was evaporated under reduced pressure. The resulting residue was purified by silica gel column chromatography (chloroform-methanol) to obtain a white amorphous solid (551 mg). To the resulting white amorphous solid was added hexane, followed by stirring. The solid was collected by filtration, washed with hexane, and then heated and dried under reduced pressure to obtain [5'-({3-[2-(2-hydroxyethoxy)-4,6-dimethylpyrimidin-5-yl]-2-methylbenzyl}oxy)-1',3'-dihydrospiro[cyclopropan-1,2'-inden]-1'-yl]acetic acid (462 mg) as a white powder solid.

In the same manner as in the method of Example 23, the compounds of Examples 23-1 to 23-3 shown in Tables below were prepared.

### Example 24

A mixture of methyl (5'-amino-1',3'-dihydrospiro[cyclopropan-1,2'-inden]-1'-yl)acetate (400 mg), 3-[2-(3-hydroxy-3-methylbutoxy)-4,6-dimethylpyrimidin-5-yl]-2-methylbenzaldehyde (625 mg), acetic acid (0.52 ml), and THF (7 mL) was stirred at room temperature for 3 hours, and then sodium triacetoxyborohydride (740 mg) was added thereto, followed by stirring at room temperature for additional 5 hours. Thereafter, a saturated aqueous sodium hydrogen carbonate solution (20 mL) was added thereto, followed by extraction with chloroform. The organic layer was dried over anhydrous magnesium sulfate. The desiccant was removed by filtration and the solvent was evaporated under reduced pressure. The resulting residue was purified by silica gel column chromatography (chloroform-methanol) to obtain a yellow amorphous solid (977 mg).
To a solution of the resulting yellow amorphous solid (977 mg) in THF (10 mL) and methanol (10 mL) was added a 1 M aqueous sodium hydroxide solution (9 mL). The reaction mixture was stirred at 50°C for 1 hour, and cooled to room temperature, and then to stand at room temperature for 12 hours. To the reaction mixture were added a 10% aqueous citric acid solution (20 mL) and water (30 mL), followed by extraction with a 2-propanol-chloroform solution. The organic layer was dried over anhydrous magnesium sulfate. The desiccant was removed by filtration and the solvent was evaporated under reduced pressure. The resulting residue was dissolved in methanol (2 mL), and a 1 M aqueous sodium hydroxide solution (1.8 mL) was added thereto, followed by purification by ODS column chromatography (acetonitrile-water) to obtain a light yellow amorphous solid (792 mg). To the resulting light yellow amorphous solid (792 mg) was added diisopropyl ether (12 mL), followed by stirring at room temperature for 0.5 hours. The solid was collected by filtration, washed with diisopropyl ether, and then heated and dried under reduced pressure to obtain sodium [5'-({3-[2-(3-hydroxy-3-methylbutoxy)-4,6-dimethylpyrimidin-5-yl]-2-methylbenzyl}amino)-1',3'-dihydrospiro[cyclopropan-1,2'-inden]-1'-yl]acetate (736 mg)) as a pale yellow solid.

### Example 25

[5'-({[4'-(2-Hydroxyethoxy)-2,2',6'-trimethylbiphenyl-3-yl]methyl}amino)-1',3'-dihydrospiro[cyclopropan-1,2'-inden]-1'-yl]acetic acid (100 mg) was subjected to optical resolution by means of HPLC [DAICEL CHIRALPAK AD-H, semi-preparative column (10×250 mm, 5 µm), mobile phase: hexane/ethanol = 70/30 (0.1% trifluoroacetic acid added)] to obtain 39 mg and 40 mg of optically active forms 25a and 25b of [5'-({[4'-(2-hydroxyethoxy)-2,2',6'-trimethylbiphenyl-3-yl]methyl}amino)-1',3'-dihydrospiro[cyclopropan-1,2'-inden]-1'-yl]acetic acid, respectively, at the first peak and the second peak (>99%ee) (absolute configuration not determined).

In the same manner as in the method of Example 25, the compounds of Examples 25-1a and 25-1b to Examples 25-7a and 25-7b shown in Tables below were collected by separation.

### Example 26

To a solution of methyl [5'-({3-[2-(2-{[tert-butyl(dimethyl)silyl]oxy}ethoxy)-4,6-dimethylpyrimidin-5-yl]-2-methylbenzyl}amino)-1',3'-dihydrospiro[cyclopropan-1,2'-inden]-1'-yl]acetate (878 mg) in THF (4.5 ml) was added 1 M hydrochloric acid (9 mL), followed by stirring at room temperature for 3 hours. To the reaction mixture was added a 5 M aqueous sodium hydroxide solution (3 mL), followed by stirring at room temperature for 1 hour. The reaction mixture was stirred at 50°C for 2 hours. The reaction mixture was cooled to room temperature, and a 10% aqueous citric acid solution (10 mL) was added thereto, followed by extraction with 2-propanol-chloroform solution. The organic layer was dried over anhydrous magnesium sulfate, the desiccant was removed by filtration, and then the solvent was evaporated under reduced pressure. The resulting residue was purified by silica gel column chromatography (chloroform-methanol) to obtain a pale yellow amorphous solid (736 mg). To the resulting pale yellow amorphous solid was added hexane, followed by stirring. The solid was collected by filtration, washed with hexane, and then heated and dried under reduced pressure to obtain [5'-({3-[2-(2-hydroxyethoxy)-4,6-dimethylpyrimidin-5-yl]-2-methylbenzyl}amino)-1',3'-dihydrospiro[cyclopropan-1,2'-inden]-1'-yl]acetic acid (573 mg) as a white powder solid.

In the same manner as in the method of Example 26, the compounds of Examples 26-1 to 26-3 shown in Tables below were prepared.

### Example 27

To a solution of methyl (5'-{[3-(2-{[(4S)-2,2-dimethyl-1,3-dioxolan-4-yl]methoxy} -4,6-dimethylpyrimidin-5-yl)-2-methylbenzyl] amino}- 1',3'-dihydrospiro[cyclopropan-1,2'-inden]-1'-yl)acetate (850 mg) in THF (4.5 mL) was added 1 M hydrochloric acid (9 mL), followed by stirring at room temperature for 3 hours. To the reaction mixture was added a 5 M aqueous sodium hydroxide solution (3 mL), followed by stirring at room temperature for 1 hour, and then stirring at 50°C for additional 2 hours. The reaction mixture was cooled to room temperature, and a 10% aqueous citric acid solution (10 mL) was added thereto, followed by extraction with a 2-propanol-chloroform solution. The organic layer was dried over anhydrous magnesium sulfate, the desiccant was removed by filtration, and then the solvent was evaporated under reduced pressure. The resulting residue was purified by silica gel column chromatography (chloroform-methanol) to obtain a pale brown amorphous solid (770 mg). To the resulting pale brown amorphous solid was added hexane, followed by stirring. The solid was collected by filtration, washed with hexane, and then heated and dried under reduced pressure to obtain (5'-{[3-(2-{[(2R)-2,3-dihydroxypropyl]oxy}-4,6-dimethylpyrimidin-5-yl)-2-methylbenzyl]amino}-1',3'-dihydrospiro[cyclopropan-1,2'-inden]-1'-yl)acetic acid (642 mg) as a pale brown powder solid.

In the same manner as in the method of Example 27, the compound of Example 27-1 shown in Tables below was prepared.

### Example 28

To a solution of methyl (6-{[4'-hydroxy-2,2',6'-trimethylbiphenyl-3-yl]methoxy}-3H-spiro[1-benzofuran-2,1'-cyclopropan]-3-yl)acetate (300 mg) in DMF (4 mL) were added potassium phosphate (350 mg) and (3-bromopropoxy)(tert-butyl) dimethylsilane (0.17 mL), and the reaction mixture was stirred at 65°C for 13 hours. The reaction mixture was cooled to room temperature, and water was added thereto, followed by extraction with a toluene-ethyl acetate solution. The aqueous layer was further extracted with a toluene-ethyl acetate solution. The organic layer was combined, washed with water and a saturated aqueous sodium chloride solution, and then dried over anhydrous magnesium sulfate. The desiccant was removed by filtration and the solvent was evaporated under reduced pressure. The resulting residue was purified by silica gel column chromatography (hexane-ethyl acetate) to obtain a colorless oil.
The resulting colorless oil was dissolved in methanol (2 mL) and THF (2 mL), and 1 M hydrochloric acid (1 mL) was added thereto, followed by stirring at 50°C for 61 hours. Then, a 1 M aqueous sodium hydroxide solution (2.6 mL) was added thereto, followed by stirring at 50°C for 8 hours. The reaction mixture was cooled to room temperature and left to stand at room temperature for 39 hours, and then the solvent was evaporated under reduced pressure. The resulting residue was purified by ODS column chromatography (acetonitrile-water) to obtain a white amorphous solid. To the resulting white amorphous solid was added diisopropyl ether (10 mL), followed by stirring at room temperature for 0.5 hours. The solid was collected by filtration, washed with diisopropyl ether, and then heated and dried under reduced pressure to obtain sodium (6-{[4'-(3-hydroxypropoxy)-2,2',6'-trimethylbiphenyl-3-yl]methoxy}-3H-spiro[1-benzofuran-2,1'-cyclopropan]-3-yl)acetate (278 mg) as a white solid.

### Example 29

To a solution of methyl (6-{[4'-hydroxy-2,2',6'-trimethylbiphenyl-3-yl]methoxy}-3H-spiro[1-benzofuran-2,1'-cyclopropan]-3-yl)acetate (300 mg) in DMF (4 mL) were added potassium phosphate (350 mg) and tert-butyl (3-bromopropyl)carbamate (180 mg), and the reaction mixture was stirred at 65°C for 13 hours. The reaction mixture was cooled to room temperature, and water was added thereto, followed by extraction with a toluene-ethyl acetate solution. The organic layer was washed with water and a saturated aqueous sodium chloride solution, and then dried over anhydrous magnesium sulfate. The desiccant was removed by filtration and the solvent was evaporated under reduced pressure. The resulting residue was purified by silica gel column chromatography (hexane-ethyl acetate) to obtain a colorless oil.
To a solution of the resulting colorless oil in methanol (1 mL) was added a 4 M hydrogen chloride solution (1 mL) in dioxane, followed by stirring at room temperature for 1 hour, and then the solvent was evaporated under reduced pressure. To the resulting residue was added a saturated aqueous sodium hydrogen carbonate solution, followed by extraction with chloroform. The organic layer was dried over anhydrous magnesium sulfate. The desiccant was removed by filtration and the solvent was evaporated under reduced pressure. The resulting residue was dissolved in dichloromethane (4 mL), and triethylamine (0.12 mL), methanesulfonyl chloride (0.06 mL) was added thereto, followed by stirring at room temperature for 41.5 hours. To the reaction mixture was added water, followed by extraction with chloroform. The organic layer was dried over anhydrous magnesium sulfate. The desiccant was removed by filtration and the solvent was evaporated under reduced pressure. The resulting residue was dissolved in THF (2 mL) and methanol (2 mL), and a 1 M aqueous sodium hydroxide solution (1.5 mL) was added thereto, followed by stirring at 55°C for 7 hours, then cooling to room temperature, and leaving to stand at room temperature for 16 hours. The solvent was evaporated under reduced pressure and the resulting residue was purified by ODS column chromatography (acetonitrile-water) to obtain a white amorphous solid. To the resulting white amorphous solid was added diisopropyl ether (10 mL), followed by stirring at room temperature for 0.5 hours. The solid was collected by filtration, washed with diisopropyl ether, and then heated and dried under reduced pressure to obtain sodium {6-[(2,2',6'-trimethyl-4'-{3-[(methylsulfonyl)amino]propoxy}biphenyl-3-yl)methoxy]-3H-spiro[1-benzofuran-2,1'-cyclopropan]-3-yl}acetate (107 mg) as a white solid.

### Example 30

(3-{[4'-(2-Hydroxyethoxy)-2,2',6'-trimethylbiphenyl-3-yl]methoxy}-9H-fluoren-9-yl)acetic acid (1.26 g) was subjected to optical resolution by means of HPLC [DAICEL CHIRALCEL OJ-H, semi-preparative column (10×250 mm, 5 µm), mobile phase: hexane/ethanol = 60/40] to obtain 389 mg and 438 mg of optically active forms 30a and 30b of (3-{[4'-(2-hydroxyethoxy)-2,2',6'-trimethylbiphenyl-3-yl]methoxy}-9H-fluoren-9-yl)acetic acid, respectively, at the first peak and the second peak (>99%ee) (absolute configuration not determined).

### Example 31

In the same manner as in the method of Example 20, the compounds of Examples 31 and 31-1 to 31-2 shown in Tables below were prepared.

### Example 32

In the same manner as in the method of Example 22, the compounds of Examples 32 and 32-1 shown in Tables below were prepared.

### Example 33

Under nitrogen air flow, to a solution of {5'-[(2,2',6'-trimethylbiphenyl-3-yl)methoxy]-1',3'-dihydrospiro[cyclopropan-1,2'-inden]-1'-yl}acetonitrile (340 mg) in toluene (10 mL) was added dropwise a 0.99 M diisobutylaluminum hydride solution in toluene (1.3 mL) at -55°C or lower, followed by stirring at the same temperature for 45 minutes. Further, a 0.99 M diisobutylaluminum hydride solution in toluene (1 mL) was added thereto, followed by stirring at the same temperature for 0.5 hours. To the reaction mixture were added ethyl acetate and a saturated aqueous potassium sodium (+)-tartrate solution, followed by stirring at room temperature and then extracting with ethyl acetate. The organic layer was washed with a saturated aqueous sodium chloride solution and dried over anhydrous magnesium sulfate. Then, the desiccant was removed by filtration and the solvent was evaporated under reduced pressure to obtain a white amorphous solid (350 mg).
To a mixture of the resulting white amorphous solid (350 mg), 2-methyl-2-butene (0.30 mL), and dioxane (8 mL) was added a mixture of sodium chlorite (150 mg), sodium dihydrogen phosphate (400 mg), and water (2 mL) under ice-cooling, followed by stirring at room temperature for 2 hours. To the reaction mixture was added water, followed by extraction with ethyl acetate, and then the organic layer was washed with a saturated aqueous sodium chloride solution and dried over anhydrous magnesium sulfate. The desiccant was removed by filtration and the solvent was evaporated under reduced pressure. The residue was dissovled in THF, a 1 M aqueous sodium hydroxide solution (0.80 mL) was added thereto, followed by concentrating under reduced pressure, and then the residue was purified by ODS column chromatography (acetonitrile-water). To the resulting oil (73 mg) were added methanol and a 1 M aqueous calcium chloride solution (0.10 mL), the solvent was evaporated under reduced pressure. Then, to the residue was added water, and the solid was collected by filtration. The resulting solid was washed with water, and heated and dried under reduced pressure to obtain 0.5 calcium {5'-[(2,2',6'-trimethylbiphenyl-3-yl)methoxy]-1',3'-dihydrospiro[cyclopropan-1,2'-inden]-1'-yl}acetate (57 mg) as a white solid.

For the Example Compounds, the structures are shown in Tables 26 to 44 and the physicochemical data are shown in Tables 45 to 56.

**[Table 26]**

| **Ex** | **Structure** |
|---|---|
| **1** | 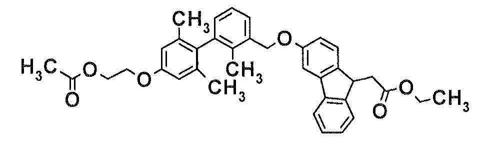 |
| **1-1** | 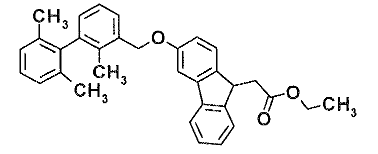 |
| **2** | 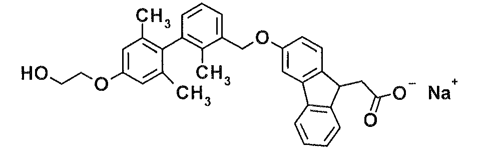 |
| **2-1** | 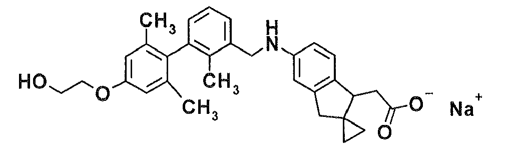 |
| **2-2** | 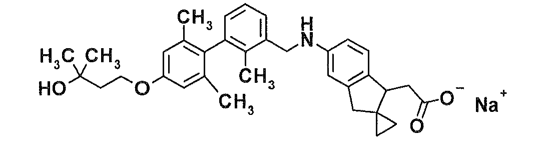 |
| **3** | 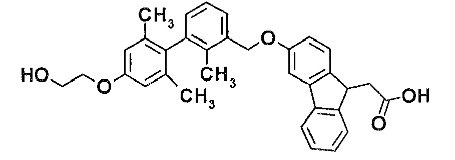 |
| **3-1** | 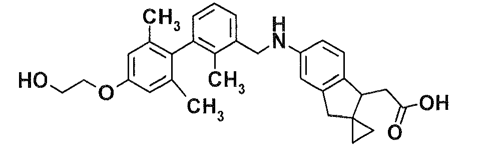 |
| **4** | 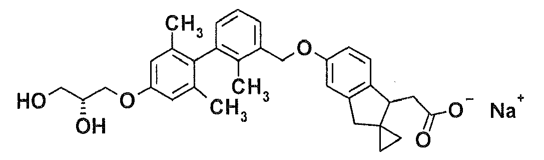 |

**[Table 27]**

| **Ex** | **Structure** |
|---|---|
| **4-1** | 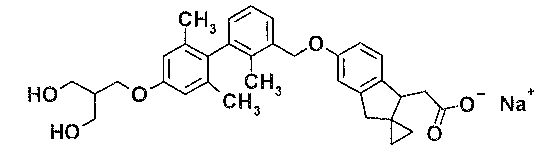 |
| **5** | 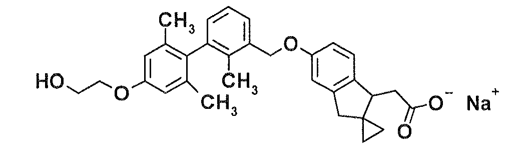 |
| **6** | 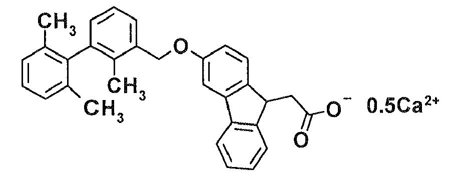 |
| **7** | 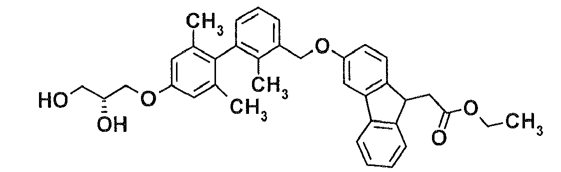 |
| **8** | 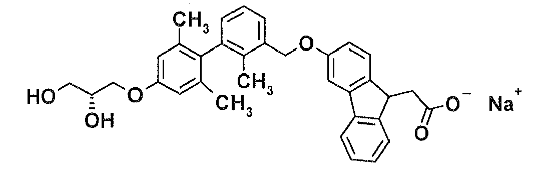 |
| **8-1** | 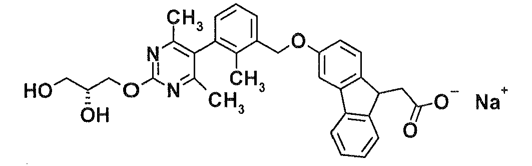 |
| **8-2** | 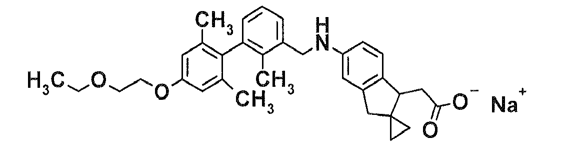 |

**[Table 28]**

| **Ex** | **Structure** |
|---|---|
| **8-3** | 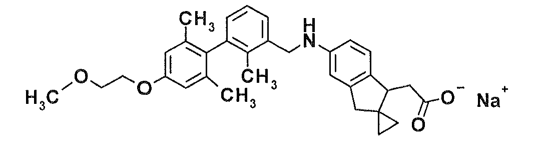 |
| **8-4** | 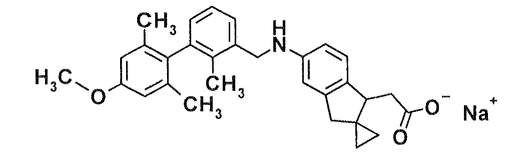 |
| **8-5** | 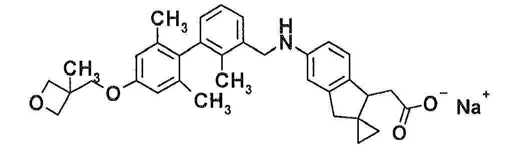 |
| **8-6** | 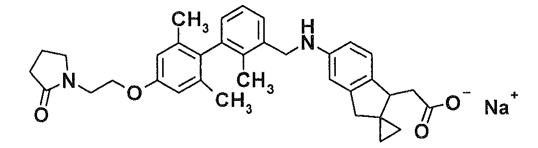 |
| **8-7** | 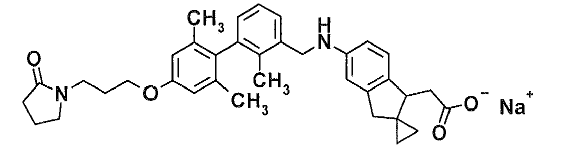 |
| **8-8** | 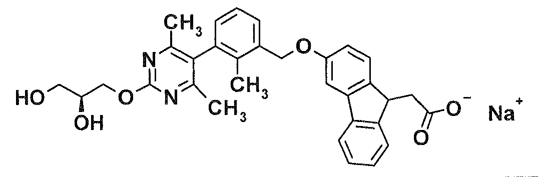 |
| **8-9** | 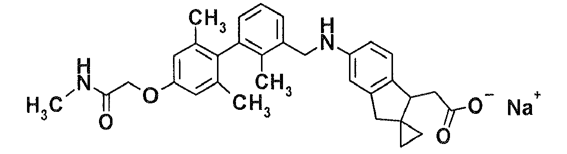 |
| **8-10** | 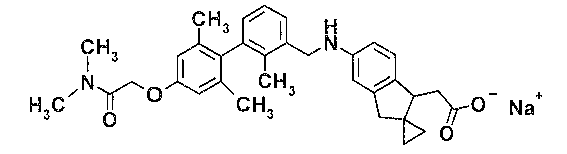 |

**[Table 29]**

| **Ex** | **Structure** |
|---|---|
| **8-11** | 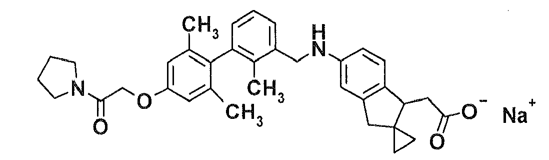 |
| **8-12** | 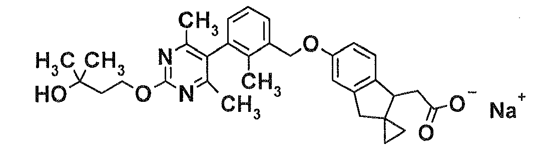 |
| **8-13** | 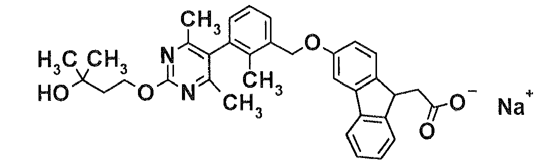 |
| **8-14** | 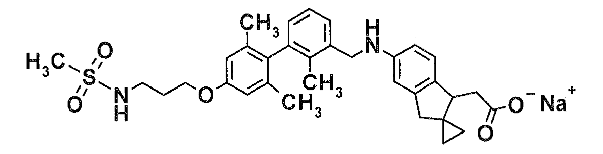 |
| **8-15** | 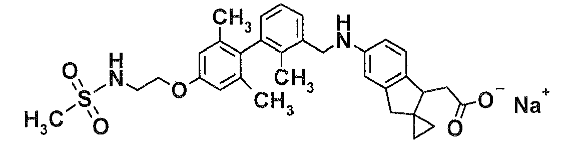 |
| **8-16** | 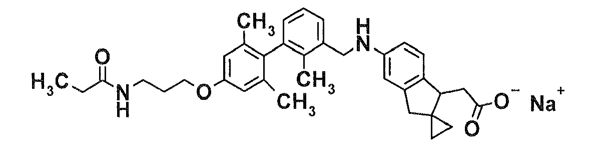 |
| **8-17** | 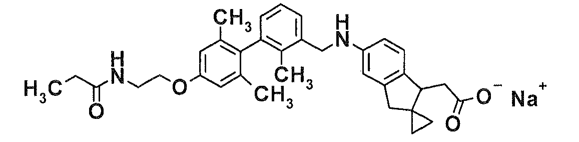 |
| **8-18** | 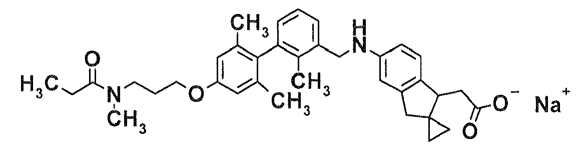 |

**[Table 30]**

| **Ex** | **Structure** |
|---|---|
| **8-19** | 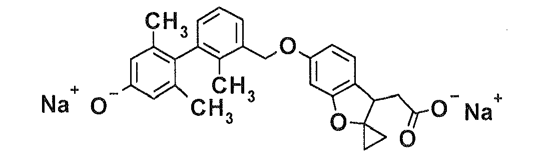 |
| **8-20** | 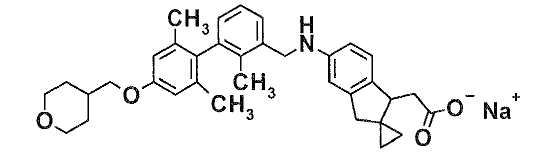 |
| **8-21** | 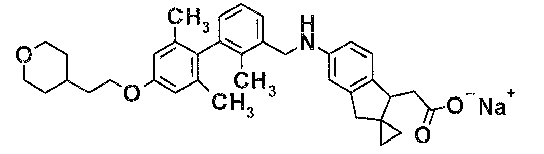 |
| **8-22** | 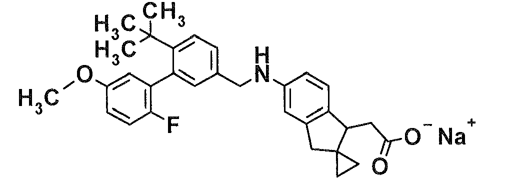 |
| **8-23** | 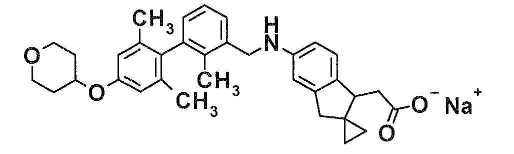 |
| **9,33** | 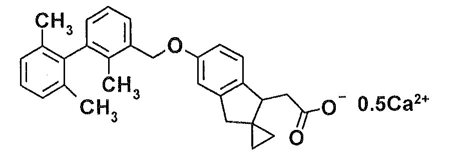 |
| **10** | 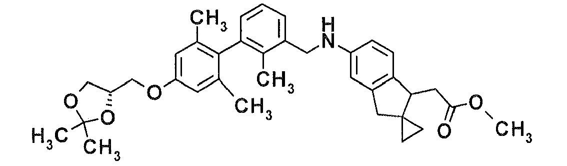 |

**[Table 31]**

| **Ex** | **Structure** |
|---|---|
| **10-1** | 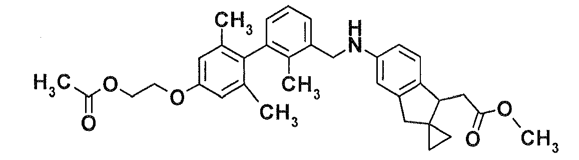 |
| **10-2** | 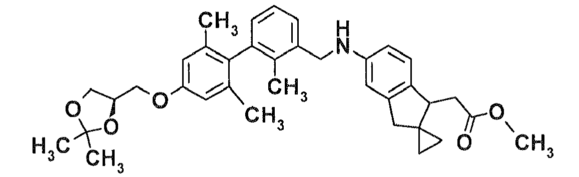 |
| **10-3** | 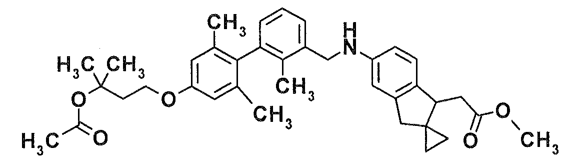 |
| **10-4** | 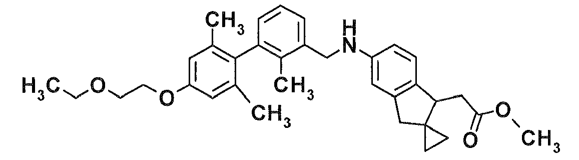 |
| **10-5** | 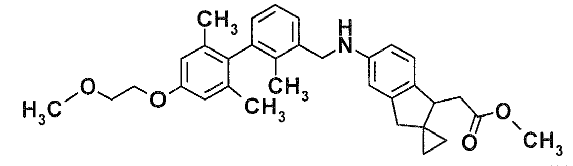 |
| **10-6** | 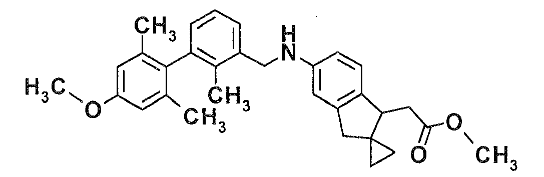 |
| **10-7** | 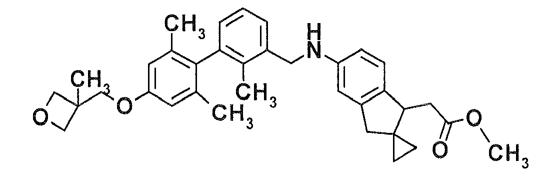 |

**[Table 32]**

| **Ex** | **Structure** |
|---|---|
| **10-8** | 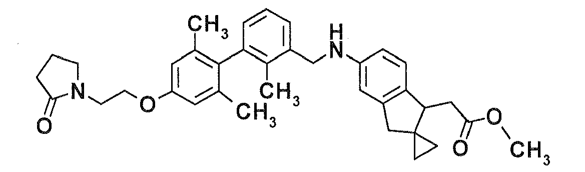 |
| **10-9** | 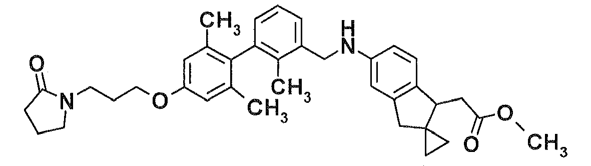 |
| **10-10** | 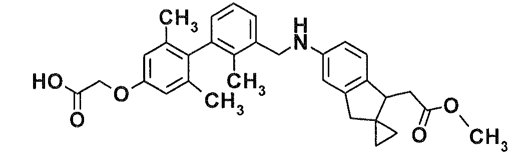 |
| **10-11** | 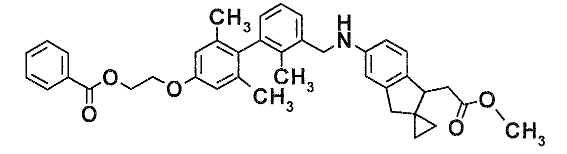 |
| **10-12** | 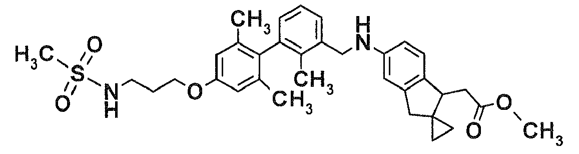 |
| **10-13** | 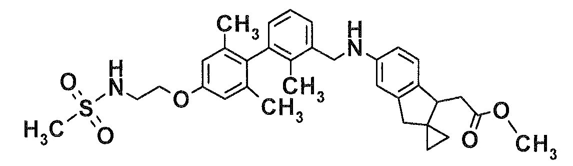 |
| **10-14** | 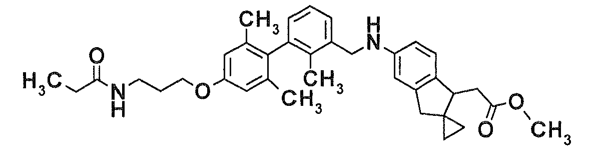 |

**[Table 33]**

| **Ex** | **Structure** |
|---|---|
| **10-15** | 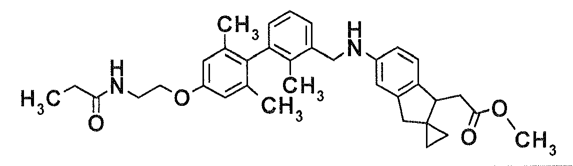 |
| **10-16** | 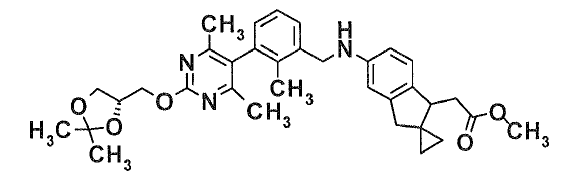 |
| **10-17** | 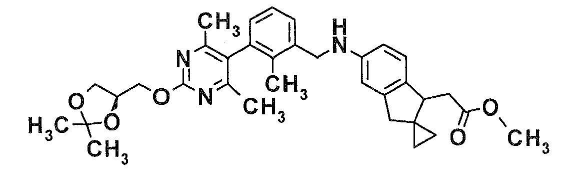 |
| **10-18** | 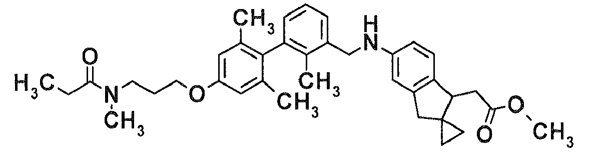 |
| **10-19** | 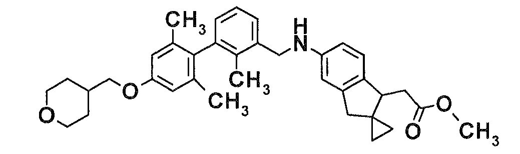 |
| **10-20** | 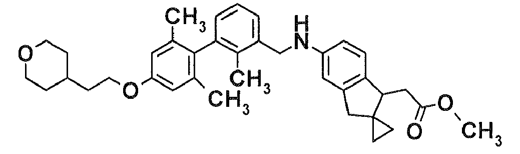 |
| **10-21** | 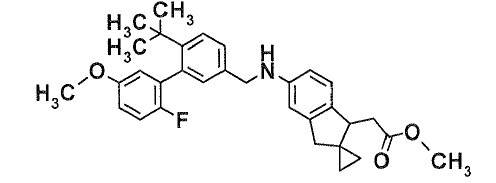 |

**[Table 34]**

| **Ex** | **Structure** |
|---|---|
| **10-22** | 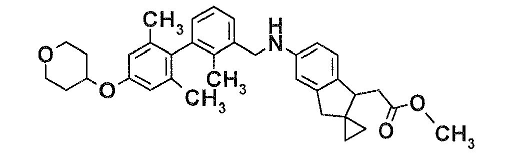 |
| **11** | 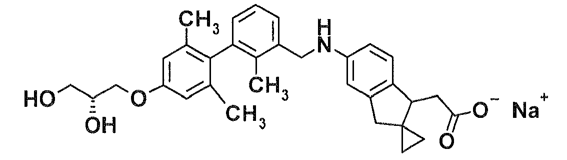 |
| **11-1** | 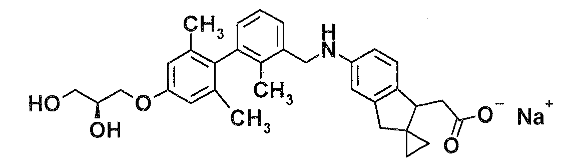 |
| **12** | 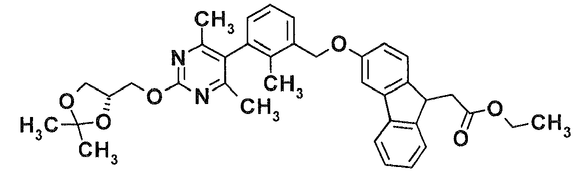 |
| **12-1** | 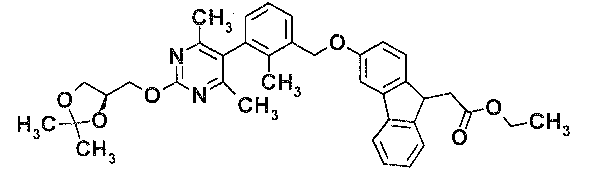 |
| **12-2** | 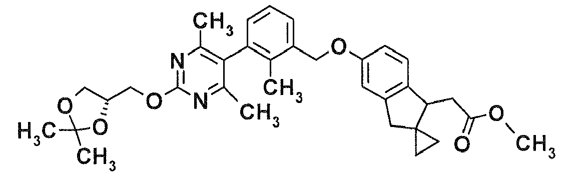 |
| **12-3** | 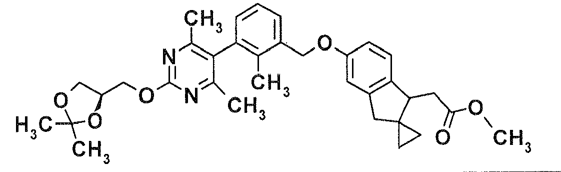 |

**[Table 35]**

| **Ex** | **Structure** |
|---|---|
| **12-4** | 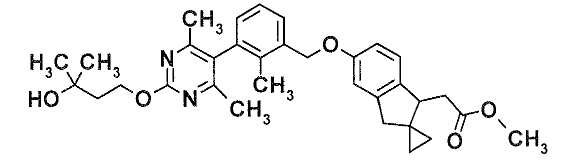 |
| **12-5** | 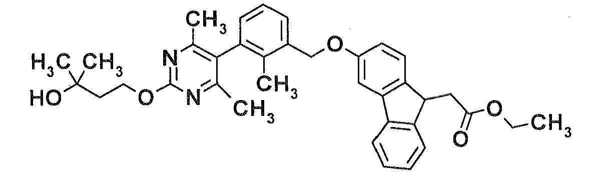 |
| **12-6** | 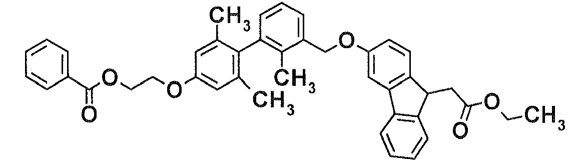 |
| **13** | 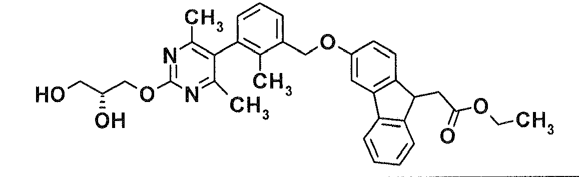 |
| **13-1** | 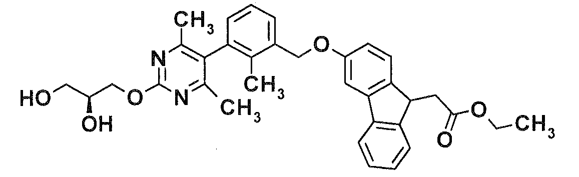 |
| **13-2** | 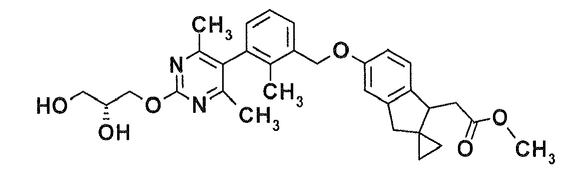 |
| **13-3** | 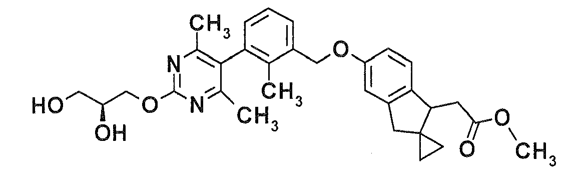 |

**[Table 36]**

| **Ex** | **Structure** |
|---|---|
| **14** | 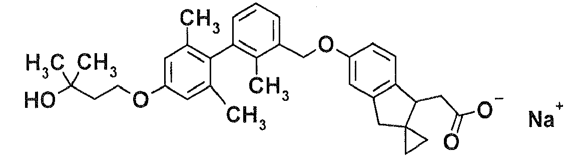 |
| **15** | 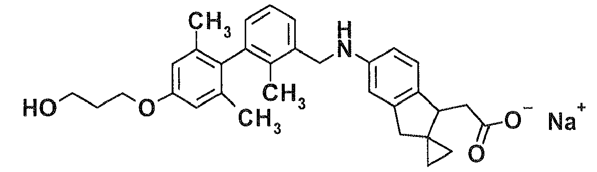 |
| **16** | 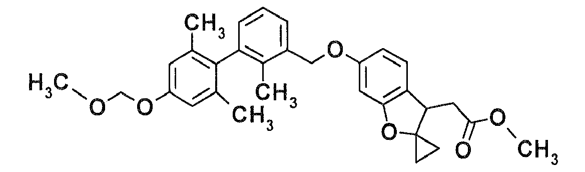 |
| **17** | 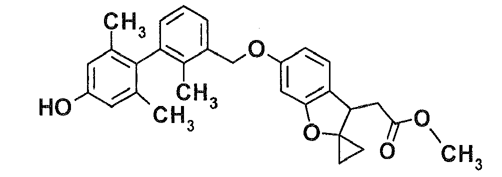 |
| **18** | 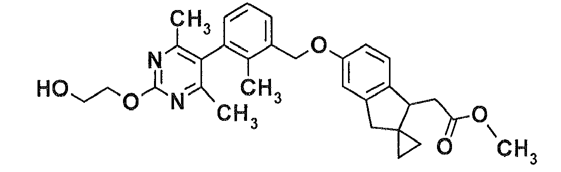 |
| **18-1** | 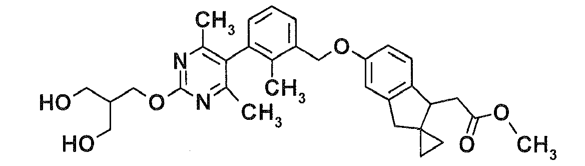 |
| **19** | 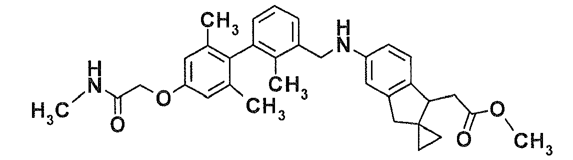 |

**[Table 37]**

| **Ex** | **Structure** |
|---|---|
| **19-1** | 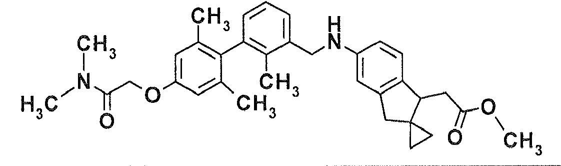 |
| **19-2** | 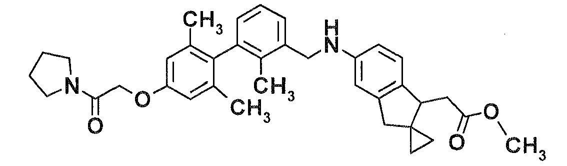 |
| **20** | 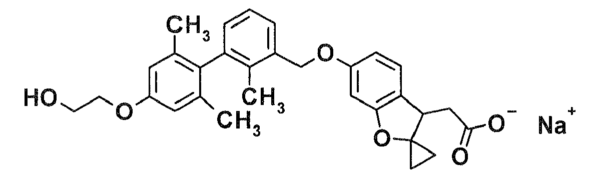 |
| **21** | 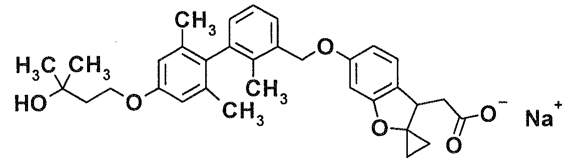 |
| **21-1** | 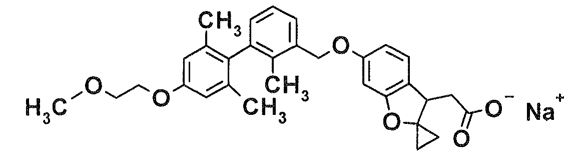 |
| **21-2** | 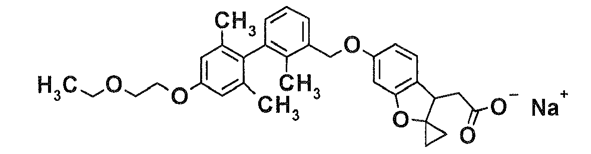 |
| **21-3** | 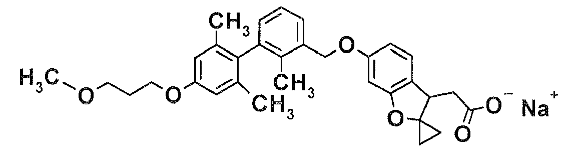 |

**[Table 38]**

| **Ex** | **Structure** |
|---|---|
| **21-4** | 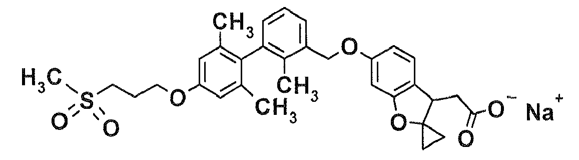 |
| **21-5** | 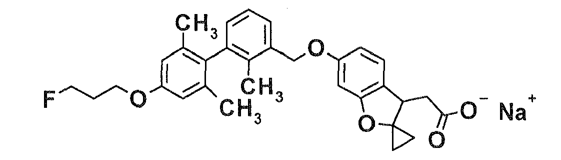 |
| **21-6** | 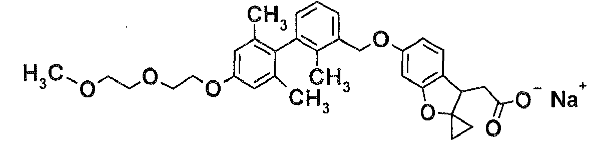 |
| **22** | 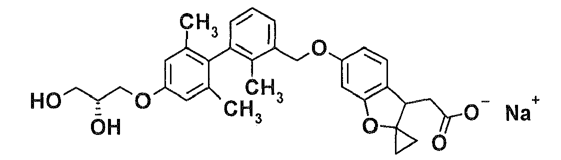 |
| **22-1** | 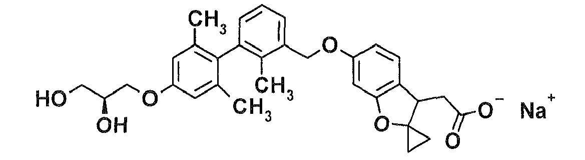 |
| **22-2** | 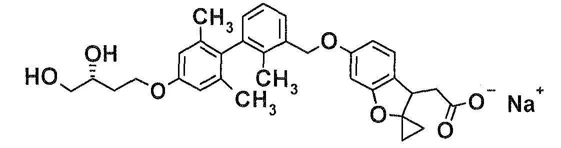 |
| **22-3** | 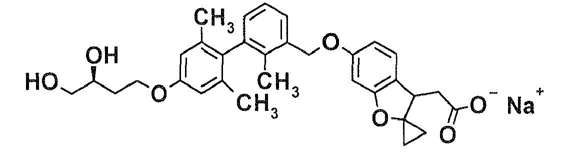 |
| **23** | 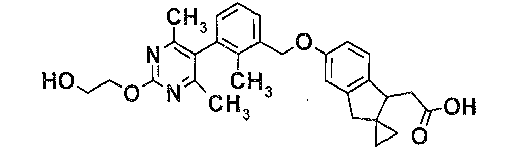 |

**[Table 39]**

| **Ex** | **Structure** |
|---|---|
| **23-1** | 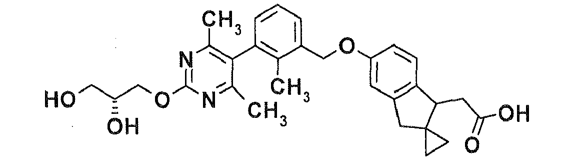 |
| **23-2** | 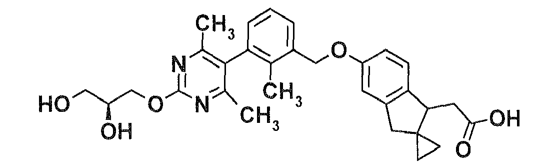 |
| **23-3** | 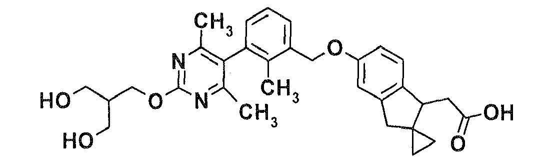 |
| **24** | 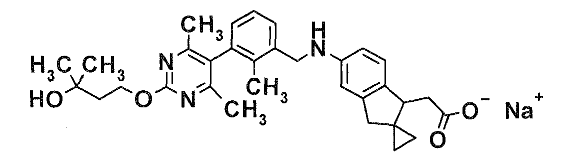 |
| **25a** | 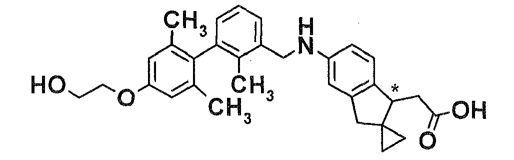 |
| **25b** | 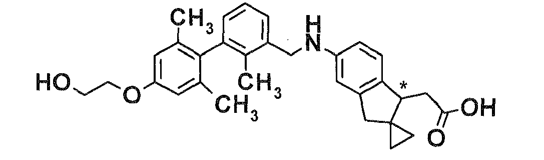 |
| **25-1a** | 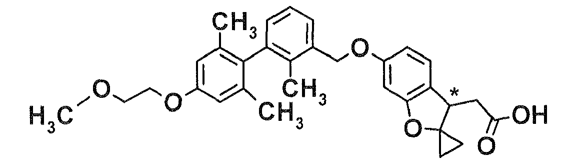 |

**[Table 40]**

| **Ex** | **Structure** |
|---|---|
| **25-1b** | 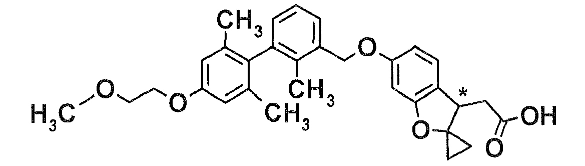 |
| **25-2a** | 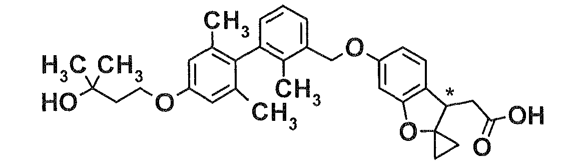 |
| **25-2b** | 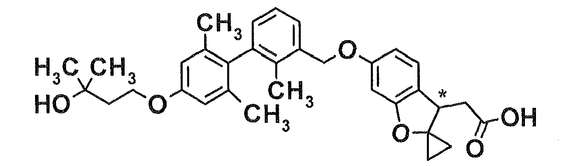 |
| **25-3a** | 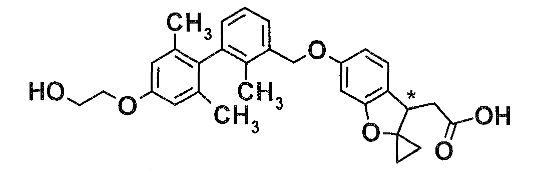 |
| **25-3b** | 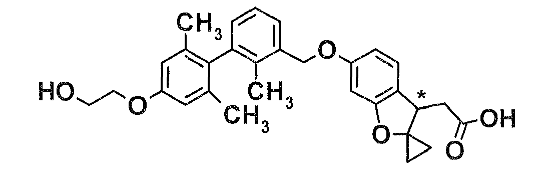 |
| **25-4a** | 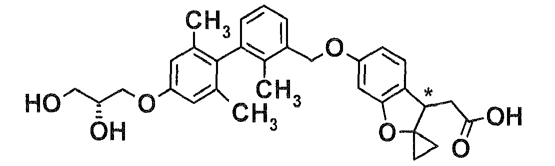 |
| **25-4b** | 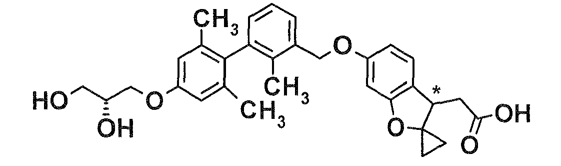 |

**[Table 41]**

| **Ex** | **Structure** |
|---|---|
| **25-5a** | 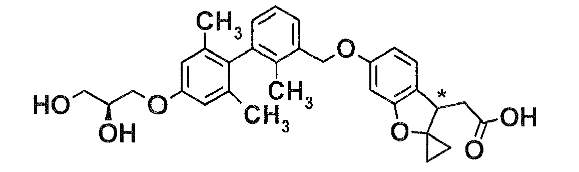 |
| **25-5b** | 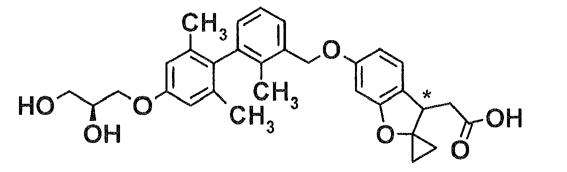 |
| **25-6a** | 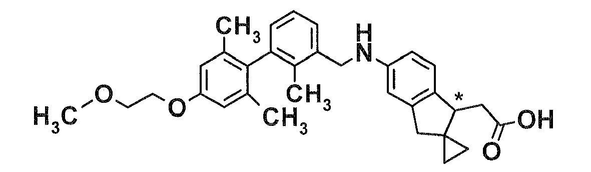 |
| **25-6b** | 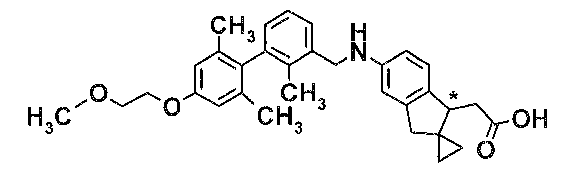 |
| **25-7a** | 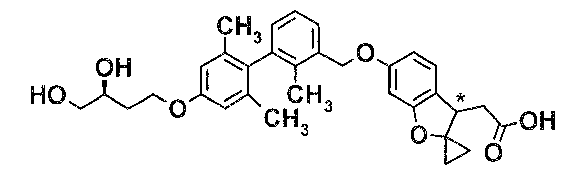 |
| **25-7b** | 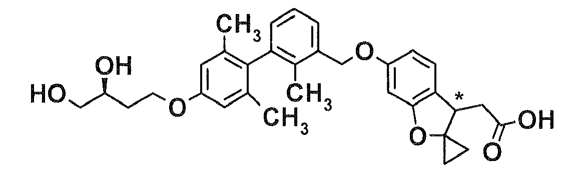 |
| **26** | 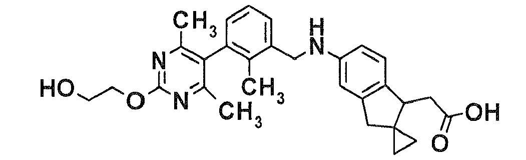 |

**[Table 42]**

| **Ex** | **Structure** |
|---|---|
| **26-1** | 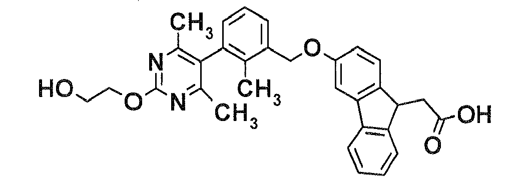 |
| **26-2** | 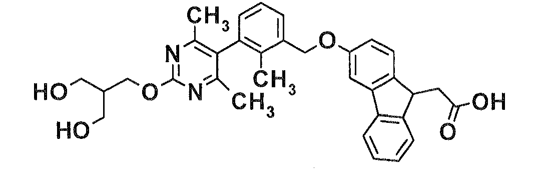 |
| **26-3** | 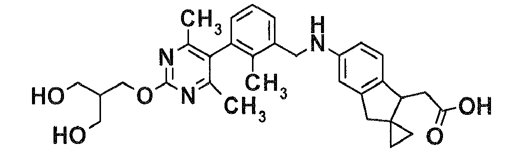 |
| **27** | 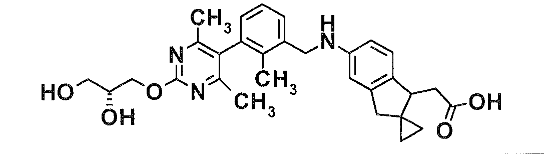 |
| **27-1** | 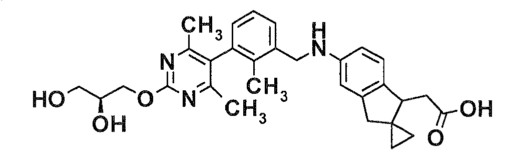 |
| **28** | 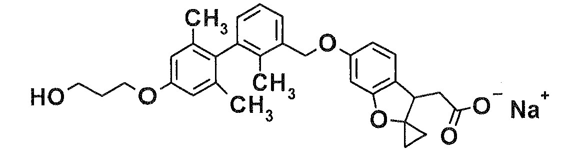 |
| **29** | 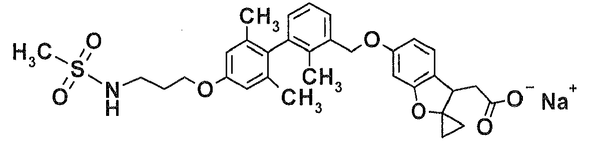 |

**[Table 43]**

| **Ex** | **Structure** |
|---|---|
| **30a** | 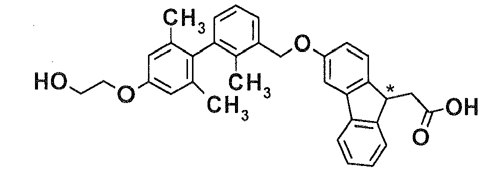 |
| **30b** | 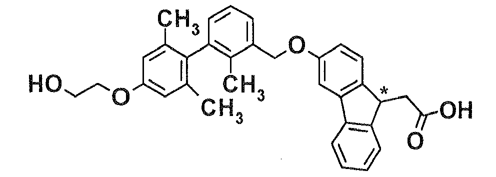 |
| **31** | 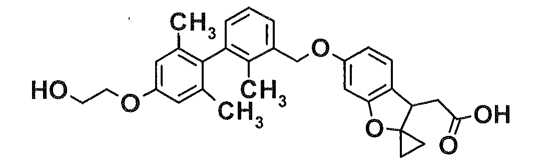 |
| **31-1** | 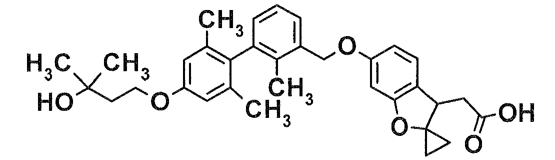 |
| **31-2** | 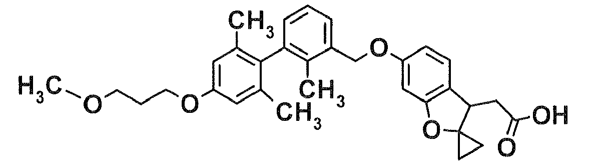 |
| **31-3** | 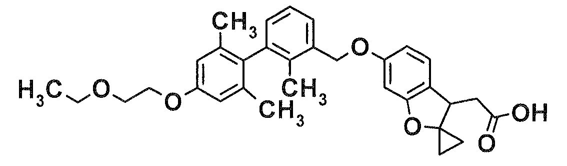 |
| **32** | 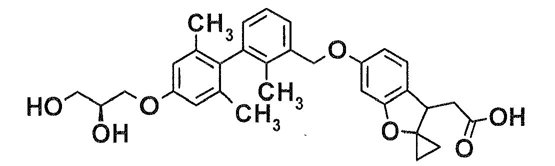 |

**[Table 44]**

| **Ex** | **Structure** |
|---|---|
| **32-1** | 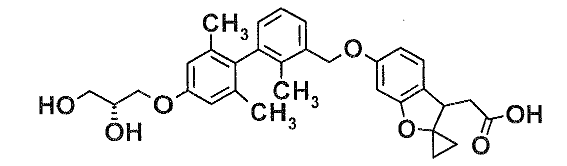 |

**[Table 45]**

| Ex | Data |
|---|---|
| 1 | NMR1 : 1.15-1.21 (3H, m), 1.86 (6H, s), 1.96 (3H, s), 2.06 (3H, s), 2.69-2.86 (2H, m), 3.66-4.28 (5H, m), 4.30-4.38 (2H, m), 5.22 (2H, s), 6.76 (2H, s), 6.95-7.04 (2H, m), 7.26-7.56 (7H, m), 7.75-7.90 (1H, m) |
| 1-1 | NMR1 : 1.18 (3H, t, J = 7.1 Hz), 1.89 (6H, s), 1.96 (3H, s), 2.72-2.86 (2F, m), 4.15 (2H, q, J = 7.1 Hz), 4.25 (1H, t, J = 7.5 Hz), 5.23 (2H, s), 6.97-7.04 (2H, m), 7.11-7.21 (3H, m), 7.31 (2H, t, J = 7.4 Hz), 7.39 (1H, t, J = 7.4 Hz), 7.46 (1H, d, J = 8.3 Hz), 7.50 (1H, d, J = 6.6 Hz), 7.54 (1H, d, J = 7.5 Hz), 7.61 (1H, d, J = 2.4 Hz), 7.87 (1H, d, J = 7.5 Hz) |
| 2 | NMR1 : 1.87 (6H, s), 1.97 (3H, s), 2.12-2.28 (2H, m), 3.68-3.76 (2H, m), 4.00 (2H, t, J = 5.1 Hz), 4.28 (1H, t, J = 7.3 Hz), 4.86-5.00 (1H, br), 5.20 (2H, s), 6.73 (2H, s), 6.91-6.99 (2H, m), 7.21-7.34 (3H, m), 7.48 (1H, d, J = 6.8 Hz), 7.52-7.59 (2H, m), 7.64 (1H, d, J = 7.5 Hz), 7.81 (1H, d, J = 7.4 Hz) ESI- : 507 |
| 2-1 | NMR1 : 0.22-0.32 (1H, m), 0.38-0.55 (2H, m), 0.69-0.79 (1H, m), 1.75-1.98 (11H, m), 2.43-2.55 (1H, m), 2.78 (1H, d, J = 15.8 Hz), 2.99-3.11 (1H, m), 3.67-3.77 (2H, m), 3.92-4.02 (2H, m), 4.21 (2H, d, J = 5.3 Hz), 4.78-5.08 (1H, m), 5.73 (1H, t, J = 5.6 Hz), 6.33-6.44 (2H, m), 6.71 (2H, s), 6.81-6.87 (1H, m), 6.93-7.01 (1H, m), 7.14-7.23 (1H, m), 7.25-7.33 (1H, m) ESI- : 484 |
| 2-2 | NMR1 : 0.23-0.32 (1H, m), 0.37-0.56 (2H, m), 0.68-0.80 (1H, m), 1.18 (6H, s), 1.78-1.97 (13H, m), 2.44-2.55 (1H, m), 2.78 (1H, d, J = 15.8 Hz), 3.02-3.11 (1H, m), 4.08 (2H, t, J = 7.3 Hz), 4.21 (2H, d, J = 5.4 Hz), 4.36-4.47 (1H, br), 5.73 (1H, t, J = 5.7 Hz), 6.31-6.43 (2H, m), 6.70 (2H, s), 6.81-6.88 (1H, m), 6.91-7.01 (1H, m), 7.13-7.23 (1H, m), 7.26-7.33 (1H, m) ESI- : 526 |
| 3 | NMR1 : 1.86 (6H, s), 1.97 (3H, s), 2.65 (1H, dd, J = 7.4, 16.4 Hz), 2.71 (1H, dd, J = 7.0, 16.2 Hz), 3.69-3.75 (2H, m), 3.99 (2H, t, J = 5.1 Hz), 4.23 (1H, t, J = 7.1 Hz), 4.84-4.90 (1H, m), 5.22 (2H, s), 6.73 (2H, s), 6.96-6.99 (1H, m), 7.01 (1H, dd, J = 2.4, 8.4 Hz), 7.26-7.34 (2H, m), 7.38(1H, t, J = 7.3 Hz), 7.46-7.52 (2H, m), 7.58 (1H, d, J = 7.4 Hz), 7.61 (1H, d, J = 2.3 Hz), 7.88 (1H, d, J = 7.4 Hz) ESI- : 507 |
| 3-1 | NMR2 : 0.53-0.77 (4H, m), 1.93 (6H, s), 1.98 (3H, s), 2.39-2.64 (3H, m), 3.06-3.20 (2H, m), 3.93-4.03 (2H, m), 4.06-4.16 (2H, m), 4.29 (2H, s), 6.47-6.58 (2H, m), 6.70 (2H, s), 6.93-7.01 (1H, m), 7.05-7.13 (1H, m), 7.15-7.28 (1H, m), 7.29-7.37 (1H,m) ESI- : 484 |
| 4 | NMR1 : 0.27-0.36 (1H, m), 0.41-0.57 (2H, m), 0.71-0.80 (1H, m), 1.80-2.00 (11H, m), 2.60 (1H, d, J = 16.1 Hz), 2.88 (1H, d, J =16.1 Hz), 3.09-3.17 (1H, m), 3.40-3.53 (2H, m), 3.74-3.90 (2H, m), 3.95-4.03 (1H, m), 4.76-4.94 (1H, m), 5.01-5.19 (3H, m), 6.69-6.78 (3H, m), 6.81-6.86 (1H, m), 6.92-6.97 (1H, m), 7.16-7.22 (1H, m), 7.22-7.30 (1H, m), 7.38-7.44 (1H, m) ESI- : 515 |
| 4-1 | NMR1 : 0.27-0.37 (1H, m), 0.41-0.57 (2H, m), 0.70-0.80 (1H, m), 1.80-2.02 (12H, m), 2.61 (1H, d, J = 16.0 Hz), 2.88 (1H, d, J = 16.0 Hz), 3.09-3.19 (1H, m), 3.47-3.59 (4H, m), 3.93-4.02 (2H, m), 4.53-4.74 (2H, m), 5.06 (2H, s), 6.68-6.79 (3H, m), 6.80-6.87 (1H, m), 6.90-6.99 (1H, m), 7.16-7.30 (2H, m), 7.37-7.46 (1H, m) ESI- : 529 |
| 5 | NMR1 : 0.27-0.37 (1H, m), 0.41-0.57 (2H, m), 0.70-0.80 (1H, m), 1.78-2.02 (11H, m), 2.61 (1H, d, J = 16.0 Hz), 2.88 (1H, d, J =16.0 Hz), 3.08-3.19 (1H, m), 3.66-3.77 (2H, m), 3.93-4.04 (2H, m), 4.75-5.15 (3H, m), 6.68-6.79 (3H, m), 6.81-6.87 (1H,m), 6.92-6.99 (1H, m), 7.14-7.30 (2H, m), 7.37-7.46 (1H, m) ESI- : 485 |

**[Table 46]**

| Ex | Data |
|---|---|
| 6 | NMR1 : 1.88 (6H, s), 1.93 (3H, s), 2.36-2.46 (2H, m), 4.31-4.44 (1H, m), 5.16 (2H, s), 6.84-6.93 (1H, m), 6.97 (1H, d, J = 7.4 Hz), 7.10-7.36 (6H, m), 7.46(1 H, d, J = 7.4 Hz), 7.54-7.58 (1H, m), 7.62(1H, d, J = 8.3 Hz), 7.67 (1H, d, J = 7.4 Hz), 7.83 (1H, d, J = 7.6 Hz) ESI- : 447 |
| 7 | ESI- : 565 |
| 8 | NMR1 : 1.86 (6H, s), 1.97 (3H, s), 2.12-2.78 (2H, m), 3.30-3.53 (2H, m), 3.75-4.31 (4H, m), 4.75-4.91 (1H, m), 5.03-5.18 (1H, m), 5.16-5.22 (2H, m), 6.73 (2H, s), 6.87-7.00 (2H, m), 7.19-7.35 (3H, m), 7.37-7.84 (5H, m) ESI- : 537 |
| 8-1 | NMR1 : 2.03 (3H, s), 2.05 (6H, s), 2.14 (1H, dd, J = 7.6, 14.8 Hz), 2.22 (1H, dd, J = 7.1, 14.5 Hz), 3.43-3.50 (2H, m), 3.79-3.86 (1H, m), 4.21 (1H, dd, J = 6.5, 10.9 Hz), 4.27 (1H, t, J = 7.2 Hz), 4.32 (1H, dd, J = 4.1, 11.1 Hz), 4.74-4.88 (1H, br), 5.04-5.15 (1H, br), 5.23 (2H, s), 6.91-6.97 (1H, m), 7.11 (1H, d, J = 7.6 Hz), 7.24 (1H, t, J = 7.2 Hz), 7.27-7.37 (2H, m), 7.51-7.60 (2H, m), 7.63 (1H, d, J = 7.4 Hz), 7.81 (1H, d, J = 7.5 Hz) ESI+ : 541 |
| 8-2 | NMR1 : 0.21-0.33 (1H, m), 0.37-0.58 (2H, m), 0.68-0.81 (1H, m), 1.14 (3H, t, J = 7.0 Hz), 1.80-1.98 (11H, m), 2.44-2.57 (1H, m), 2.78 (1H, d ,J = 15.8 Hz), 3.01-3.11 (1H, m), 3.52 (2H, q, J = 7.0 Hz), 3.66-3.75 (2H, m), 4.03-4.13 (2H, m), 4.15-4.27 (2H, m), 5.74 (1H, t, J = 5.7 Hz), 6.32-6.43 (2H, m), 6.72 (2H, s), 6.82-6.88 (1H, m), 6.93-7.01 (1H, m), 7.15-7.23 (1H, m), 7.27-7.34 (1H, m) ESI- : 512 |
| 8-3 | NMR1 : 0.21-0.32 (1H, m), 0.35-0.57 (2H, m), 0.68-0.80 (1H, m), 1.79-1.96 (11H, m), 2.43-2.57 (1H, m), 2.77 (1H, d, J = 15.8 Hz), 2.98-3.10 (1H, m), 3.32 (3H, s), 3.62-3.70 (2H, m), 4.04-4.14 (2H, m), 4.16-4.25 (2H, m), 5.74 (1H, t, J = 5.6 Hz), 6.33-6.44 (2H, m), 6.72 (2H, s), 6.81-6.87 (1H, m), 6.92-7.00 (1H, m), 7.15-7.23 (1H, m), 7.25-7.34 (1H, m) ESI- : 498 |
| 8-4 | NMR1 : 0.19-0.32 (1H, m), 0.38-0.54 (2H, m), 0.70-0.80 (1H, m), 1.80-1.96 (11H, m), 2.44-2.55 (1H, m), 2.77 (1H, d, J = 15.9 Hz), 3.01-3.09 (1H, m), 3.76 (3H, s), 4.16-4.26 (2H, m), 5.74 (1H, t, J = 5.7 Hz), 6.31-6.44 (2H, m), 6.71 (2H, s), 6.81-6.88 (1H, m), 6.93-6.99 (1H, m), 7.15-7.23 (1H, m), 7.26-7.33 (1H, m) ESI- : 454 |
| 8-5 | NMR1 : 0.22-0.32 (1H, m), 0.37-0.55 (2H, m), 0.69-0.79 (1H, m), 1.38 (3H, s), 1.79-1.94 (11H, m), 2.44-2.54 (1H, m), 2.77 (1H, d, J = 15.8 Hz), 3.01-3.09 (1H, m), 4.05 (2H, s), 4.16-4.25 (2H, m), 4.32 (2H, d, J = 5.7 Hz), 4.51 (2H, d, J = 5.7 Hz), 5.73 (1H, t, J = 5.6 Hz), 6.34-6.43 (2H, m), 6.77 (2H, s), 6.81-6.87 (1H, m), 6.92-7.00 (1H, m), 7.14-7.22 (1H, m), 7.27-7.33 (1H, m) ESI- : 524 |
| 8-6 | NMR1 : 0.21-0.31 (1H, m), 0.37-0.55 (2H, m), 0.69-0.80 (1H, m), 1.79-1.99 (13H, m), 2.23 (2H, t, J = 8.1 Hz), 2.43-2.55 (1H, m), 2.77 (1H, d, J = 15.8 Hz), 3.02-3.09 (1H, m), 3.48 (2H, t, J = 7.0 Hz), 3.55 (2H, t, J = 5.4 Hz), 4.08 (2H, t, J = 5.4 Hz), 4.17-4.25 (2H, m), 5.73 (1H, t, J = 5.7 Hz), 6.32-6.42 (2H, m), 6.72 (2H, s), 6.80-6.88 (1H, m), 6.92-7.00 (1H, m), 7.14-7.23 (1H, m), 7.26-7.32 (1H, m) ESI- : 551 |
| 8-7 | NMR1 : 0.22-0.32 (1H, m), 0.36-0.56 (2H, m), 0.68-0.80 (1H, m), 1.78-2.00 (15H, m), 2.22 (2H, t, J = 8.0 Hz), 2.45-2.57 (1H, m), 2.77 (1H, d, J = 15.8 Hz), 3.01-3.10 (1H, m), 3.30-3.43 (4H, m), 3.96 (2H, t, J = 6.2 Hz), 4.16-4.26 (2H, m), 5.73 (1H, t, J = 5.7 Hz), 6.32-6.44 (2H, m), 6.70 (2H, s), 6.81-6.88 (1H, m), 6.91-6.99 (1H, m), 7.14-7.22 (1H, m), 7.26-7.32 (1H, m) ESI- : 565 |

**[Table 47]**

| Ex | Data |
|---|---|
| 8-8 | NMR1 : 2.03 (3H, s), 2.05 (6H, s), 2.13(1H, dd, J = 7.7,14.7 Hz), 2.21 (1H, dd, J = 7.1, 14.7 Hz), 3.44-3.49 (2H, m), 3.78-3.87 (1H, m), 4.21 (1H, dd, J = 6.3, 10.9 Hz), 4.27 (1H, t, J = 7.4 Hz), 4.33 (1H, dd, J = 4.4, 10.9 Hz), 4.73-4.81 (1H, m), 5.03-5.10 (1H, m), 5.23 (2H, s), 6.95 (1H, dd, J = 2.4, 8.3 Hz), 7.09-7.13 (1H, m), 7.24 (1H, dt, J = 1.1, 7.4 Hz), 7.31 (1H, t, J = 7.3 Hz), 7.34 (1H, t, J = 7.6 Hz), 7.53-7.59 (2H, m), 7.63 (1H, d, J = 7.5 Hz), 7.81 (1H, d, J = 7.5 Hz) ESI+ : 541 |
| 8-9 | NMR1 : 0.22-0.32 (1H, m), 0.36-0.55 (2H, m), 0.69-0.80 (1H, m), 1.78-1.97 (11H, m), 2.43-2.56 (1H, m), 2.67 (3H, d, J = 4.5 Hz), 2.77 (1H, d, J = 15.8 Hz), 3.00-3.10 (1H, m), 4.21 (2H, d, J = 5.3 Hz), 4.46 (2H, s), 5.73 (1H, t, J = 5.8 Hz), 6.32-6.42 (2H, m), 6.75 (2H, s), 6.81-6.87 (1H, m), 6.93-6.99 (1H, m), 7.15-7.23 (1H, m), 7.26-7.33 (1H, m), 8.01-8.10 (1H, m) ESI- : 511 |
| 8-10 | NMR1 : 0.21-0.30 (1H, m), 0.37-0.55 (2H, m), 0.70-0.80 (1H, m), 1.80-1.96 (11H, m), 2.45-2.57 (1H, m), 2.77 (1H, d, J = 15.8 Hz), 2.87 (3H, s), 3.02 (3H, s), 3.02-3.10 (1H, m), 4.21 (2H, d, J = 5.2 Hz), 4.78 (2H, s), 5.74 (1H, t, J = 5.6 Hz), 6.31-6.42 (2H, m), 6.70 (2H, s), 6.82-6.87 (1H, m), 6.92-6.99 (1H, m), 7.14-7.23 (1H, m), 7.26-7.34 (1H, m) ESI- : 525 |
| 8-11 | NMR1 : 0.23-0.33 (1H, m), 0.38-0.55 (2H, m), 0.70-0.79 (1H, m), 1.72-1.96 (15H, m), 2.42-2.57 (1H, m), 2.77 (1H, d, J = 15.8 Hz), 3.02-3.10 (1H, m), 3.24-3.44 (2H, m), 3.49 (2H, t, J = 6.8 Hz), 4.21 (2H, d, J = 5.2 Hz), 4.70 (2H, s), 5.74 (1H, t, J = 5.7 Hz), 6.32-6.44 (2H, m), 6.71 (2H, s), 6.81-6.87 (1H, m), 6.93-7.00 (1H, m), 7.15-7.24 (1H, m), 7.26-7.33 (1H, m) ESI- : 551 |
| 8-12 | NMR1 : 0.28-0.39 (1H, m), 0.42-0.58 (2H, m), 0.70-0.80 (1H, m), 1.18 (6H, s), 1.82-2.02 (5H, m), 2.03 (6H, s), 2.60 (1H, d, J = 16.2 Hz), 2.89 (1H, d, J = 16.1 Hz), 3.13 (1H, t, J = 7.2 Hz), 3.20-3.80 (2H, m), 4.30-4.55 (3H, m), 5.09 (2H, s), 6.76 (1H, dd, J = 2.4, 8.3 Hz), 6.82-6.89 (1H, m), 7.06-7.12 (1H, m), 7.19 (1H, d, J = 8.4 Hz), 7.32 (1H, t, J = 7.6 Hz), 7.46-7.52 (1H, m) ESI- : 529 |
| 8-13 | NMR1 : 1.18 (6H, s), 1.87 (2H, t, J = 7.3 Hz), 2.03 (3H, s), 2.05 (6H, s), 2.14-2.32 (2H, m), 4.27 (1H, t, J = 7.3 Hz), 4.35-4.56 (3H, m), 5.23 (2H, s), 6.95 (1H, dd, J = 2.4, 8.3 Hz), 7.09-7.14 (1H, m), 7.21-7.28 (1H, m), 7.28-7.37 (2H, m), 7.52-7.60 (3H, m), 7.63 (1H, d, J = 7.4 Hz), 7.82 (1H, d, J = 7.4 Hz) ESI- : 551 |
| 8-14 | NMR1 : 0.21-0.32 (1H, m), 0.36-0.55 (2H, m), 0.70-0.79 (1H, m), 1.81-1.96 (13H, m), 2.44-2.53 (1H, m), 2.77 (1H, d, J = 15.9 Hz), 2.91 (3H, s), 3.02-3.08 (1H, m), 3.12 (2H, t, J = 6.9 Hz), 3.97-4.09 (2H, m), 4.21 (2H, d, J = 5.3 Hz), 5.73 (1H, t, J = 5.7 Hz), 6.30-6.44 (2H, m), 6.71 (2H, s), 6.81-6.88 (1H, m), 6.92-7.01 (1H, m), 7.09-7.24 (2H, m), 7.25-7.35 (1H, m) ESI- : 575 |
| 8-15 | NMR1 : 0.20-0.32 (1H, m), 0.38-0.55 (2H, m), 0.70-0.79 (1H, m), 1.80-1.97 (11H, m), 2.44-2.55 (1H, m), 2.77 (1H, d, J = 15.9 Hz), 2.97 (3H, s), 3.02-3.11 (1H,m), 3.25-3.45 (2H, m), 3.98-4.08 (2H, m), 4.21 (2H, d, J = 5.2 Hz), 5.74 (1H, t, J = 5.6 Hz), 6.32-6.44 (2H, m), 6.73 (2H, s), 6.81-6.88 (1H, m), 6.93-7.01 (1H, m), 7.14-7.23 (1H, m), 7.26-7.52 (2H, m) ESI- : 561 |

**[Table 481**

| Ex | Data |
|---|---|
| 8-16 | NMR1 : 0.23-0.32 (1H, m), 0.37-0.56 (2H, m), 0.69-0.80 (1H, m), 0.99 (3H, t, J = 7.6 Hz), 1.79-1.97 (13H, m), 2.08 (2H, q, J = 7.6Hz), 2.46-2.55 (1H, m), 2.77 (1H, d, J = 15.9 Hz), 3.01-3.11 (1H, m), 3.14-3.25 (2H, m), 3.98 (2H, t, J = 6.2 Hz), 4.21 (2H, d, J = 5.3 Hz), 5.74 (1H, t, J = 5.7 Hz), 6.32-6.44 (2H, m), 6.70 (2H, s), 6.81-6.87 (1H, m), 6.92-7.00 (1H, m), 7.13-7.23 (1H, m), 7.26-7.34 (1H, m), 7.85-7.95 (1H, m) ESI- : 553 |
| 8-17 | NMR1 : 0.22-0.33 (1H, m), 0.38-0.56 (2H, m), 0.69-0.80 (1H, m), 1.00 (3H, t, J = 7.6 Hz), 1.81-1.96 (11H, m), 2.11 (2H, q, J = 7.6 Hz), 2.45-2.55 (1H, m), 2.78 (1H, d, J = 15.8 Hz), 3.02-3.10 (1H, m), 3.30-3.49 (2H, m), 3.99 (2H, t, 5.8 Hz), 4.21 (2H, d, J = 5.3 Hz), 5.74 (1H, t, J = 5.6 Hz), 6.30-6.43 (2H, m), 6.72 (2H, s), 6.79-6.87 (1H, m), 6.93-7.01 (1H, m), 7.14-7.23 (1H, m), 7.24-7.34 (1H, m), 8.07 (1H, t, J = 5.5 Hz) ESI- : 539 |
| 8-18 | NMR1 : 0.23-0.33 (1H, m), 0.37-0.55 (2H, m), 0.69-0.78 (1H, m), 0.90-1.01 (3H, m), 1.78-2.05 (13H, m), 2.26-2.38 (2H, m), 2.44-2.54 (1H, m), 2.77 (1H, d, J = 15.9 Hz), 2.81-3.00 (3H, m), 3.02-3.11 (1H, m), 3.25-3.51 (2H, m), 3.92-4.05 (2H, m), 4.21 (2H, d, J = 5.3 Hz), 5.74 (1H, t, J = 5.7 Hz), 6.34-6.42 (2H, m), 6.66-6.74 (2H, m), 6.81-6.87 (1H, m), 6.94-7.00 (1H, m), 7.14-7.23 (1H, m), 7.26-7.32 (1H, m) ESI- : 567 |
| 8-19 | NMR1 : 0.65-0.87 (2H, m), 0.90-1.01 (2H, m), 1.78 (6H, s), 1.90 (3H, s), 2.18-2.27 (2H, m), 3.52-3.70 (1H, m), 5.04 (2H, s), 6.43 (1H, d, J = 2.2 Hz), 6.49 (1H, dd, J = 2.2, 8.2 Hz), 6.53 (2H, s), 6.89-6.98 (1H, m), 7.11-7.18 (1H, m), 7.19-7.28 (1H, m), 7.33-7.42 (1H, m) ESI- : 443 |
| 8-20 | NMR1 : 0.20-0.32 (1H, m), 0.36-0.46 (1H, m), 0.46-0.56 (1H, m), 0.68-0.80 (1H, m), 1.28-1.41 (2H, m), 1.65-1.74 (2H, m), 1.85 (6H, s), 1.91 (3H, s), 2.00 (1H, m), 2.49 (1H, d, J = 15.5 Hz), 2.77 (1H, d, J = 15.5 Hz), 3.06 (1H, t, J = 7.0 Hz), 3.30-3.50 (4H, m), 3.83 (2H, d, J = 6.5 Hz), 3.85-3.94 (2H, m), 4.21 (2H, d, J = 5.5 Hz), 5.74 (1H, t, J = 5.5 Hz), 6.36 (1H, d, J = 8.0 Hz), 6.40 (1H, s), 6.71 (2H, s), 6.84 (1H, d, J = 7.5 Hz), 6.96 (1H, d, J = 8.0 Hz), 7.19 (1H, dd, J = 7.5, 7.5 Hz), 7.29 (1H, d, J = 7.5 Hz) ESI+ : 540 |
| 8-21 | NMR1 : 0.20-0.30 (1H, m), 0.36-0.45 (1H, m), 0.45-0.54 (1H, m), 0.68-0.79 (1H, m), 1.17-1.30 (2H, m), 1.60-1.80 (5H, m), 1.80-1.94 (3H, m), 1.85 (6H, s), 1.90 (3H, s), 2.49 (1H, d, J = 15.5 Hz), 2.76 (1H, d, J = 15.5 Hz), 3.06 (1H, t, J = 7.0 Hz), 3.24-3.50 (2H, m), 3.81-3.89 (2H, m), 4.01 (2H, t, J = 6.5 Hz), 4.20 (2H, d, J = 5.5 Hz), 5.73 (1H, t, J = 5.5 Hz), 6.36 (1H, d, J = 8.0 Hz), 6.71 (2H, s), 6.84 (1H, d, J = 7.5 Hz), 6.96 (1H, d, J = 8.0 Hz), 7.19 (1H, t, J = 7.5 Hz), 7.29 (1H, d, J = 7.5 Hz) ESI+ : 554 |
| 8-22 | NMR1 : 0.20-0.30 (1H, m), 0.35-0.44 (1H, m), 0.44-0.54 (1H, m), 0.67-0.78 (1H, m), 1.14 (9H, s), 1.80-1.94 (2H, m), 2.48 (1H, dd, J = 4.0, 16.0 Hz), 2.75 (1H, dd, J = 3.0, 16.0 Hz), 3.04 (1H, dd, J = 3.0, 4.0 Hz), 3.74 (3H, s), 4.17 (2H, t, J = 6.0 Hz), 5.86 (1H, t, J = 6.0 Hz), 6.32 (1H, dd, J = 1.8, 8.0 Hz), 6.37 (1H, s), 6.78 (1H, dd, J = 3.0, 6.0 Hz), 6.90-7.00 (3H, m), 7.15 (1H, t, J = 9.0 Hz), 7.32 (1H, dd, J =1.8, 8.0 Hz), 7.49 (1H, d, J = 8.0 Hz) ESI+ : 488 |
| 8-23 | NMR1 : 0.21-0.32 (1H, m), 0.36-0.46 (1H, m), 0.46-0.55 (1H, m), 0.68-0.79 (1H, m), 1.53-1.65 (2H, m), 1.80-2.03 (4H, m), 1.85 (6H, s), 1.91 (3H, s), 2.46 (1H, d, J = 16.0 Hz), 2.77 (1H, d, J = 16.0 Hz), 3.06 (1H, t, J = 7.0 Hz), 3.40-3.54 (2H, m), 3.82-3.90 (2H, m), 4.20 (2H, d, J = 5.5 Hz), 4.54 (1H, m), 5.74 (1H, t, J = 5.5 Hz), 6.36 (1H, d, J = 8.0 Hz), 6.40 (1H, s), 6.75 (2H, s), 6.85 (1H, d, J = 7.5 Hz), 6.96 (1H, d, J = 8.0 Hz), 7.18 (1H, t, J = 7.5 Hz), 7.29 (1H, d, J = 7.5 Hz) ESI+ : 526 |

**[Table 491**

| Ex | Data |
|---|---|
| 9 | NMR1 : 0.30-0.40 (1H, m), 0.43-0.59 (2H, m), 0.70-0.79 (1H, m), 1.87 (6H, s), 1.89 (3H, s), 1.99-2.18 (2H, m), 2.60 (1H, d, J = 16.0 Hz), 2.92 (1H, d, J =16.0 Hz), 3.12-3.27 (1H, m), 5.05 (2H, s), 6.70-6.76 (1H, m), 6.85 (1H, s), 6.96 (1H, d, J = 7.5 Hz), 7.09-7.19 (3H, m), 7.21-7.31 (2H, m), 7.41 (1H, d, J = 7.3 Hz) ESI- : 425 |
| 10 | ESI+ : 570 |
| 10-1 | ESI+ : 542 |
| 10-2 | ESI+ : 570 |
| 10-3 | ESI+ : 584 |
| 10-4 | ESI+ : 528 |
| 10-5 | ESI+ : 514 |
| 10-6 | ESI+ : 470 |
| 10-7 | ESI+ : 540 |
| 10-8 | ESI+ : 567 |
| 10-9 | ESI+ : 581 |
| 10-10 | ESI- : 512 |
| 10-11 | ESI+ : 604 |
| 10-12 | ESI+ : 591 |
| 10-13 | ESI+ : 577 |
| 10-14 | ESI+ : 569 |
| 10-15 | ESI+ : 555 |
| 10-16 | ESI+ : 572 |
| 10-17 | ESI+ : 572 |
| 10-18 | NMR2 : 0.49-0.72 (4H, m), 1.14 (3H, q, J = 7.6 Hz), 1.92 (6H, s), 1.97 (3H, s), 2.01-2.11 (2H, m), 2.29-2.56 (5H, m), 2.94-3.07 (3H, m), 3.08-3.17 (2H, m), 3.49-3.64 (2H, m), 3.68 (3H, s), 4.00 (2H, t, J = 6.0 Hz), 4.29 (2H, s), 6.47-6.57 (2H, m), 6.63-6.69 (2H, m), 6.91-7.06 (2H, m), 7.16-7.36 (2H, m) ESI+ : 583 |
| 10-19 | ESI+ : 554 |
| 10-20 | ESI+ : 568 |
| 10-21 | ESI+ : 502 |
| 10-22 | ESI+ : 540 |
| 11 | NMR1 : 0.22-0.32 (1H, m), 0.38-0.55 (2H, m), 0.69-0.79 (1H, m), 1.78-1.98 (11H, m), 2.43-2.55 (1H, m), 2.78 (1H, d, J = 15.8 Hz), 3.00-3.10 (1H, m), 3.27-3.52 (2H, m), 3.74-3.90 (2H, m), 3.95-4.03 (1H, m), 4.21 (2H, d, J = 5.3 Hz), 4.81-4.95 (1H,m), 5.07-5.21 (1H, m), 5.73 (1H, t, J = 5.7 Hz), 6.33-6.43 (2H, m), 6.71 (2H, s), 6.82-6.87 (1H, m), 6.93-6.99 (1H, m), 7.15-7.22 (1H, m), 7.26-7.33 (1H, m) ESI- : 514 |
| 11-1 | NMR1 : 0.22-0.31 (1H, m), 0.37-0.55 (2H, m), 0.70-0.79 (1H, m), 1.80-1.98 (11H, m), 2.41-2.54 (1H, m), 2.78 (1H, d, J = 15.8 Hz), 3.01-3.10 (1H, m), 3.41-3.53 (2H, m), 3.74-3.90 (2H, m), 3.93-4.04 (1H, m), 4.21 (2H, d, J = 5.4 Hz), 4.79-4.99 (1H, m), 5.07-5.26 (1H, m), 5.73 (1H, t, J = 5.7 Hz), 6.33-6.44 (2H, m), 6.71 (2H, s), 6.81-6.88 (1H, m), 6.93-7.00 (1H, m), 7.14-7.22 (1H, m), 7.26-7.33 (1H, m) ESI- : 514 |
| 12 | ESI+ : 609 |
| 12-1 | ESI+ : 609 |
| 12-2 | ESI+ : 573 |
| 12-3 | ESI+ : 573 |
| 12-4 | ESI+ : 545 |

**[Table 501**

| Ex | Data |
|---|---|
| 12-5 | ESI+ : 581 |
| 12-6 | ESI+ : 641 |
| 13 | ESI+ : 569 |
| 13-1 | ESI+ : 569 |
| 13-2 | ESI+ : 533 |
| 13-3 | ESI+ : 533 |
| 14 | ESI- : 527 |
| 15 | NMR1 : 0.21-0.32 (1H, m), 0.38-0.55 (2H, m), 0.70-0.80 (1H, m), 1.79-1.97 (13H, m), 2.45-2.55 (1H, m), 2.77 (1H, d, J = 15.9 Hz), 3.02-3.11 (1H, m), 3.52-3.61 (2H, m), 3.97-4.08 (2H, m), 4.15-4.26 (2H, m), 4.56-4.68 (1H, m), 5.74 (1H, t, J = 5.6 Hz), 6.32-6.43 (2H, m), 6.70 (2H, s), 6.81-6.88 (1H, m), 6.93-7.00 (1H, m), 7.13-7.22 (1H, m), 7.25-7.34 (1H, m) ESI- : 498 |
| 16 | NMR1 : 0.65-1.08 (4H, m), 1.84 (6H, s), 1.90 (3H, s), 2.48-2.74 (2H, m), 3.40 (3H, s), 3.54-3.67 (4H, m), 5.07 (2H, s), 5.19 (2H, s), 6.52 (1H, d, J = 2.3 Hz), 6.56 (1H, dd, J = 2.3, 8.2 Hz), 6.81 (2H, s), 6.92-7.00 (1H, m), 7.10 (1H, d, J = 8.3 Hz), 7.27 (1H, t, J = 7.6 Hz), 7.37-7.43 (1H, m) |
| 17 | ESI+ : 459 |
| 18 | ESI+ : 503 |
| 18-1 | ESI+ : 547 |
| 19 | ESI+ : 527 |
| 19-1 | ESI+ : 541 |
| 19-2 | ESI+ : 567 |
| 20 | NMR1 : 0.62-0.85 (2H, m), 0.85-1.13 (2H, m), 1.84 (6H, s), 1.90 (3H, s), 2.00-2.17 (2H, m), 3.63 (1H, t, J = 7.2 Hz), 3.67-3.77 (2H, m), 3.99 (2H, t, J = 5.1 Hz), 4.77-5.00 (1H, br), 5.05 (2H, s), 6.41 (1H, d, J = 2.2 Hz), 6.48 (1H, dd, J = 2.3, 8.2 Hz), 6.72 (2H, s), 6.92-6.98 (1H, m), 7.15 (1H, d, J = 8.2 Hz), 7.26 (1H, t, J = 7.6 Hz), 7.36-7.43 (1H, m) ESI- : 487 |
| 21 | NMR1 : 0.62-0.85 (2H, m), 0.85-1.06 (2H, m), 1.18 (6H, s), 1.84 (6H, s), 1.90 (3H, s), 2.04-2.20 (2H, m), 3.64 (1H, t, J = 7.1 Hz), 4.08 (2H, t, J = 7.1 Hz), 4.33-4.53 (1H, br), 5.05 (2H, s), 6.42 (1H, d, J = 2.2 Hz), 6.49 (1H, dd, J = 2.3, 8.2 Hz), 6.71 (2H, s), 6.93-6.98 (1H, m), 7.13-7.18 (1H, m), 7.26 (1H, t, J = 7.5 Hz), 7.36-7.42 (1H, m) ESI- : 529 |
| 21-1 | NMR1 : 0.62-0.89 (2H, m), 0.89-1.01 (2H, m), 1.84 (6H, s), 1.90 (3H, s), 2.20-2.31 (2H, m), 3.32 (3H, s), 3.42-3.76 (3H, m), 4.04-4.14 (2H, m), 5.05 (2H, s), 6.45 (1H, d, J = 2.2 Hz), 6.50 (1H, dd, J = 2.2, 8.2 Hz), 6.72 (2H, s), 6.91-6.98 (1H, m), 7.12-7.19 (1H, m), 7.22-7.30 (1H, m), 7.36-7.42 (1H, m) ESI- : 501 |
| 21-2 | NMR1 : 0.65-0.82 (2H, m), 0.90-1.02 (2H, m), 1.14 (3H, t, J = 7.0 Hz), 1.84 (6H, s), 1.90 (3H, s), 2.13-2.19 (2H, m), 3.51 (2H, q, J = 7.0 Hz), 3.56-3.66 (1H, m), 3.66-3.74 (2H, m), 4.05-4.13 (2H, m), 5.05 (2H, s), 6.43 (1H, d, J = 2.2 Hz), 6.49 (1H, dd, J = 2.2, 8.2 Hz), 6.73 (2H, s), 6.89-6.99 (1H, m), 7.11-7.19 (1H, m), 7.21-7.31 (1H, m), 7.36-7.44 (1H, m) ESI- : 515 |
| 21-3 | NMR1 : 0.66-0.82 (2H, m), 0.90-1.01 (2H, m), 1.84 (6H, s), 1.87-2.00 (5H, m), 2.12-2.22 (2H, m), 3.26 (3H, s), 3.48 (2H, t, J = 6.3 Hz), 3.58-3.69 (1H, m), 4.02 (2H, t, J = 6.3 Hz), 5.05 (2H, s), 6.43 (1H, d, J = 2.2 Hz), 6.49 (1H, dd, J = 2.2, 8.2 Hz), 6.71 (2H, s), 6.89-6.97 (1H, m), 7.11-7.19 (1H, m), 7.21-7.29 (1H, m), 7.36-7.43 (1H, m) ESI- : 515 |

**[Table 51]**

| Ex | Data |
|---|---|
| 21-4 | NMR1 : 0.67-0.83 (2H, m), 0.91-1.02 (2H, m), 1.85 (6H, s), 1.90 (3H, s), 2.10-2.21 (4H, m), 3.04 (3H, s), 3.22-3.33 (2H, m), 3.55-3.67 (1H, m), 4.09 (2H, t, J = 6.1 Hz), 5.05 (2H, s), 6.43 (1H, d, J = 2.2 Hz), 6.49 (1H, dd, J = 2.2, 8.2 Hz), 6.73 (2H, s), 6.92-6.98 (1H, m), 7.11-7.18 (1H, m), 7.22-7.30 (1H, m), 7.36-7.43 (1H, m) ESI- : 563 |
| 21-5 | NMR1 : 0.66-0.81 (2H, m), 0.89-1.02 (2H, m), 1.84 (6H, s), 1.90 (3H, s), 2.05-2.19 (4H, m), 3.55-3.67 (1H, m), 4.08 (2H, t, J = 6.2 Hz), 4.54-4.72 (2H, m), 5.05 (2H, s), 6.42 (1H, d, J = 2.2 Hz), 6.49 (1H, dd, J = 2.2, 8.2 Hz), 6.74 (2H, s), 6.90-6.99 (1H, m), 7.11-7.20 (1H, m), 7.22-7.30 (1H, m), 7.35-7.43 (1H, m) ESI- : 503 |
| 21-6 | NMR1 : 0.66-0.76 (1H, m), 0.79-0.89 (1H, m), 0.90-1.01 (2H, m), 1.84 (6H, s), 1.90 (3H, s), 2.24-2.36 (2H, m), 3.26 (3H, s), 3.29-3.82 (7H, m), 4.03-4.15 (2H, m), 5.06 (2H, s), 6.45 (1H, d, J = 2.2 Hz), 6.51 (1H, dd, J = 2.2, 8.2 Hz), 6.73 (2H, s), 6.90-6.99 (1H, m), 7.10-7.20 (1H, m), 7.22-7.32 (1H, m), 7.34-7.45 (1H, m) ESI- : 545 |
| 22 | NMR1 : 0.62-0.85 (2H, m), 0.85-1.08 (2H, m), 1.84 (6H, s), 1.90 (3H, s), 2.04-2.18 (2H, m), 3.39-3.55 (2H, m), 3.64 (1H, t, J = 7.1 Hz), 3.73-3.83 (1H, m), 3.86 (1H, dd, J = 6.0, 9.7 Hz), 3.99 (1H, dd, J = 4.5, 9.7 Hz), 4.78-4.95 (1H, br), 5.05 (2H, s), 5.10-5.25 (1H, br), 6.42 (1H, d, J = 2.2 Hz), 6.49 (1H, dd, J = 2.3, 8.2 Hz), 6.71 (2H, s), 6.92-6.98 (1H, m), 7.16 (1H, d, J = 8.2 Hz), 7.26 (1H, t, J = 7.6 Hz), 7.36-7.42 (1H, m) ESI- : 517 |
| 22-1 | NMR1 : 0.62-0.85 (2H, m), 0.85-1.08 (2H, m), 1.84 (6H, s), 1.90 (3H, s), 2.04-2.18 (2H, m), 3.39-3.55 (2H, m), 3.64 (1H, t, J = 7.1 Hz), 3.73-3.83 (1H, m), 3.86 (1H, dd, J = 6.0, 9.7 Hz), 3.99 (1H, dd, J = 4.5, 9.7 Hz), 4.72-4.93 (1H, br), 5.05 (2H, s), 5.07-5.22 (1H, br), 6.42 (1H, d, J = 2.2 Hz), 6.49 (1H, dd, J = 2.3, 8.2 Hz), 6.71 (2H, s), 6.92-6.98 (1H, m), 7.16 (1H, d, J = 8.2 Hz), 7.26 (1H, t, J = 7.6 Hz), 7.36-7.42 (1H, m) ESI- : 517 |
| 22-2 | NMR1 : 0.66-0.82 (2H, m), 0.90-1.06 (2H, m), 1.56-1.71 (1H, m), 1.84 (6H, s), 1.85-1.99 (4H, m), 2.09-2.20 (2H, m), 3.17-3.54 (2H, m), 3.57-3.72 (2H, m), 4.01-4.12 (2H, m), 4.58-4.83 (2H, m), 5.05 (2H, s), 6.42 (1H, d, J = 2.2 Hz), 6.49 (1H, dd, J = 2.2, 8.2 Hz), 6.71 (2H, s), 6.91-6.98 (1H, m), 7.11-7.19 (1H, m), 7.22-7.30 (1H, m), 7.36-7.42 (1H, m) ESI- : 531 |
| 22-3 | NMR1 : 0.67-0.84 (2H, m), 0.88-1.01 (2H, m), 1.58-1.70 (1H, m), 1.84 (6H, s), 1.85-1.99 (4H, m), 2.04-2.19 (2H, m), 3.17-3.54 (2H, m), 3.57-3.69 (2H, m), 4.02-4.11 (2H, m), 4.62-4.86 (2H, m), 5.05 (2H, s), 6.42 (1H, d, J = 2.2 Hz), 6.49 (1H, dd, J = 2.2, 8.2 Hz), 6.71 (2H, s), 6.92-6.99 (1H, m), 7.13-7.19 (1H, m), 7.22-7.30 (1H, m), 7.36-7.43 (1H, m) ESI- : 531 |
| 23 | NMR1 : 0.45-0.59 (3H, m), 0.69-0.75 (1H, m), 1.97 (3H, s), 2.03 (6H, s), 2.22-2.31 (1H, m), 2.37-2.57 (2 H, m), 3.00-3.09 (2H, m), 3.73 (2H, q, J = 5.2 Hz), 4.32 (2H, t, J = 5.1 Hz), 4.89 (1H, t, J = 5.4 Hz), 5.11 (2H, s), 6.83 (1H, dd, J = 2.4, 8.2 Hz), 6.91-6.93 (1H, m), 7.08-7.14 (2H, m), 7.32 (1H, t, J = 7.6 Hz), 7.48-7.52 (1H, m) ESI+ : 489 |
| 23-1 | NMR1 : 0.46-0.60 (3H, m), 0.69-0.75 (1H, m), 1.97 (3H, s), 2.04 (6H, s), 2.26 (1H, dd, J = 8.5, 15.8 Hz), 2.38-2.45 (1H, m), 2.48-2.57 (1H, m), 3.01-3.10 (2H, m), 3.43-3.49 (2H, m), 3.78-3.87 (1H, m), 4.20 (1H, dd, J = 6.4,10.9 Hz), 4.32 (1H, dd, J = 4.2, 10.8 Hz), 4.66-4.71 (1H, m), 4.96-5.01 (1H, m), 5.11 (2H, s), 6.83 (1H, dd, J=2.4, 8.3 Hz), 6.91-6.93 (1H, m), 7.08-7.14 (2H, m), 7.32 (1H, t, J = 7.6 Hz), 7.48-7.52 (1H, m), 12.00-12.50 (1H, m) ESI+ : 519 |

**[Table 52]**

| Ex | Data |
|---|---|
| 23-2 | NMR1 : 0.46-0.59 (3H, m), 0.68-0.75 (1H, m), 1.97 (3H, s), 2.04 (6H, s), 2.27 (1H, dd, J = 8.4, 15.7 Hz), 2.42 (1H, dd, J = 6.2, 15.7 Hz), 2.48-2.57 (1H, m), 3.00-3.10 (2H, m), 3.43-3.49 (2H, m), 3.79-3.85 (1H, m), 4.20 (1H, dd, J = 6.4,10.9 Hz), 4.32 (1H, dd, J = 4.3,10.9 Hz), 4.65-4.72 (1H, m), 4.95-5.01 (1H, m), 5.11 (2H, s), 6.84 (1H, dd, J = 2.4, 8.3 Hz), 6.92 (1H, d, J = 2.2 Hz), 7.08-7.14 (2H, m), 7.32 (1H, t, J = 7.6 Hz), 7.48-7.52 (1H, m), 12.01-12.54 (1H, m) ESI+ : 519 |
| 23-3 | NMR1 : 0.46-0.59 (3H, m), 0.69-0.75 (1H, m), 1.94-2.05 (10H, m), 2.27 (1H, dd, J = 8.5, 15.7 Hz), 2.42 (1H, dd, J = 6.2, 15.7 Hz), 2.47-2.57 (1H, m), 3.00-3.10 (2H, m), 3.48-3.59 (4H, m), 4.29 (2H, d, J = 6.0 Hz), 4.52-4.58 (1H, m), 5.11 (2H, s), 6.83 (1H, dd, J = 2.4, 8.3 Hz), 6.92 (1H, d, J = 2.2 Hz), 7.08-7.14 (2H, m), 7.32 (1H, t, J = 7.6 Hz), 7.48-7.52 (1H, m), 12.00-12.55 (1H, m) ESI+: 533 |
| 24 | NMR1 : 0.22-0.34 (1H, m), 0.36-0.56 (2H, m), 0.68-0.80 (1H, m), 1.18 (6H, s), 1.82-2.00 (5H, m), 2.04 (6H, s), 2.44-2.54 (1H, m), 2.79 (1H, d, J = 15.9 Hz), 3.06 (1H, t, J = 7.1 Hz), 3.20-3.60 (2H, m), 4.23 (2H, d, J = 5.1 Hz), 4.32-4.60 (3H, m), 5.79 (1H, t, J = 5.7 Hz), 6.32-6.39 (1H, m), 6.39-6.44 (1H, m), 6.94-7.02 (2H, m), 7.24 (1H, t, J = 7.6 Hz), 7.36 (1H, d, J = 7.3 Hz) ESI- : 528 |
| 25a | NMR2 : 0.48-0.96 (4H, m), 1.93 (6H, s), 1.98 (3H, s), 2.36-2.70 (3H, m), 3.00-3.22 (2H, m), 3.92-4.03 (2H, m), 4.05-4.18 (2H, m), 4.29 (2H, s), 6.46-6.64 (2H, m), 6.70 (2H, s), 6.97 (1H, d, J = 7.3 Hz), 7.09 (1H, d, J = 8.0 Hz), 7.16-7.40 (2H, m) ESI- : 484 [α]²³_{D} : -44.2°(c = 0.65, CHCl₃) |
| 25b | NMR2 : 0.48-0.96 (4H, m), 1.93 (6H, s), 1.98 (3H, s), 2.36-2.70 (3H, m), 3.00-3.22 (2H, m), 3.92-4.03 (2H, m), 4.05-4.18 (2H, m), 4.30 (2H, s), 6.46-6.64 (2H, m), 6.70 (2H, s), 6.97 (1H, d, J = 7.3 Hz), 7.09 (1H, d, J = 8.0 Hz), 7.16-7.40 (2H, m) ESI-:484 [α]²³_{D} : +38.7°(c = 0.50, CHCl₃) |
| 25-1a | NMR1 : 0.70-0.76 (1H, m), 0.92-1.05 (3H, m), 1.84 (6H, s), 1.90 (3H, s), 2.44-2.60 (2H, m), 3.32 (3H, s), 3.58 (1H, t, J = 7.0 Hz), 3.66 (2H, t, J = 4.2 Hz), 4.09 (2H, t, J = 4.8 Hz), 5.07 (2H, s), 6.50-6.57 (2H, m), 6.72 (2H, s), 6.96 (1H, d, J = 7.3 Hz), 7.15 (1H, d, J = 8.3 Hz), 7.26 (1H, t, J = 7.3 Hz), 7.40 (1H, d, J = 7.3 Hz) ESI- : 501.2 [α]²³_{D} : -46.4°(c = 1.00, CHCl₃) |
| 25-1b | NMR1: 0.70-0.75 (1H, m), 0.90-1.04 (3H, m), 1.84 (6H, s), 1.89 (3H, s), 2.44-2.60 (2H, m), 3.32 (3H, s), 3.58 (1H, t, J = 7.4 Hz), 3.66 (2H, t, J = 4.5 Hz), 4.09 (2H, t, J = 4.4 Hz), 5.07 (2H, s), 6.51 (1H, d, J = 2.2 Hz), 6.55-6.57 (1H, m), 6.72 (2H, s), 6.96 (1H, d, J = 6.8 Hz), 7.14 (1H, d, J = 8.4 Hz), 7.26 (1H, t, J = 7.4 Hz), 7.40 (1H, d,J=7.0Hz) ESI- : 501.2 [α]²³_{D} : +42.6°(c = 1.00, CHCl₃) |
| 25-2a | NMR1 : 0.70-0.75 (1H, m), 0.90-1.02 (3H, m), 1.18 (6H, s), 1.83-1.86 (8H, m), 1.90 (3H, s), 2.45-2.60 (2H, m), 3.58 (1H, t, J= 7.2 Hz), 4.07 (2H, t, J = 7.2 Hz), 5.07 (2H, s), 6.51 (1H, d, J = 2.2 Hz), 6.55-6.57 (1H, m), 6.71 (2H, s), 6.96 (1H, d, J = 6.5 Hz), 7.14 (1H, d, J = 8.4 Hz), 7.26 (1H, t, J = 7.7 Hz), 7.39 (1H, d, J = 7.4 Hz) ESI- : 529.2 [α]²³_{D} : -38.8°(c = 0.96, CHCl₃) |

**[Table 531**

| Ex | Data |
|---|---|
| 25-2b | NMR1 : 0.71-0.75 (1H, m), 0.90-1.03 (3H, m), 1.18 (6H, s), 1.83-1.86 (8H, m), 1.90 (3H, s), 2.44-2.60 (2H, m), 3.58 (1H, t, J = 6.7 Hz), 4.07 (2H, t, J = 6.7 Hz), 5.07 (2H, s), 6.50 (1H, d, J = 2.5 Hz), 6.55-6.57 (1H, m), 6.71 (2H, s), 6.96 (1H, d, J = 6.7 Hz), 7.14 (1H, d, J = 8.3 Hz), 7.26 (1H, t, J = 7.5 Hz), 7.39 (1H, d, J = 7.0 Hz) ESI- : 529.2 [α]²³ _{D} : +34.8°(c = 0.83, CHCl₃) |
| 25-3a | NMR1 : 0.71-0.75 (1H, m), 0.90-1.01 (3H, m), 1.84 (6H, s), 1.90 (3H, s), 2.44-2.60 (2H, m), 3.58 (1H, t, J = 6.8 Hz), 3.70-3.73 (2H, m), 4.00 (2H, t, J = 5.3 Hz), 4.86 (1H, t, J = 4.5 Hz), 5.07 (2H, s), 6.50 (1H, d, J = 2.3 Hz), 6.54-6.57 (1H, m), 6.72 (2H, s), 6.94-6.97 (1H, m), 7.14 (1H, d, J = 8.3 Hz), 7.26 (1H, t, J = 7.5 Hz), 7.40 (1H, d, J = 6.6 Hz) ESI- : 487.1 [α]²³_{D :} -48.0°(c = 1.00, CHCl₃) |
| 25-3b | NMR1 : 0.71-0.75 (1H, m), 0.90-1.03 (3H, m), 1.84 (6H, s), 1.90 (3H, s), 2.44-2.60 (2H, m), 3.58 (1H, t, J = 7.1 Hz), 3.70-3.73 (2H, m), 3.99 (2H, t, J = 5.1 Hz), 4.86 (1H, t, J = 5.8 Hz), 5.07 (2H, s), 6.50 (1H, d, J = 2.7 Hz), 6.55-6.57 (1H, m), 6.72 (2H, s), 6.95 (1H, d, J = 6.5 Hz), 7.14 (1H, d, J = 8.4 Hz), 7.26 (1H, t, J = 7.1 Hz), 7.39 (1H, d, J = 7.1 Hz) ESI- : 487.1 [α]²³_{D} : +45.7°(c = 0.70, CHCl₃) |
| 25-4a | NMR1 : 0.70-0.75 (1H, m), 0.90-1.04 (3H, m), 1.84 (6H, s), 1.90 (3H, s), 2.44-2.59 (2H, m), 3.42-3.47 (2H, m), 3.58 (1H, t, J = 6.8 Hz), 3.79-3.88 (2H, m), 3.97-4.04 (1H, m), 4.67 (1H, t, J = 6.1 Hz), 4.94 (1H, d, J = 4.7 Hz), 5.07 (2H, s), 6.51 (1H, d, J = 2.7 Hz), 6.55-6.57 (1H, m), 6.72 (2H, s), 6.95 (1H, d, J = 6.8 Hz), 7.14 (1H, d, J = 8.8 Hz), 7.26 (1H, t, J = 7.4 Hz), 7.40 (1H, d, J = 7.4 Hz) ESI- : 517.1 [α]²³_{D} : -41.0°(c = 1.00, CHCl₃) |
| 25-4b | NMR1 : 0.70-0.75 (1H, m), 0.86-1.02 (3H, m), 1.84 (6H, s), 1.90 (3H, s), 2.44-2.60 (2H, m), 3.43-3.47 (2H, m), 3.58 (1H, t, J = 6.6H z), 3.76-3.88 (2H, m), 3.97-4.04 (1H, m), 4.67 (1H, t, J = 5,3 Hz), 4.94 (1H, d, J = 5.3 Hz), 5.07 (2H, s), 6.51 (1H, d, J = 2.7 Hz), 6.55-6.57 (1H, m), 6.72 (2H, s), 6.96 (1H, d, J = 6.0 Hz), 7.14 (1H, d, J = 8.6 Hz), 7.26 (1H, t, J = 7.2 Hz), 7.40 (1H, d, J = 7.2 Hz) ESI- : 517.1 [α]²³_{D} : +45.1°(c = 1.00, CHCl₃) |
| 25-5a | NMR1 : 0.71-0.75 (1H, m), 0.90-1.02 (3H, m), 1.84 (6H, s), 1.90 (3H, s), 2.44-2.58 (2H, m), 3.43-3.49 (2H, m), 3.58 (1H, t, J = 6.3 Hz), 3.77-3.88 (2H, m), 3.97-4.04 (1H, m), 4.67 (1H, t, J = 5.5 Hz), 4.94 (1H, d, J = 4.7 Hz), 5.07 (2H, s), 6.51 (1H, d, J = 2.7 Hz), 6.55-6.57 (1H, m), 6.72 (2H, s), 6.95 (1H, d, J = 6.3 Hz), 7.14 (1H, d, J = 7.8 Hz), 7.26 (1H, t, J = 7.8 Hz), 7.40 (1H, d, J = 6.3 Hz) ESI- : 517.1 [α]²³_{D} : -39.6°(c = 0.70, CHCl₃) |
| 25-5b | NMR1 : 0.71-0.75 (1H, m), 0.90-1.02 (3H, m), 1.84 (6H, s), 1.90 (3H, s), 2.44-2.60 (2H, m), 3.45 (2H, s), 3.58 (1H, t, J = 6.9 Hz), 3.80-3.88 (2H, m), 3.97-4.04 (1H, m), 4.67 (1H, t, J = 4.9 Hz), 4.94 (1H, d, J = 4.4 Hz), 5.07 (2H, s), 6.50 (1H, d, J = 2.7 Hz), 6.55-6.57 (1H, m), 6.72 (2H, s), 6.95 (1H, d, J = 6.2 Hz), 7.14 (1H, d, J = 8.1 Hz), 7.26 (1H, t, J = 7.5 Hz), 7.40 (1H, d, J = 6.9 Hz) ESI- : 517.2 [α]²³_{D} : +44.5°(c = 0.99, CHCl₃) |

**[Table 541**

| Ex | Data |
|---|---|
| 25-6a | NMR1: 0.44-0.54 (3H, m), 0.65-0.69 (1H, m), 1.85 (6H, s), 1.91 (3H, s), 2.17-2.43 (3H, m), 2.92-2.98 (2H, m), 3.32 (3H, s), 3.65-3.67 (2H, m), 4.08-4.10 (2H, m), 4.22 (2H, d, J = 4.8 Hz), 5.89 (1H, t, J = 4.8 Hz), 6.40-6.44 (2H, m), 6.72 (2H, s), 6.84-6.90 (2H, m), 7.19 (1H, t, J = 7.6 Hz), 7.29 (1H, d, J = 7.7 Hz) ESI- : 498.2 [α]²³_{D :} -46.9°(c = 0.86, CHCl₃) |
| 25-6b | NMR1: 0.45-0.53 (3H, m), 0.65-0.69 (1H, m), 1.85 (6H, s), 1.91 (3H, s), 2.18-2.43 (3H, m), 2.92-2.98 (2H, m), 3.32 (3H, s), 3.65-3.67 (2H, m), 4.08-4.10 (2H, m), 4.22 (2H, d, J = 4.5 Hz), 5.89 (1H, t, J = 4.5 Hz), 6.40-6.44 (2H, m), 6.72 (2H, s), 6.84-6.90 (2H, m), 7.19 (1H, t, J = 7.8 Hz), 7.29 (1H, d, J = 7.8 Hz) ESI- : 498.2 [α]²³_{D} : +53.0°(c = 1.00, CHCl₃) |
| 25-7a | NMR1: 0.71-0.75 (1H, m), 0.90-1.02 (3H, m), 1.59-1.67 (1H, m), 1.84 (6H, s), 1.90-1.95 (4H, m), 2.44-2.60 (2H, m), 3.30-3.39 (2H, m), 3.58 (1H, t, J = 6.9 Hz), 3.65 (1H, s), 4.07 (2H, t, J = 6.9 Hz), 4.57 (1H, s), 4.62 (1H, d, J = 4.7 Hz), 5.07 (2H, s), 6.50 (1H, d, J = 2.7 Hz), 6.55-6.57 (1H, m), 6.71 (2H, s), 6.95 (1H, d, J = 7.5 Hz), 7.14 (1H, d, J = 8.9 Hz), 7.26 (1H, t, J = 7.5 Hz), 7.40 (1H, d, J = 7.5 Hz) ESI- : 531.2 [α]²³_{D} : -40.3°(c=0.43, CHCl₃) |
| 25-7b | NMR1 : 0.71-0.75 (1H, m), 0.90-1.03 (3H, m), 1.63-1.67 (1H, m), 1.84 (6H, s), 1.90-1.95 (4H, m), 2.44-2.60 (2H, m), 3.34-3.42 (2H, m), 3.58 (1H, t, J = 7.0 Hz), 3.65 (1H, s), 4.07 (2H, t, J = 7.0 Hz), 4.57 (1H, s), 4.62 (1H, d, J = 4.0 Hz), 5.07 (2H, s), 6.50 (1H, d, J = 2.7 Hz), 6.55-6.57 (1H, m), 6.71 (2H, s), 6.96 (1H, d, J = 7.0 Hz), 7.14 (1H, d, J = 9.0 Hz), 7.26 (1H, t, J = 7.3 Hz), 7.40 (1H, d, J = 6.9 Hz) ESI- : 531.2 [α]²³_{D} : +38.9°(c=0.69, CHCl₃) |
| 26 | NMR1 : 0.41-0.56 (3H, m), 0.64-0.71 (1H, m), 1.97 (3H, s), 2.04 (6H, s), 2.21 (1H, dd, J = 8.4, 15.5 Hz), 2.32-2.45 (2H, m), 2.91-2.99 (2H, m), 3.70-3.76 (2H, m), 4.24 (2H, d, J = 5.0 Hz), 4.32 (2H, t, J = 5.1 Hz), 4.89 (1H, t, J = 5.3 Hz), 5.92 (1H, t, J = 5.5 Hz), 6.41 (1H, dd, J = 1.9, 8.1 Hz), 6.44-6.47 (1H, m), 6.90 (1H, d, J = 8.2 Hz), 6.97-7.01 (1H, m), 7.25 (1H, t, J = 7.6 Hz), 7.36 (1H, d, J = 7.4 Hz), 11.87-12.43 (1H, br) ESI+:488 |
| 26-1 | NMR1 : 2.03 (3H, s), 2.05 (6H, s), 2.65 (1H, dd, J = 7.4, 16.2 Hz), 2.72 (1H, dd, J = 7.0, 16.1 Hz), 3.69-3.79 (2H, m), 4.20-4.26 (1H, m), 4.30-4.37 (2H, m), 4.78-4.91 (1H, br), 5.24 (2H, s), 7.02 (1H, dd, J = 2.3, 8.3 Hz), 7.11 (1H, d, J = 7.4 Hz), 7.28-7.42 (3H, m), 7.50 (1H, d, J = 8.4 Hz), 7.54-7.60 (2H, m), 7.61 (1H, d, J = 2.2 Hz), 7.87 (1H, d, J = 7.4 Hz), 11.87-13.03 (1H, br) ESI+ : 511 |
| 26-2 | NMR1 : 1.96-2.07 (10H, m), 2.64 (1H, dd, J = 7.4, 16.2 Hz), 2.72 (1H, dd, J = 7.1, 16.2 Hz), 3.49-3.58 (4H, m), 4.20-4.25 (1H, m), 4.30 (2H, d, J = 6.0 Hz), 4.51-4.59 (2H, m), 5.24 (2H, s), 7.02 (1H, dd, J = 2.4, 8.3 Hz), 7.09-7.14 (1H, m), 7.29-7.41 (3H, m), 7.50 (1H, d, J = 8.4 Hz), 7.54-7.60 (2H, m), 7.62 (1H, d, J = 2.4 Hz), 7.88 (1H, d, J = 7.6 Hz) ESI+ : 555 |
| 26-3 | NMR1 : 0.41-0.56 (3H, m), 0.64-0.71 (1H, m), 1.94-2.06 (10H, m), 2.21 (1H, dd, J = 8.4, 15.5 Hz), 2.32-2.45 (2H, m), 2.91-2.99 (2H, m), 3.49-3.59 (4H, m), 4.24 (2H, d, J = 4.9 Hz), 4.29 (2H, d, J = 5.9 Hz), 4.51-4.59 (2H, m), 5.88-5.95 (1H, m), 6.38-6.47 (2H, m), 6.90 (1H, d, J = 8.2 Hz), 6.96-7.01 (1H, m), 7.25 (1H, t, J = 7.6 Hz), 7.33-7.39 (1H, m), 11.91-12.43 (1H, br) ESI+ : 532 |

**[Table 55]**

| Ex | Data |
|---|---|
| 27 | NMR1 : 0.41-0.56 (3H, m), 0.64-0.71 (1H, m), 1.97 (3H, s), 2.04 (6H, s), 2.21 (1H, dd, J = 8.4, 15.5 Hz), 2.32-2.45 (2H, m), 2.91-3.00 (2H, m), 3.42-3.49 (2H, m), 3.78-3.86 (1H, m), 4.17-4.27 (3H, m), 4.32 (1H, dd, J = 4.3, 10.9 Hz), 4.63-4.75 (1H, br), 4.92-5.04 (1H, br), 5.87-5.97 (1H, m), 6.41 (1H, dd, J = 2.1, 8.2 Hz), 6.44-6.47 (1H, m), 6.90 (1H, d, J = 8.2 Hz), 6.97-7.01 (1H, m), 7.25 (1H, t, J = 7.6 Hz), 7.36 (1H, d, J = 7.4 Hz), 11.76-12.49 (1H, br) ESI+ : 518 |
| 27-1 | NMR1 : 0.41-0.56 (3H, m), 0.64-0.71 (1H, m), 1.97 (3H, s), 2.04 (6H, s), 2.21 (1H, dd, J = 8.4, 15.5 Hz), 2.32-2.45 (2H, m), 2.91-3.00 (2H, m), 3.42-3.51 (2H, m), 3.78-3.86 (1H, m), 4.17-4.27 (3H, m), 4.32 (1H, dd, J = 4.4, 10.9 Hz), 4.64-4.74 (1H,m), 4.94-5.02 (1H, m), 5.88-5.96 (1H, m), 6.41 (1H, dd, J = 2.0, 8.2 Hz), 6.44-6.47 (1H, m), 6.90 (1H, d, J = 8.2 Hz), 6.97-7.01 (1H, m), 7.25 (1H, t, J = 7.6 Hz), 7.34-7.38 (1H, m), 11.85-12.49 (1H, br) ESI+ : 518 |
| 28 | NMR1 : 0.66-0.89 (2H, m), 0.91-1.01 (2F, m), 1.80-1.93 (11H, m), 2.21-2.28 (2H, m), 3.38-3.67 (3H, m), 4.03 (2H, t, J = 6.4 Hz), 4.30-4.80 (1H, m), 5.05 (2H, s), 6.44 (1H, d, J = 2.2 Hz), 6.50 (1H, dd, J = 2.2, 8.2 Hz), 6.71 (2H, s), 6.91-6.98 (1H, m), 7.11-7.19 (1H, m), 7.21-7.29 (1H, m), 7.34-7.44 (1H, m) ESI- : 501 |
| 29 | NMR1 : 0.66-0.82 (2H, m), 0.90-1.07 (2H, m), 1.84 (6H, s), 1.85-1.97 (5H, m), 2.06-2.20 (2H, m), 2.91 (3H, s), 3.06-3.18 (2H, m), 3.56-3.69 (1H, m), 4.03 (2H, t, J = 6.1 Hz), 5.05 (2H, s), 6.42 (1H, d, J = 2.2 Hz), 6.49 (1H, dd, J = 2.2, 8.2 Hz), 6.72 (2H, s), 6.92-6.99 (1H, m), 7.10-7.20 (2H, m), 7.21-7.32 (1H, m), 7.35-7.42 (1H, m) ESI- : 578 |
| 30a | NMR1 : 1.87 (6H, s), 1.97 (3H, s), 2.65 (1H, dd, J = 7.4, 16.2 Hz), 2.72 (1H, dd, J = 7.0, 16.2 Hz), 3.68-3.76 (2H, m), 3.99 (2H, t, J = 5.1 Hz), 4.23 (1H, t, J = 7.1 Hz), 4.76-4.97 (1H, br), 5.22 (2H, s), 6.73 (2H, s), 6.95-7.03 (2H, m), 7.25-7.34 (2H, m), 7.38 (1H, t, J = 7.4 Hz), 7.46-7.52 (2H, m), 7.58 (1H, d, J = 7.5 Hz), 7.61 (1H, d, J = 2.4 Hz), 7.88 (1H, d, J = 7.4 Hz) ESI- : 507 [α]²⁵_{D} : -8.7°(c 0.89,CHCl₃) |
| 30b | NMR1 : 1.86 (6H, s), 1.97 (3H, s), 2.60-2.74 (2H, m), 3.69-3.75 (2H, m), 3.99 (2H, t, J = 5.1 Hz), 4.20-4.26 (1H, m), 4.84-4.89 (1H, m), 5.22 (2H, s), 6.73 (2H, s), 6.95-7.03 (2H, m), 7.26-7.33 (2H, m), 7.35-7.41 (1H, m), 7.46-7.52 (2H, m), 7.58 (1H, d, J = 7.6 Hz), 7.60 (1H, d, J = 2.4 Hz), 7.87 (1H, d, J = 7.4 Hz) ESI- : 507 [α]²⁵_{D :} +8.7° (c 0.89,CHCl₃) |
| 31 | NMR1 : 0.69-0.75 (1H, m), 0.90-1.02 (3H, m), 1.84 (6H, s), 1.90 (3H, s), 2.44-2.59 (2H, m), 3.56 (1H, t, J = 7.7 Hz), 3.71 (2H, s), 4.00 (2H, t, J = 5.1 Hz), 4.81 (1H, s), 5.07 (2H, s), 6.49 (1H, d, J = 2.2 Hz), 6.54-6.57 (1H, m), 6.71 (2H, s), 6.95 (1H, d, J = 7.2 Hz), 7.14 (1H, d, J = 7.5 Hz), 7.25 (1H, t, J = 7.2 Hz), 7.39 (1H, d, J = 7.5 Hz) ESI+ : 489 |
| 31-1 | NMR1 : 0.72-0.75 (1H, m), 0.90-1.02 (3H, m), 1.16 (6H, s), 1.84-1.86 (8H, m), 1.90 (3H, s), 2.44-2.60 (2H, m), 3.59 (1H, t, J = 6.6 Hz), 4.08 (2H, t, J = 6.6 Hz), 4.34 (1H, s), 5.07 (2H, s), 6.50 (1H, d, J = 2.0 Hz), 6.55-6.57 (1H, m), 6.74 (2H, s), 6.95 (1H, d, J = 6.9 Hz), 7.15 (1H, d, J = 8.3 Hz), 7.26 (1H, t, J = 7.3 Hz), 7.40 (1H, d, J = 6.9 Hz) ESI+ : 531 |

**[Table 56]**

| Ex | Data |
|---|---|
| 31-2 | NMR1 : 0.71-0.75 (1H, m), 0.90-1.03 (3H, m), 1.84 (6H, s), 1.90 (3H, s), 1.91-1.98 (2H, m), 2.44-2.59 (2H, m), 3.26 (3H, s), 3.48 (2H, t, J = 6.3 Hz), 3.57-3.62 (1H, m), 4.02 (2H, t, J = 6.2 Hz), 5.07 (2H, s), 6.50 (1H, d, J = 2.1 Hz), 6.54-6.57 (1H, m), 6.71 (2H, s), 6.95 (1H, d, J = 6.7 Hz), 7.14 (1H, d, J = 8.4 Hz), 7.26 (1H, d, J = 7.4 Hz), 7.39 (1H, d, J = 7.2 Hz) ESI+: 517 |
| 31-3 | NMR1 : 0.70-0.75 (1H, m), 0.90-1.05 (3H, m), 1.14 (3H, t, J = 7.0 Hz), 1.84 (6H, s), 1.90 (3H, s), 2.44-2.60 (2H, m), 3.52 (2H, q, J = 7.0 Hz), 3.57-3.62 (1H, m), 3.70 (2H, t, J = 4.7 Hz), 4.09 (2H, t, J = 4.7 Hz), 5.07 (2H, s), 6.50 (1H, d, J = 2.2 Hz), 6.54-6.57 (1H, m), 6.72 (2H, s), 6.95 (1H, d, J = 7.7 Hz), 7.14 (1H, d, J = 8.3 Hz), 7.25 (1H, t, J = 7.6 Hz), 7.39 (1H, d, J = 7.6 Hz) ESI+ : 517 |
| 32 | NMR1 : 0.70-0.75 (1H, m), 0.89-1.02 (3H, m), 1.84 (6H, s), 1.90 (3H, s), 2.44-2.60 (2H, m), 3.46 (2H, s), 3.58 (1H, t, J = 6.9 Hz), 3.79 (1H, s), 3.84-3.88 (1H, m), 3.98-4.01 (1H, m), 4.64 (1H, s), 4.90 (1H, s), 5.07 (2H, s), 6.50 (1H, d, J = 2.2 Hz), 6.55-6.57 (1H, m), 5.71 (2H, s), 6.95 (1H, d, J = 7.6 Hz), 7.14 (1H, d, J = 8.3 Hz), 7.26 (1H, t, J = 7.5 Hz), 7.40 (1H, d, J = 7.6 Hz) ESI+ : 519 |
| 32-1 | NMR1 : 0.71-0.75 (1H, m), 0.90-1.01 (3H, m), 1.84 (6H, s), 1.90 (3H, s), 2.44-2.59 (2H, m), 3.46 (2H, d, J = 5.5 Hz), 3.59 (1H, t, J = 7.1 Hz), 3.78-3.88 (2H, m), 3.98-4.01 (1H, m), 4.65 (1H, s), 4.91 (1H, s), 5.07 (2H, s), 6.50 (1H, d, J = 2.2 Hz), 6.54-6.57 (1H, m), 6.71 (2H, s), 6.95 (1H, d, J = 7.2 Hz), 7.14 (1H, d, J = 8.2 Hz), 7.26 (1H, t, J = 7.7 Hz), 7.39 (1H, d, J = 7.7 Hz) ESI+ : 519 |

**[Table 57]**

| **Pr** | **Structure** |
|---|---|
| **16-10** | 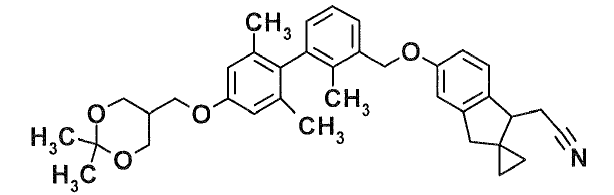 |
| **16-11** | 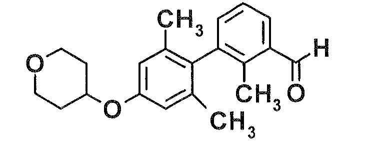 |
| **16-12** | 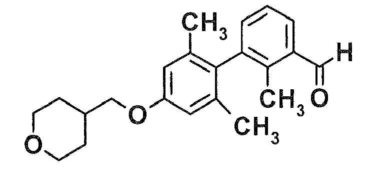 |
| **16-13** | 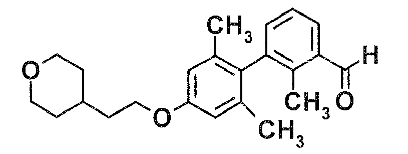 |
| **27** | 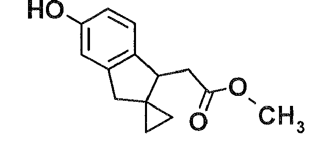 |

### Industrial Applicability

The compound of the formula (I) has an excellent GPR40 agonistic activity, and can be therefore used as an insulin secretion promoter, or an agent for preventing and/or treating GPR40-related diseases diabetes (insulin-dependent diabetes (IDDM), non-insulin-dependent diabetes (NIDDM), or borderline type (abnormal glucose tolerance and fasting blood glucose level) mild diabetes), insulin-resistant diseases, obesity, and the like.

### [Sequence List Free Text]

Under the number title <223> in the following sequence listing, provided is description on "Artificial Sequence". Specifically, the base sequence as set forth as SEQ NO. 1 in the sequence listing is the base sequence of an artificially synthesized primer. Furthermore, the primer sequence as set forth as SEQ NO. 2 in the sequence listing is the base sequence of an artificially synthesized primer.

## Claims

1. A compound of the formula (I) or a salt thereof: 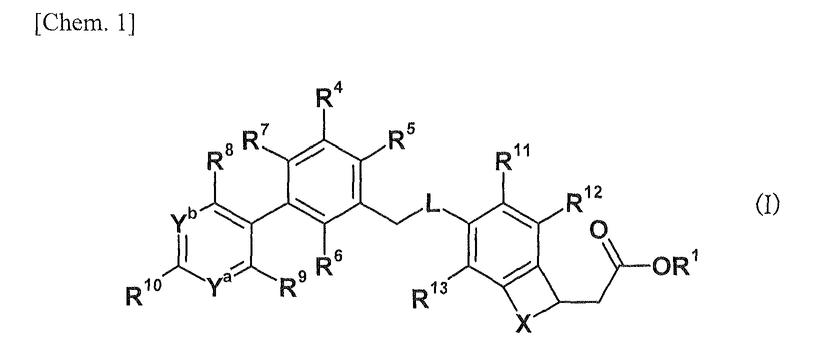 (wherein
L represents O or NH,
R¹ represents H or lower alkyl,
X represents 1,2-phenylene or -Z-C(R²)(R³)-,
Z represents O or CH₂,
R² and R³ are combined with each other to form C₂-₇ alkylene which may be substituted,
R⁴, R⁵, R⁶, R⁷, R⁸, and R⁹ are the same as or different from each other and represent H, halogen, lower alkyl which may be substituted, or -O-(lower alkyl which may be substituted),
R¹⁰ represents H, OH, -O-(hetero ring group which may be substituted), or -O-(CR¹⁰¹R¹⁰²)ₙ-R¹⁰³,
R¹⁰¹ and R¹⁰² are the same as or different from each other and represent H, OH, halogen, or lower alkyl which may be substituted, or
R¹⁰¹ and R¹⁰² are combined with each other to form oxo (=O),
n represents 1, 2, 3, or 4,
R¹⁰³ represents H, OH, halogen, NR^{N1}R^{N2}, -SO₂-(lower alkyl which may be substituted), aryl which may be substituted, -O-(lower alkyl which may be substituted), or a hetero ring group which may be substituted,
R^{N1} and R^{N2} are the same as or different from each other and represent H, -SO₂-(lower alkyl which may be substituted), or lower alkyl which may be substituted,
R¹¹, R¹², and R¹³ are the same as or different from each other and represent H, halogen, lower alkyl which may be substituted, or -O-(lower alkyl which may be substituted),
Y^{a} and Y^{b} are the same as or different from each other, N, or C-R^{Y}, and
R^{Y} represents H, halogen, lower alkyl which may be substituted, or -O-(lower alkyl which may be substituted)).

2. A compound of the formula (I') or a salt thereof: 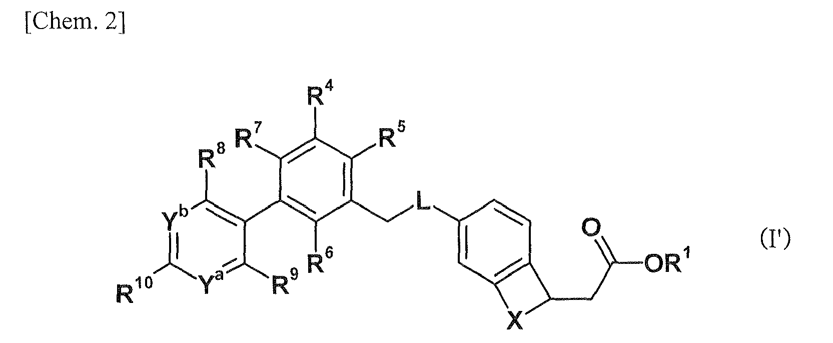 (wherein
L represents O or NH,
R¹ represents H or lower alkyl,
X represents 1,2-phenylene or -Z-C(R²)(R³)-,
Z represents O or CH₂,
R² and R³ are combined with each other to form C₂-₇ alkylene,
R⁴, R⁵, R⁶, R⁷, R⁸, and R⁹ are the same as or different from each other and represent H, halogen, lower alkyl, or -O-lower alkyl,
R¹⁰ represents H, OH, -O-hetero ring group, or -O-(CR¹⁰¹R¹⁰²)ₙ-R¹⁰³,
R¹⁰¹ and R¹⁰² are the same as or different from each other and represent H, OH, halogen, or lower alkyl which may be substituted with OH, or
R¹⁰¹ and R¹⁰² are combined with each other to form oxo (=O),
n represents 1, 2, 3, or 4,
R¹⁰³ represents H, OH, halogen, NR^{N1}R^{N2}, -SO₂-lower alkyl, or -O-lower alkyl which may be substituted with aryl or oxo (=O), or a hetero ring group which may be substituted with lower alkyl or oxo (=O),
R^{N1} and R^{N2} are the same as or different from each other and represent H, -SO₂-lower alkyl, or lower alkyl which may be substituted with oxo (=O),
Y^{a} and Y^{b} are the same as or different from each other, N, or C-R^{Y}, and
R^{Y} represents H, halogen, lower alkyl, or -O-lower alkyl).

3. The compound or a salt thereof as set forth in claim 1, wherein X is 1,2-phenylene, R⁴, R⁵, R⁶, R⁷, R⁸, and R⁹ are the same as or different from each other and represent H or lower alkyl, R¹⁰ is H or -O-(CR¹⁰¹R¹⁰²)ₙ-R¹⁰³, R¹⁰¹ and R¹⁰² are the same as or different from each other and represent H, OH, or lower alkyl, and R¹⁰³ is OH, or -O-lower alkyl which may be substituted with aryl or oxo (=O).

4. The compound or a salt thereof as set forth in claim 3, wherein R¹ is H, R⁶ is lower alkyl, R⁴, R⁵, and R⁷ are H, R⁸ and R⁹ are lower alkyl, R¹⁰ is -O-(CR¹⁰¹R¹⁰²)ₙ-R¹⁰³, n is 2, 3, or 4, Y^{a} and Y^{b} are C-R^{Y}, and R^{Y} is H.

5. The compound or a salt thereof as set forth in claim 4, wherein X is -Z-C(R²)(R³)-, Z is CH₂, R² and R³ are combined with each other to form C₂-₇ alkylene, R⁴, R⁵, R⁶, R⁷, R⁸, and R⁹ are the same as or different from each other and represent H or lower alkyl, R¹⁰ is H or -O-(CR¹⁰¹R¹⁰²)ₙ-R¹⁰³, R¹⁰¹ and R¹⁰² are the same as or different from each other and represent H, OH, or lower alkyl, and R¹⁰³ is OH, or -O-lower alkyl which may be substituted with aryl or oxo (=O).

6. The compound or a salt thereof as set forth in claim 4, wherein R¹ is H, methyl, or ethyl, R² and R³ are combined with each other to form ethylene, R⁶ is lower alkyl, R⁴, R⁵, and R⁷ are H, R⁸ and R⁹ are lower alkyl, R¹⁰ is -O-(CR¹⁰¹R¹⁰²)ₙ-R¹⁰³, n is 2, 3, or 4, Y^{a} and Y^{b} are C-R^{Y}, and R^{Y} is H.

7. The compound or a salt thereof as set forth in claim 6, wherein R¹ is H, R⁶ is methyl, R⁴, R⁵, and R⁷ are H, R⁸ and R⁹ are methyl, R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, R¹⁰³ is OH or methoxy, Y^{a} and Y^{b} are C-R^{Y}, and R^{Y} is H.

8. The compound or a salt thereof as set forth in claim 1, wherein X is -Z-C(R²)(R³)-, Z is O, R² and R³ are combined with each other to form C₂₋₇ alkylene, R⁴, R⁵, R⁶, R⁷, R⁸, and R⁹ are the same as or different from each other and represent H or lower alkyl, R¹⁰ is H or -O-(CR¹⁰¹R¹⁰²)ₙ-R¹⁰³, R¹⁰¹ and R¹⁰² are the same as or different from each other and represent H, OH, or lower alkyl, and R¹⁰³ is OH, or -O-lower alkyl which may be substituted with aryl or oxo (=O).

9. The compound or a salt thereof as set forth in claim 8, wherein R¹ is H, methyl, or ethyl, X is -Z-C(R²)(R³)-, Z is O, R² and R³ are combined with each other to form ethylene, R⁶ is lower alkyl, R⁴, R⁵, and R⁷ are H, R⁸ and R⁹ are lower alkyl, R¹⁰ is -O-(CR¹⁰¹R¹⁰²)ₙ-R¹⁰³, n is 2, 3, or 4, Y^{a} and Y^{b} are C-R^{Y}, and R^{Y} is H.

10. The compound or a salt thereof as set forth in claim 9, wherein R¹ is H, R⁶ is H, R⁶ is methyl, R⁴, R⁵, and R⁷ are H, R⁸ and R⁹ are methyl, R¹⁰ is H or -O-(CR¹⁰¹R¹⁰²)ₙ-R¹⁰³, R¹⁰¹ and R¹⁰² are the same as or different from each other and represent H, OH, or methyl, n is 2, 3, or 4, R¹⁰³ is OH or methoxy, Y^{a} and Y^{b} are C-R^{Y}, and R^{Y} is H.

11. The compound or a salt thereof as set forth in claim 1, which is
(3-{[4'-(2-hydroxyethoxy)-2,2',6'-trimethylbiphenyl-3-yl]methoxy}-9H-fluoren-9-yl)acetic acid,
{5'-[(4'-{[(2R)-2,3-dihydroxypropyl]oxy}-2,2',6'-trimethylbiphenyl-3-yl)methoxy]-1',3'-dihydrospiro[cyclopropan-1,2'-inden]-1'-yl} acetic acid,
{5'-[(4'-{[(2S)-2,3-dihydroxypropyl]oxy}-2,2',6'-trimethylbiphenyl-3-yl)methoxy]-1',3'-dihydrospiro[cyclopropan-1,2'-inden]-1'-yl}aceticacid,
{3-[(2,2',6'-trimethylbiphenyl-3-yl)methoxy]-9H-fluorene f-9-yl}acetic acid,
{3-[(4'-{[(2R)-2,3-dihydroxypropyl]oxy}-2,2',6'-trimethylbiphenyl-3-yl)methoxy]-9H-fluoren-9-yl}acetic acid,
{3-[(4'-{[(2S)-2,3-dihydroxypropyl]oxy}-2,2',6'-trimethylbiphenyl-3-yl)methoxy]-9H-fluoren-9-yl}acetic acid,
[5'-({[4'-(2-hydroxyethoxy)-2,2',6'-trimethylbiphenyl-3-yl]methyl amino)-1',3'-dihydrospiro[cyclopropan-1,2'-inden]-1'-yl]acetic acid,
(5'-{[(4'-{[(2R)-2,3-dihydroxypropyl]oxy}-2,2',6'-trimethylbiphenyl-3-yl)methyl]amino}-1',3'-dihydrospiro[cyclopropan-1,2'-inden]-1'-yl)acetic acid,
(5'-{[(4'-{[(2S)-2,3-dihydroxypropyl]oxy}-2,2',6'-trimethylbiphenyl-3-yl)methyl]amino}-1',3'-dihydrospiro[cyclopropan-1,2'-inden]-1'-yl)acetic acid,
[5'-({[4'-(3-hydroxy-3-methylbutoxy)-2,2',6'-trimethylbiphenyl-3-yl]methyl}amino)-1',3'-dihydrospiro[cyclopropan-1,2'-inden]-1'-yl]aceticacid,
(6-{[4'-(2-hydroxyethoxy)-2,2',6'-trirnethylbiphenyl-3-yl]methoxy}-3H-spiro[1-benzofuran-2,1'-cyclopropan]-3-yl)acetic acid,
(6-{[4'(3-hydroxy-3-methylbutoxy)-2,2',6'-trimethylbiphenyl-3-yl]methoxy}-3H-spiro[1-benzofuran-2,1'-cyclopropan]-3-yl)acetic acid,
{6-[(4'-{[(2R)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
{6-[(4'-{[(2S)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
[5'-({[4'-(2-methoxyethoxy)-2,2',6'-trimethylbiphenyl-3-yl]methyl}amino)-1',3'-dihydrospiro[cyclopropan-1,2'-inden]-1'-yl]acetic acid,
[5'-({3-[2-(2-hydroxyethoxy)-4,6-dimethylpyrimidin-5-yl]-2-methylbenzyl}oxy)-1',3'-dihydrospiro [cyclopropan-1,2'-inden]-1'-yl]aceticacid,
[5'-({3-[2-(3-hydroxy-3-methylbutoxy)-4,6-dimethylpyrimidin-5-yl]-2-methylbenzyl}oxy)-1',3'-dihydrospiro[cyclopropan-1,2'-inden]-1'-yl] acetic acid,
[(1'S)-5'-({[4'-(2-hydroxyethoxy)-2,2',6'-trimethylbiphenyl-3-yl]methyl}amino)-1',3'-dihydrospiro[cyclopropan-1,2'-inden]-1'-yl]acetic acid,
[(1'R)-5'-({[4'-(2-hydroxyethoxy)-2,2',6'-trimethylbiphenyl-3-yl]methyl}amino)-1',3'-dihydrospiro[cyclopropan-1,2'-inden]-1'-yl]acetic acid,
(6-{[4'-(2-methoxyethoxy)-2,2',6'-trimethylbiphenyl-3-yl]methoxy}-3H-spiro[1-benzofuran-2,1'-cyclopropan]-3-yl)acetic acid,
{6-[(4'-{[(3R)-3,4-dihydroxybutyl]oxy}-2,2',6'-trimethylbiphenyl-3-yl)methoxy]-3H-spiro[1-benzofuran-2,1'-cyclopropan]-3-yl} acetic acid,
{6-[(4'-{[(3S)-3,4-dihydroxybutyl]oxy}-2,2',6'-trimethylbiphenyl-3-yl)methoxy]-3H-spiro[1-benzofuran-2,1'-cyclopropan]-3-yl}aceticacid,
(6-{[4'-(2-ethoxyethoxy)-2,2',6'-trimethylbiphenyl-3-yl]methoxy}-3H-spiro[1-benzofuran-2,1'-cyclopropan]-3-yl)acetic acid,
(6-{[4'-(3-methoxypropoxy)-2,2',6'-trimethylbiphenyl-3-yl]methoxy}-3H-spiro[1-benzofuran-2,1'-cyclopropan]-3-yl)acetic acid,
[(9S)-3-{[4'-(2-hydroxyethoxy)-2,2',6'-trimethylbiphenyl-3-yl]methoxy}-9H-fluoren-9-yl]acetic acid,
[(9R)-3-{[4'-(2-hydroxyethoxy)-2,2',6'-trimethylbiphenyl-3-yl]methoxy}-9H-fluoren-9-yl]acetic acid,
[(3R)-6-{[4'-(2-methoxyethoxy)-2,2',6'-trimethylbiphenyl-3-yl]methoxy}-3H-spiro[1-benzofuran-2,1'-cyclopropan]-3-yl]acetic acid,
[(3S)-6-{[4'-(2-methoxyethoxy)-2,2',6'-trimethylbiphenyl-3-yl]methoxy}-3H-spiro[1-benzofuran-2,1'-cyclopropan]-3-yl]acetic acid,
[(3R)-6-{[4'-(3-hydroxy-3-methylbutoxy)-2,2',6'-trimethylbiphenyl-3-yl]methoxy}-3H-spiro[1-benzofuran-2,1'-cyclopropan]-3-yl)acetic acid,
[(3S)-6-{[4'-(3-hydroxy-3-methylbutoxy)-2,2',6'-trimethylbiphenyl-3-yl]methoxy} -3H-spiro[1-benzofuran-2,1'-cyclopropan]-3-yl]acetic acid,
[(3R)-6-{[4'-(2-hydroxyethoxy)-2,2',6'-trimethylbiphenyl-3-yl]methoxy}-3H-spiro[1-benzofuran-2,1'-cyclopropan]-3-yl]acetic acid,
[(3 S)-6- {[4'-(2-hydroxyethoxy)-2,2',6'-trimethylbiphenyl-3-yl]methoxy}-3 H-spiro[1-benzofuran-2,1'-cyclopropan]-3-yl]aceticacid,
{(3R)-6-[(4'-{[(2R)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
{(3S)-6-[(4'-{[(2R)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
{(3R)-6-[(4'-{[(2S)-2,3-dihydroxypropyl]oxy}-2,2',6'-trimethylbiphenyl-3-yl)methoxy]-3H-spiro[1-benzofuran-2,1'-cyclopropan]-3-yl}acetic acid,
{(3S)-6-[(4'-{[(2S)-2,3-dihydroxypropyl]oxy}-2,2',6'-trimethylbiphenyl-3-yl)methoxy]-3H-spiro[1-benzofuran-2,1'-cyclopropan]-3-yl} acetic acid,
[(1'S)-5'-({[4'-(2-methoxyethoxy)-2,2',6'-trimethylbiphenyl-3-yl]methyl}amino)-1',3'-dihydrospiro[cyclopropan-1,2'-inden]-1'-yl] acetic acid,
[(1'R)-5'-({[4'-(2-methoxyethoxy)-2,2',6'-trimethylbiphenyl-3-yl]methyl}amino)-1',3'-dihydrospiro[cyclopropan-1,2' -inden]-1'-yl]acetic acid,
{(3R)-6-[(4'-{[(3S)-3,4-dihydroxybutyl]oxy}-2,2',6'-trimethylbiphenyl-3-yl)methoxy]-3H-spiro[1-benzofuran-2,1'-cyclopropan]-3-yl}acetic acid,
{(3S)-6-[(4'-{[(3S)-3,4-dihydroxybutyl]oxy}-2,2',6'-trimethylbiphenyl-3-yl)methoxy]-3H-spiro[1-benzofuran-2,1'-cyclopropan]-3-yl}acetic acid,
{(3R)-6-[(4'-{[(3R)-3,4-dihydroxybutyl]oxy}-2,2',6'-trimethylbiphenyl-3-yl)methoxy]-3H-spiro[1-benzofuran-2,1'-cyclopropan]-3-yl}acetic acid, or
{(3S)-6-[(4'-{[(3R)-3,4-dihydroxybutyl]oxy}-2,2',6'-trimethylbiphenyl-3-yl)methoxy]-3H-spiro[1-benzofuran-2,1'-cyclopropan]-3-yl}acetic acid.

12. A pharmaceutical composition comprising the compound or a salt thereof as set forth in claim 1, and a pharmaceutically acceptable excipient.

13. A pharmaceutical composition for preventing or treating GPR40-related diseases, comprising the compound or a salt thereof as set forth in claim 1.

14. Use of the compound or a salt thereof as set forth in claim 1 for the manufacture of a pharmaceutical composition for preventing or treating GPR40-related diseases.

15. Use of the compound or a salt thereof as set forth in claim 1 for prevention or treatment of GPR40-related diseases.

16. A method for preventing or treating GPR40-related diseases, comprising administering to a patient an effective amount of the compound or a salt thereof as set forth in claim 1.

17. The compound or a salt thereof as set forth in claim 1, for prevention or treatment of GPR40-related diseases.
